# EUROPEAN PATENT APPLICATION

(11) **EP 1 443 041 A1**
(43) Date of publication of application: **04.08.2004**
(21) Application number: 02777984.2
(22) Date of filing: 29.10.2002
(51) Int. Cl.: C07C 311/37, C07C 311/29, C07D 207/00, C07D 211/62, C07D 213/00, C07D 231/12, C07D 233/36, C07D 241/04, C07D 261/08, C07D 263/20, C07D 271/00, C07D 277/44, C07D 295/00, C07D 333/00, A61K 31/00, A61P 7/02, A61P 9/00, A61P 9/04, A61P 9/10, A61P 11/00, A61P 13/12

(54) **5-AMIDINO-2-HYDROXYBENZENESULFONAMIDE DERIVATIVES, MEDICINAL COMPOSITIONS CONTAINING THE SAME, MEDICINAL USE THEREOF AND INTERMEDIATES IN THE PRODUCTION THEREOF**

(30) Priority: 09.11.2001 JP 2001345435
(71) Applicant: Kissei Pharmaceutical Co., Ltd., Matsumoto-shi Nagano 399-8710 (JP)
(72) Inventor: UCHIDA, Masahiko, Minamiazumi-gun, Nagano 399-8302 (JP); KOBAYASHI, Hiroaki, Matsumoto-shi, Nagano 399-0011 (JP); KAI, Yuichiro, Minamiazumi-gun, Nagano 399-8201 (JP); YOKOYAMA, Kenji, Toyoshina-machi, Minamiazumi-gun, Nagano (JP); TERAO, Yoshihiro, Matsumoto-shi, Nagano 390-0312 (JP); MURANAKA, Hideyuki, Toyoshina-machi, Minamiazumi-gunNagano (JP)
(74) Representative: Hayes, Adrian Chetwynd
(86) International application number: PCT/JP2002/011199
(87) International publication number: WO 2003/040086

(57) **Abstract**

The present invention relates to a 5-amidino-2-hydroxybenzenesulfonamide derivative represented by the general formula: 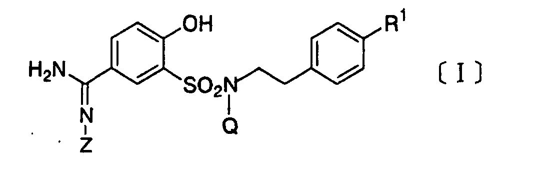 wherein R¹ is an optionally substituted lower alkyl group, an optionally substituted lower alkoxy group, an optionally substituted lower alkenyl group, a cycloalkyl group or a lower acyl group etc.;
Q is a hydrogen atom or an optionally substituted lower alkyl group; and
Z is a hydrogen atom or a hydroxy group etc.,
or a pharmaceutically acceptable salt thereof, which exert a potent and selective activated blood coagulation factor X inhibitory activity and is useful as an agent for the prevention or treatment of a disease occurred associating an activated blood coagulation factor X, a pharmaceutical composition comprising the same and an intermediate thereof. These compounds are useful as preventives or remedies for various diseases such as brain infarction, cerebral thrombosis, cerebral embolism, TIA, cerebral vascular jerk, Alzheimer's diseases, myocardial infarction, heart attack, heart failure, thrombosis, pulmonary infarction and pulmonary embolism.

## Description

### Technical Field

The present invention relates to novel 5-amidino-2-hydroxybenzenesulfonamide derivatives or pharmaceutically acceptable salts thereof which are useful as medicaments.

More particularly, the present invention relates to 5-amidino-2-hydroxybenzenesulfonamide derivatives or pharmaceutically acceptable salts thereof, which exert an excellent inhibitory activity on activated blood coagulation factor X and are useful as activated blood coagulation factor X inhibitors, pharmaceutical compositions comprising the same, their pharmaceutical uses and intermediates for their preparation.

### Background Art

The anticoagulation therapy has been extensively performed for the prevention and treatment of thromboembolic diseases caused by blood hypercoagulability, and drugs such as heparin and warfarin potassium have been frequently used as anticoagulant agents at present.

However, heparin shows inhibitory activity on thrombin and activated blood coagulation factor X, and has been known to have a risk of causing bleeding tendency.

Warfarin potassium is an anticoagulant which controls biosynthesis of vitamin K-dependent coagulation factor, and it is difficult to control the anticoagulation capacity due to its action mechanism when this drug is used in the prevention and treatment of thromboembolic diseases. Therefore, this drug is extremely hard to use clinically.

In recent years, selective thrombin inhibitors have been developed and have been used clinically. However, since thrombin plays a close part in the conversion of fibrinogen into fibrin in blood coagulation cascade reactions and platelet activation and aggregation, the thrombin inhibitors have similar disadvantages to heparin on the point of view of the safety such as bleeding tendency. Moreover, it has been reported that their efficacies are not necessarily sufficient.

On the other hand, activated blood coagulation factor X, which acts at the juncture of the extrinsic and intrinsic blood coagulation cascade reactions, located on the upstream of thrombin, so that an anticoagulation activity of activated blood coagulation factor X is more efficient than that of thrombin inhibitors. Therefore, activated blood coagulation factor X inhibitors attract public attentions as drugs having a possibility to inhibit the coagulation system effectively.

Furthermore, with the changing into European and American life styles and the aging of population have been developed in recent years, incidences of thromboembolic diseases such as myocardial infarction and arteriovenous obstruction will go on increasing, and therefore, the social importance of more efficient anticoagulants has been going on increasing, and the demands on development of such anticoagulants are great.

### Disclosure of the Invention

The present inventors have studied hard to find novel compounds having an excellent inhibitory activity on activated blood coagulation factor X. As a result, it was surprisingly found that certain 5-amidino-2-hydroxybenzenesulfonamide derivatives show a potent and selective activated blood coagulation factor X inhibitory activity, thereby forming the basis of the present invention.

The present invention is to provide novel compounds which exert a potent and selective activated blood coagulation factor X inhibitory activity.

This is, (1) the present invention relates to a 5-amidino-2-hydroxybenzenesulfonamide derivative represented by the general formula: 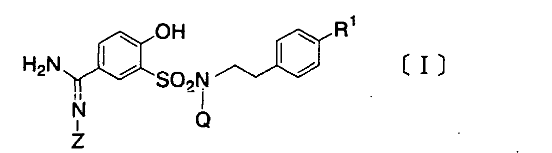 wherein R¹ represents a lower alkyl group which may have -COOR^{A} in which R^{A} is a hydrogen atom or a lower alkyl group, a lower alkoxy group which may have -COOR^{A} in which R^{A} has the same meaning as defined above, a lower alkenyl group which may have -COOR^{A} in which R^{A} has the same meaning as defined above, a 3 to 10-membered cycloalkyl group, a lower acyl group, -COOR^{A} in which R^{A} has same meaning as defined above, -CONR^{B}R^{C} in which R^{B} and R^{C} are independently a hydrogen atom or a lower alkyl group, or -NR^{B}R^{C} forms a cyclic amino group, an amino group, a mono or di(lower alkyl)amino group, a cyclic amino group which may have an oxo group, a 3 to 10-membered heterocycloalkyl group which may have an oxo group, a 6 to 10-membered aryl group which may have one to three substituents selected from the following group (A), or a 5 to 10-membered aromatic heterocyclic group which may have a substituent selected from the following group (B);
Q represents a hydrogen atom, a lower alkenyl group, a lower alkynyl group or a lower alkyl group which may have a substituent selected from the following group (C);
Z represents a hydrogen atom, a hydroxy group, -COOR^{N} in which R^{N} is a halo(lower alkyl) group, a 6 to 10-membered aryl group or a lower alkyl group which may have a substituent selected from the following group (vii);
(A) an oxo group, a lower alkyl group, a halo (lower alkyl) group, -Y¹-R^{D} in which Y¹ is a oxygen atom or a sulfur atom; R^{D} is a hydrogen atom, a halo(lower alkyl) group or a lower alkyl group which may have -COOR^{D1} in which R^{D1} is a hydrogen atom, a 3 to 10-membered cycloalkyl group or a lower alkyl group, a halogen atom, a nitro group, an amino group, -COOR^{E} in which R^{E} is a hydrogen atom, a 3 to 10-membered cycloalkyl group or a lower alkyl group, a carbamoyl group, a sulfamoyl group, a (lower alkyl)sulfonyl group, a mono(lower alkyl)sulfamoyl group which may have -COOR^{F} in which R^{F} is a hydrogen atom, a 3 to 10-membered cycloalkyl group or a lower alkyl group, and a (lower alkyl)sulfonylamino-substituted (lower alkyl) group;
(B) a lower alkyl group, an amino group and -COOR^{G} in which R^{G} is a hydrogen atom, a 3 to 10-membered cycloalkyl group and a lower alkyl group;
(C) -OR^{H} in which R^{H} is a hydrogen atom, a lower alkyl group, a lower alkenyl group, a phenyl group or a lower alkyl group which may have -OR^{H1} in which R^{H1} is a hydrogen atom or a lower alkyl group, -COOR^{I} in which R^{I} is a hydrogen atom, a 3 to 10-membered cycloalkyl group or a lower alkyl group which may have a substituent selected from the following group (iii), -CONR^{J}R^{K} in which R^{J} and R^{K} are independently a hydrogen atom, an amino group, a 6 to 10-membered aryl group which may have a carbamoyl group, a 5 to 10-membered aromatic heterocyclic group which may have a substituent selected from the following group (iv) or a lower alkyl group which may have a substituent selected from the following group (v), or -NR^{J}R^{K} forms a cyclic amino group which may have a substituent selected from the following group (vi), a cyclic amino group which may have an oxo group, a 3 to 10-membered heterocycloalkyl group which may have an oxo group or a halogen atom, a 6 to 10-membered aryl group which may have one to three substituents selected from the following group (i), and a 5 to 10-membered aromatic heterocyclic group which may have one to three substituents selected from the following group (ii);

(i) a halogen atom, a nitro group, an amidino group, a lower alkyl group, a halo(lower alkyl) group, -OR^{L} in which R^{L} is a hydrogen atom or a lower alkyl group, and -COOR^{M} in which R^{M} is a hydrogen atom or a lower alkyl group;
(ii) a halogen atom, an oxo group, a lower alkyl group and a phenyl group;
(iii) -COOR^{I1} in which R^{I1} is a hydrogen atom, a 3 to 10-membered cycloalkyl group or a lower alkyl group, -OCOR^{I2} in which R^{I2} is a 3 to 10-membered cycloalkyl group or a lower alkyl group, -OCOOR^{I3} in which R^{I3} is a 3 to 10-membered cycloalkyl group or a lower alkyl group, -OR^{I4} in which R^{I4} is a hydrogen atom, a 3 to 10-membered cycloalkyl group or a lower alkyl group, -CONR^{I5}R^{I6} in which R^{I5} and R^{I6} are independently a hydrogen atom or a lower alkyl group, or -NR^{I5}R^{I6} forms a cyclic amino group, a 3 to 10-membered cycloalkyl group, a 6 to 10-membered aryl group, a 3 to 10-membered heterocycloalkyl group and a 5 to 10-membered aromatic heterocyclic group;
(iv) a halogen atom, a lower alkyl group which may have -COOR^{J1} in which R^{J1} is a hydrogen atom or a lower alkyl group, a carbamoyl group and -COOR^{J2} in which R^{J2} is a hydrogen atom, or a lower alkyl group;
(v) -OR^{J3} in which R^{J3} is a hydrogen atom or a lower alkyl, group, and a 5 to 10-membered aromatic heterocyclic group;
(vi) a hydroxy group, a lower alkyl group, a hydroxy (lower alkyl) group,a di(lower alkyl)amino-substituted(lower alkyl)group, a carbamoyl group, a di(lower alkyl)amino group, a lower acyl group, and -COOR^{J4} in which R^{J4} is a hydrogen atom or a lower alkyl group;
(vii) -OR^{N1} in which R^{N1} is a hydrogen atom or a lower alkyl group which may have a 6 to 10-membered aryl group, -COOR^{N2} in which R^{N2} is a lower alkyl group which may have -COOR^{N21} where R^{N21} is a lower alkyl group, -CONR^{N3}R^{N4} in which R^{N3} and R^{N4} are independently a hydrogen atom or a lower alkyl group, or -NRR^{N3}R^{N4} forms a cyclic amino group, -OCOR^{N5} in which R^{N5} is a lower alkyl which may have -OCOR^{N51} where R^{N51} is a lower alkyl group, a cyclic amino group, a 3 to 10-membered heterocycloalkyl group, and a 6 to 10-membered aryl group;
with the proviso that Q does not represent a hydrogen atom when Z represents a hydrogen atom and R¹ represents a lower alkyl group, a lower alkoxy group or a 3 to 10-memberedheterocycloalkyl group, or a pharmaceutically acceptable salt thereof;
(2) A 5-amidino-2-hydroxybenzenesulfonamide derivative represented by the above general formula (I) or a pharmaceutically acceptable salt thereof, wherein R¹ represents a 6 to 10-membered aryl group which may have one to three substituents selected from the following group (A);
(A) an oxo group, a lower alkyl group, a halo (lower alkyl) group, -Y¹-R^{D} in which Y¹ is an oxygen atom or a sulfur atom; R^{D} is a hydrogen atom, a halo(lower alkyl) group, a lower alkyl group which may have -COOR^{D1} in which R^{D1} is a hydrogen atom, a 3 to 10-membered cycloalkyl group or a lower alkyl group, a halogen atom, a nitro group, an amino group, -COOR^{E} in which R^{E} is a hydrogen atom, a 3 to 10-membered cycloalkyl group or a lower alkyl group, a carbamoyl group, a sulfamoyl group, a (lower alkyl) sulfonyl group, a monno (lower alkyl) sulfamoyl group which may have -COOR^{F} in which R^{F} is a hydrogen atom, a 3 to 10-membered cycloalkyl group or a lower alkyl group, and a (lower alkyl)sulfonylamino-substituted (lower alkyl) group.

The present invention relates to a pharmaceutical composition comprising as an active ingredient a 5-amidino-2-hydroxybenzenesulfonamide derivative described in the above (1) or (2), or a pharmaceutically acceptable salt thereof.

The present invention relates to an agent for the prevention or treatment of a disease occurred associating an activated blood coagulation factor X, which comprises as an active ingredient a 5-amidino-2-hydroxybenzenesulfonamide derivative described in the above (1) or (2), or a pharmaceutically acceptable salt thereof.

The present invention relates to an agent for the prevention or treatment of a disease occurred associating an activated blood coagulation factor X, which comprises as an active ingredient a 5-amidino-2-hydroxybenzenesulfonamide derivative described in the above (1) or (2), or a pharmaceutically acceptable salt thereof.

The present invention relates to a method for the prevention or treatment of a disease occurred associating an activated blood coagulation factor X, which comprises administering a therapeutically effective amount of a 5-amidino-2-hydroxybenzenesulfonamide derivative described in the above (1) or (2), or a pharmaceutically acceptable salt thereof.

The present invention relates to a use of a 5-amidino-2-hydroxybenzenesulfonamide derivative described in the above (1) or (2), or a pharmaceutically acceptable salt thereof for the manufacture of a pharmaceutical composition for the prevention or treatment of a disease occurred associating an activated blood coagulation factor X.

The present invention relates to a pharmaceutical composition which comprises (a) a 5-amidino-2-hydroxybenzenesulfonamide derivative described in the above (1) or (2), or a pharmaceutically acceptable salt thereof, and (b) at least one member selected from the group consisting of adrenocortical hormone, antiplatelet drugs, adenylate cyclase activators, PGF2α antagonists, cyclooxygenase inhibitors, adenosine antagonists, GPIIb/IIIa antagonists, anticoagulants, thrombolitic drugs, antithrombin drugs, free-radical scavengers, immunosuppressants, erythropoietin, fish oil, angiotensin-converting enzyme inhibitors, angiotensin II receptor antagonists, glycation inhibitors, protein C kinase inhibitors, aldose reductase inhibitors, endothelin receptor antagonists, endothelin-converting enzyme inhibitors, neutral endopeptidase inhibitors, thromoxane A₂ synthetase inhibitors, thromboxane A₂ receptor antagonists and PGI₂ agonists.

The present invention relates to an agent for the prevention or treatment of a disease occurred associating an activated blood coagulation factor X, which comprises as an active ingredient (a) a 5-amidino-2-hydroxybenzenesulfonamide derivative described in the above (1) or (2), or a pharmaceutically acceptable salt thereof, and (b) at least one member selected from the group consisting of adrenocortical hormone, platelet aggregation inhibitors, adenylate cyclase activators, PGF2□ antagonists, cyclooxygenase inhibitors, adenosine antagonists, GPIIb/IIIa antagonists, anticoagulants, thrombolitic drugs, antithrombin drugs, free-radical scavengers, immunosuppressants, erythropoietin, fish oil, angiotensin-converting enzyme inhibitors, angiotensin II receptor antagonists, glycation inhibitors, protein C kinase inhibitors, aldose reductase inhibitors, endothelin receptor antagonists, endothelin-converting enzyme inhibitors, neutral endopeptidase inhibitors, thromoxane A₂ synthetase inhibitors, thromboxane A₂ receptor antagonists and PGI₂ agonists.

The present invention relates to a method for the prevention or treatment of a disease occurred associating an activated blood coagulation factor X, which comprises administering a therapeutically effective amount of (a) a 5-amidino-2-hydroxybenzenesulfonamide derivative described in the above (1) or (2), or a pharmaceutically acceptable salt thereof, and (b) at least one member selected from the group consisting of adrenocortical hormone, platelet aggregation inhibitors, adenylate cyclase activators, PGF2□ antagonists, cyclooxygenase inhibitors, adenosine antagonists, GPIIb/IIIa antagonists, anticoagulants, thrombolitic drugs, antithrombin drugs, free-radical scavengers, immunosuppressants, erythropoietin, fish oil, angiotensin-converting enzyme inhibitors, angiotensin II receptor antagonists, glycation inhibitors, protein C kinase inhibitors, aldose reductase inhibitors, endothelin receptor antagonists, endothelin-converting enzyme inhibitors, neutral endopeptidase inhibitors, thromoxane A₂ synthetase inhibitors, thromboxane A₂ receptor antagonists and PGI₂ agonists.

The present invention relates to a use of (a) a 5-amidino-2-hydroxybenzenesulfonamide derivative described in the above (1) or (2), or a pharmaceutically acceptable salt thereof, and (b) at least one member selected from the group consisting of adrenocortical hormone, platelet aggregation inhibitors, adenylate cyclase activators, PGF2α antagonists, cyclooxygenase inhibitors, adenosine antagonists, GPIIb/IIIa antagonists, anticoagulants, thrombolitic drugs, antithrombin drugs, free-radical scavengers, immunosuppressants, erythropoietin, fish oil, angiotensin-converting enzyme inhibitors, angiotensin II receptor antagonists, glycation inhibitors, protein C kinase inhibitors, aldose reductase inhibitors, endothelin receptor antagonists, endothelin-converting enzyme inhibitors , neutral endopeptidase inhibitors, thromoxane A₂ synthetase inhibitors, thromboxane A₂ receptor antagonists and PGI₂ agonists, for the manufacture of a pharmaceutical composition for the prevention or treatment of a disease occurred associating an activated blood coagulation factor X.

Furthermore, the present invention relates to a 5-cyano-2-hydroxybenzenesulfonamide derivative represented by the general formula: 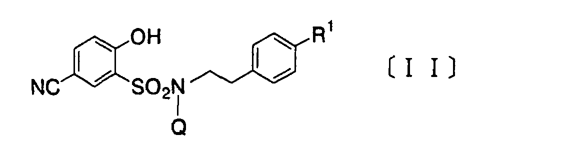 wherein R¹ represents a lower alkyl group which may have -COOR^{A} in which R^{A} is a hydrogen atom or a lower alkyl group, a lower alkyl group which may have -COOR^{A} in which R^{A} has the same meaning as defined above, a lower alkenyl group which may have -COOR^{A} in which R^{A} has the same meaning as defined above, a 3 to 10-membered cycloalkyl group, a lower acyl group, -COOR^{A} in which R^{A} has same meaning as defined above, -CONR^{B}R^{C} in which R^{B} and R^{C} are independently a hydrogen atom or a lower alkyl group, or -NR^{B}R^{C} forms a cyclic amino group, an amino group, a mono or di(lower alkyl)amino group, a cyclic amino group which may have an oxo group, a 3 to 10-membered heterocycloalkyl group which may have an oxo group, a 6 to 10-membered aryl group which may have one to three substituents selected from the following group (A), or a 5 to 10-membered aromatic heterocyclic group which may have a substituent selected from the following group (B);
Q represents a hydrogen atom, a lower alkenyl group, a lower alkynyl group or a lower alkyl group which may have a substituent selected from the following group (C);
(A) an oxo group, a lower alkyl group, a halo (lower alkyl) group, -Y¹-R^{D} in which Y¹ is an oxygen atom or a sulfur atom; RD is a hydrogen atom, a halo(lower alkyl ) group, a lower alkyl group which may have -COOR^{D1} in which R^{D1} is a hydrogen atom, a 3 to 10-membered cycloalkyl group or a lower alkyl group, a halogen atom, a nitro group, an amino group, -COOR^{E} in which R^{E} is a hydrogen atom, a 3 to 10-membered cycloalkyl group or a lower alkyl group, a carbamoyl group, a sulfamoyl group, a (lower alkyl)sulfonyl group, a mono (lower alkyl) sulfamoyl group which may have -COOR^{F} in which R^{F} is a hydrogen atom, a 3 to 10-membered cycloalkyl group or a lower alkyl group, and a (lower alkyl)sulfonylamino-substituted (lower alkyl) group;
(B) a lower alkyl group, an amino group and -COOR^{G} in which R^{G} is a hydrogen atom, a 3 to 10-membered cycloalkyl group and a lower alkyl group;
(C) -OR^{H} in which R^{H} is a hydrogen atom, a lower alkyl group, a lower alkenyl group, a phenyl group or a lower alkyl group which may have -OR^{H1} in which R^{H1} is a hydrogen atom or a lower alkyl group, -COOR^{I} in which R^{I} is a hydrogen atom, a 3 to 10-membered cycloalkyl group or a lower alkyl group which may have a substituent selected from the following group (iii), -CONR^{J}R^{K} in which R^{J} and R^{K} are independently a hydrogen atom, an amino group, a 6 to 10-membered aryl group which may have a carbamoyl group, a 5 to 10-membered aromatic heterocyclic group which may have a substituent selected from the following group (iv), a lower alkyl group which may have a substituent selected from the following group (v), or -NR^{J}R^{K} forms a cyclic amino group which may have a substituent selected from the following group (vi), a cyclic amino group which may have an oxo group, a 3 to 10-membered heterocycloalkyl group which may have an oxo group or a halogen atom, a 6 to 10-membered aryl group which may have one to three substituents selected from the following group (i), and a 5 to 10-membered aromatic heterocyclic group which may have one to three substituents selected from the following group (ii);

(i) a halogen atom, a nitro group, an amidino group, a lower alkyl group, a halo(lower alkyl) group, -OR^{L} in which R^{L} is a hydrogen atom or a lower alkyl group, and -COOR^{M} in which R^{M} is a hydrogen atom or lower alkyl group;
(ii) a halogen atom, an oxo group, a lower alkyl group and a phenyl group;
(iii) -COOR^{I1} in which R^{I1} is a hydrogen atom, a 3 to 10-membered cycloalkyl group or a lower alkyl group, a 3 to 10-memmbered cycloalkyl group or a lower alkyl group, -OCOR^{I2} in which R^{I2} is a 3 to 10-membered cycloalkyl group or a lower alkyl group, -OCOOR^{I3} in which R^{I3} is a 3 to 10-membered cycloalkyl group or a lower alkyl group, -ORI4 in which R^{I4} is a hydrogen atom, a 3 to 10-membered cycloalkyl group or a lower alkyl group, -CONR^{I5}R^{I6} in which R^{I5} and R^{I6} are independently a hydrogen atom or a lower alkyl group, a 3 to 10-membered cycloalkyl group, a 6 to 10-membered aryl group, a 3 to 10-membered heterocycloalkyl group and a 5 to 10-membered aromatic heterocyclic group;
(iv) a halogen atom, a lower alkyl group which may have -COOR^{J1} in which R^{J1} is a hydrogen atom or a lower alkyl group, a carbamoyl group and -COOR^{J2} in which R^{J2} is a hydrogen atom, or a lower alkyl group;
(v) -OR^{J3} in which R^{J3} is a hydrogen atom or a lower alkyl group, and a 5 to 10-membered aromatic heterocyclic group;
(vi) a hydroxy group, a lower alkyl group, a hydroxy (lower alkyl) group, a di (lower alkyl) group, a carbamoyl group, a di ( lower alkyl)amino group, a lower acyl group, and -COOR^{J4} in which R^{J4} is a hydrogen atom or a lower alkyl group;
with the proviso that Q does not represent a hydrogen atom when R¹ represents a lower alkyl group, a lower alkoxy group or a 3 to 10-membered heterocycloalkyl group, or a salt thereof.

In the present invention, the term "lower alkyl group" means a straight-chained or branched alkyl group having 1 to 6 carbon atoms such as a methyl group, an ethyl group, a propyl group, an isopropyl group, a butyl group, an isobutyl group, a sec-butyl group, apentylgroup, an isopentyl group, a neopentyl group, a 1-methylbutyl group, a 2-methylbutyl group, a hexyl group or the like. The term "(lower alkyl)sulfonyl group" means a sulfonyl group having the above lower alkyl group, such as a methanesulfonyl group, an ethanesulfonyl group, a propanesulfonyl group, an isopropanesulfonyl group, a butanesulfonyl group, an isobutanesulfonyl group, a *sec*-butanesulfonyl group, a pentanesulfonyl group, an isopentanesulfonyl group, a neopentanesulfonyl group, a hexanesulfonyl group or the like. The term "mono ( lower alkyl)sulfamoyl group" means a monoalkylsulfamoyl group wherein the alkyl moiety is the same as the above lower alkyl group. The term "mono(lower alkyl)amino group" means a monoalkyled amino group wherein the alkyl moiety is the same as the above lower alkyl group. The term "di(lower alkyl)amino group" means an amino group di-substituted by same or different lower alkyl groups as defined above. The term "di(lower alkyl)amino-substituted (lower alkyl) group" means a lower alkyl group di-substituted by the above di(lower alkyl)amino group. The term "(lower alkyl)sulfonylamino-substituted (lower alkyl) group" means the above alkyl group having an amino group N-substituted by the above (lower alkyl)sulfonyl group. The term "hydroxy(lower alkyl) group" means a straight-chained or branched alkyl group having 2 to 6 carbon atoms and substituted by a hydroxy group. The term "lower alkylene group" means a straight-chained or branched alkylene group having 1 to 6 carbon atoms such as a methylene group, an ethylene group, a trimethylne group, a propylene group or the like.

The term "lower alkoxy group" means a straight-chained or branched alkoxy group having 1 to 6 carbon atoms such as a methoxy group, an ethoxy group, a propoxy group, a isopropoxy group, a butoxy group, a isobutoxy group, a *sec*-butoxy group, a pentyloxy group, a isopentyloxy group, a neopentyloxy group, a 1-methylbutyloxy group, a 2-methylbutyloxy group, a hexyloxy group and the like. The term " lower alkenyl group" means a straight-chained or branched alkenyl group having 2 to 6 carbon atoms such as a vinyl group, an aryl group, a 1-propenyl group, a isopropenyl group, a 1-butenyl group, a 2-butenyl group, a 2-methylaryl group and the like. The term "lower acyl group" means a straight-chained or branched alkylcarbonyl group having 2 to 6 carbon atoms such as an acetyl group, a propionyl group, a butyryl group, an isobutyryl group, a valeryl group, a hexanoyl group or the like.

The term "3 to 10-membered cycloalkyl group" means a 3 to 7-membered monocyclic aliphatic alkyl group such as a cyclopropyl group, a cyclobutyl group, a cyclopentyl group, a cyclohexyl group or a cycloheptyl group, or a cyclopentyl group or a cyclohexyl group which are fused with a benzene ring. The term "6 to 10-membered aryl group" means a phenyl group, a naphthyl group, or a phenyl group which is fused with a cyclopentane ring or a cyclohexane ring.

The term "3 to 10-membered heterocycloalkyl group" means a 3 to 7-membered monocyclic heteroalkyl group containing one to two hetero atoms selected from a nitrogen atom, an oxygen atom and a sulfur atom in the ring, or a bicyclic heteroalkyl group which is benzene-fused 5 or 6-membered monocyclic heteroalkyl group as defined above, and as examples of such groups, for example, a monovalent group derived from morpholine, thiomorpholine, pyrrolidine, imidazoline, oxazoline, piperidine, piperazine, tetrahydrofuran, aziridine, azetidine, indoline, isoindoline, chroman, isochroman or the like can be illustrated.

The term "5 to 10-membered aromatic heterocyclic group" means a 5 to 6-membered monocyclic aromatic group containing one to four hetero atoms selected from a nitrogen atom, an oxygen atom and a sulfur atom in the ring, or a bicyclic heteroalkyl group which is benzene or pyridine-fused 5 or 6-membered monocyclic aromatic group as defined above, and as examples of such groups, for example, a monovalent group derived from pyridine, pyrimidine, pyrazine, pyridazine, pyrrole, thiophene, oxazole, thiazole, imidazole, pyrazole, oxadiazole, thiodiazole, tetrazole, indole, indolizine, benzofuran, benzothiophene, quinoline, isoquinoline, phthalazine, naphthyridine, quinoxaline, quinazoline, cinnoline or the like can be illustrated. As examples of aromatic heterocyclic group having an oxo group, for example, a monovalent group derived from a 1,3,4-oxadiazol-2-one or the like can be illustrated.

The term "cyclic amino group" means a 5 to 6-membered monocyclic amino group which may contain one hetero atom selected from a nitrogen atom, an oxygen atom and a sulfur atom other than the nitrogen atom at the binding site in the ring, such as a 1-pyrrolodinyl group, a piperidino group, a morpholino group, a thiomorpholino group, a 1-piperazinyl group or the like. As examples of cyclic amino group having an oxo group, for example, a 2-oxo-1-pyrrolidinyl group, a 2-oxopiperidino group or the like.

The term "halogen atom" means a fluorine atom, a chlorine atom, a bromine atom or an iodine atom. The term "halo(lower alkyl) group" means the above alkyl group substituted by one to three halogen atom as defined above, such as a trifluoromethyl group, a 2,2,2-trifluoroethyl group or the like.

The term "hydroxy-protective group" means a hydroxy-protective group used generally in organic synthesis, which is described in PROTECTIVE GROUPS IN ORGANIC SYNTHESIS, THEODORA W.GREENE, PETER G.WUTS by JOHN WILEY&SONS, INC, such as a benzyl group, a methoxymethyl group, an acetyl group or the like.

For example, the compounds represented by the above general formula (I) of the present invention can be prepared by allowing a 5-cyano-2-hydroxybenzenesulfonamide derivative represented by the above general formula (II) or a salt thereof to react with an alcohol in the presence of hydrogen chloride (hereinafter referred to as Process 1), allowing the resulting compound to react with ammonia or a salt thereof, or hydroxylamine or a salt thereof (hereinafter referred to as Process 2), carrying out, as occasion demands, suitably one to four processes selected from the group consisting of (1) hydrolysis of the resulting ester group (hereinafter referred to as Process 3), (2) ester interchange or esterification of the resulting compound using an alcohol compound represented by the general formula:

R⁰-OH [III]

wherein R⁰ represents a 3 to 10-membered cycloalkyl group or a lower alkyl group which may have a substituent selected from the above group (v), or esterification of the resulting compound using a compound represented by the general formula:

R⁰-X¹ [IV]

wherein X¹ represents a leaving group such as a halogen atom, a toluenesulfonyloxy group, a methanesulfonyloxy group or the like; and R⁰ has the same meaning as defined above (hereinafter referred to as Process 4), (3) introduction of a protective group into a phenolic hydroxy group (hereinafter referred to as Process 5) and (4) *N*-acylation of the resulting compound using a compound represented by the general formula:

R^{N}OCO-X² [V]

wherein X² represents a leaving group such as a halogen atom, a 4-nitrophenoxy group or the like; and R^{N} has the same meaning as defined above, and subjecting, as occasion demands, to removal of the protective group of the phenolic hydroxy group or *O*-deacylation in the usual way.

In the aforementioned production process, the reaction from a 5-cyano-2-hydroxybenzenesulfonamide derivative represented by the above general formula (II) into a 5-amidino-2-hydroxybenzenesulfonamide derivative represented by the above general formula (I) is as follows in detail.

### Process 1

A corresponding imidate compound can be prepared by allowing a 5-cyano-2-hydroxybenzenesulfonamide derivative represented by the above general formula (II) to react with an alcohol such as methanol or ethanol in the presence of a hydrogen halide such as hydrogen chloride or hydrogen bromide at usually -20°C to room temperature. As a solvent used, methanol, ethanol, a mixed solvent of such alcohol with tetrahydrofuran, dichloromethane or *N,N*-dimethylformamide, and the like can be illustrated. The reaction time is usually from 1 hour to 3 days, varying based on sorts and volumes of a used starting material and solvent.

### Process 2

A corresponding amidino compound can be prepared by allowing an imidate compound to react with ammonia or an ammonium salt such as ammonium carbonate, ammonium chloride or ammonium acetate, or hydroxylamine or a salt thereof in the presence or absence of a base such as triethylamine at usually 0°C to room temperature. As a solvent used, methanol, ethanol, tetrahydrofuran, dichloromethane and the like can be illustrated. The reaction time is usually from 1 hour to 3 days, varying based on sorts and volumes of a used starting material and solvent.

### Process 3

In case of compounds having an ester group in the amidino derivatives obtained by Process 2, a corresponding carboxylic acid compound can be prepared by subjecting such compound to hydrolysis using an acid such as hydrochloric acid or sulfuric acid at usually room temperature to reflux temperature, or a base such as sodium hydroxide at usually 0°C to reflux temperature. As a solvent used, water, acetonitrile, tetrahydrofuran, alcohols, a mixed solvent thereof and the like can be illustrated. The reaction time is usually from 1 hour to 2 days, varying based on sorts and volumes of a used starting material and solvent.

### Process 4

A corresponding ester compound can be prepared by 1) subjecting an amidino derivative having an ester group or a carboxy group obtained by Process 2 or 3 to ester interchange or esterification using an alcohol compound represented by the above general formula (III) in the presence of an acid such as hydrochloric acid, sulfuric acid or *p*-toluenesulfonic acid at usually 0°C to reflux temperature, by 2) subjecting a compound having a carboxy group of the amidino derivatives obtained by Process 2 or 3 to esterification using an alcohol compound represented by the above general formula (III) in the presence of a condensing agent such as 1-(3-dimethylaminopropyl)-3-ethylcarbodiimide hydrochloride at usually 0°C to reflux temperature, or by 3) subjecting a compound having a carboxy group of the amidino derivatives obtained by Process 2 or 3 to esterification using a compound represented by the above general formula (IV) in the presence of a base such as potassium carbonate or triethylamine, or silver carbonate at usually 0°C to reflux temperature. As a solvent used, an aprotic solvent such as tetrahydrofuran and the like can be illustrated. The reaction time is usually from 1 hour to 2 days, varying based on sorts and volumes of a used starting material and solvent.

### Process 5

A corresponding *O*-protected compound can be prepared by suitably protecting a phenolic hydroxy group of a compound having an amidino group obtained by Processes 2 to 4 according to a method described in PROTECTIVE GROUPS IN ORGANIC SYNTHESIS, THEODORA W.GREENE, PETER G.WUTS by JOHN WILEY&SONS, INC.

### Process 6

A corresponding carbamate compound can be prepared by allowing a compound having an amidino group obtained by Processes 2-5 to react using a compound represented by the above general formula (V) in the presence of a base such as triethylamine or diisopropylethylamine at usually 0°C to room temperature. As a solvent used, *N*,*N*-dimethylformamide and the like can be illustrated. The reaction time is usually from 1 hour to 2 days, varying based on sorts and volumes of a used starting material and solvent.

The removal of the protective group of the hydroxy group can be commonly carried out according to a method described in PROTECTIVE GROUPS INORGANIC SYNTHESIS, THEODORAW.GREENE, PETER G.WUTS by JOHN WILEY&SONS, INC.

Of the compounds represented by the above general formula (I), a compound represented by the following general formula (Ia) can be also prepared according to the following method.

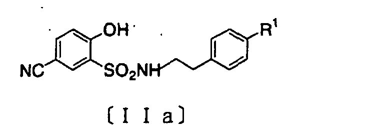

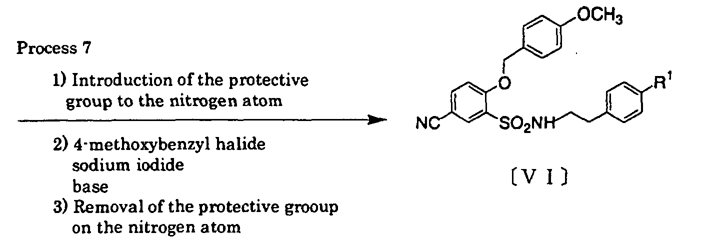

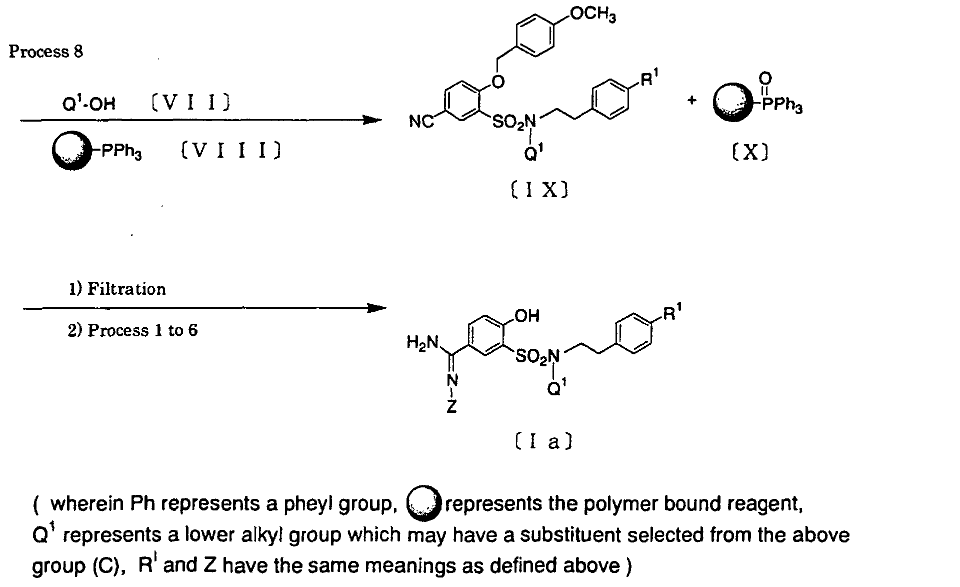

### Process 7

To the nitrogen atom of the sulfonamide group of the 5-cyano-2-hydroxybenzenesulfonamide derivative represented by the general formula(IIa) is introduced appropriate protecting group such as *tert*-butoxy carbonyl group according to a conventional method and after *O*-alkylation with 4-methoxybenzyl halide in the presence of an appropriate base such as potassium iodide, cesium carbonate, potassium carbonate or sodium hydride at room temperature to 80 ºC and removal of the protecting group on the nitrogen atom to give the compound represented by the general formula(VI). The appropriate solvent of the *O*-alkylation reaction is *N,N*-dimethylformamide, *N,N*-dimethylacetamide, acetone or these combined solvent. Although the reaction time change depends on the starting materials and solvents, it is usually for 0.5 to 24hours.

### Process 8

The compound represented by the general formula (VI) is allowed *N*-alkylation reaction with the alcohol derivative represented by the general formula (VII) in the presence of the polymer bound reagent represented by the general formula (VIII) and the dehydrating agents such as diisopropylazodicarboxylate and diethylazodicarboxylate at usually 0 ºC to room temperature to give the compound represented by the general formula (IX). The appropriate solvent of the reaction is tetrahydrofuran, dichloromethane or these combined solvent. Although the reaction time change depends on the starting materials and solvents, it is usually for 1 to 24hours.

After the production process 8, the polymer bound reagents represented by the general formula (VIII) and (X) are removed by the filtration and the resulting compound represented by the general formula (IX) can be converted into the compound represented by the general formula (Ia) by subjecting to the process 1 to 6 described above appropriately.

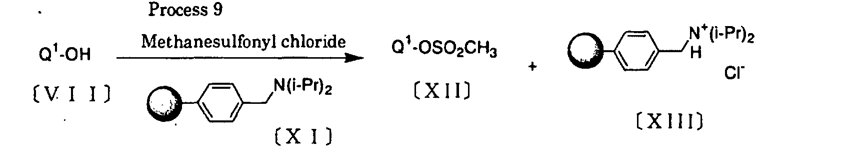

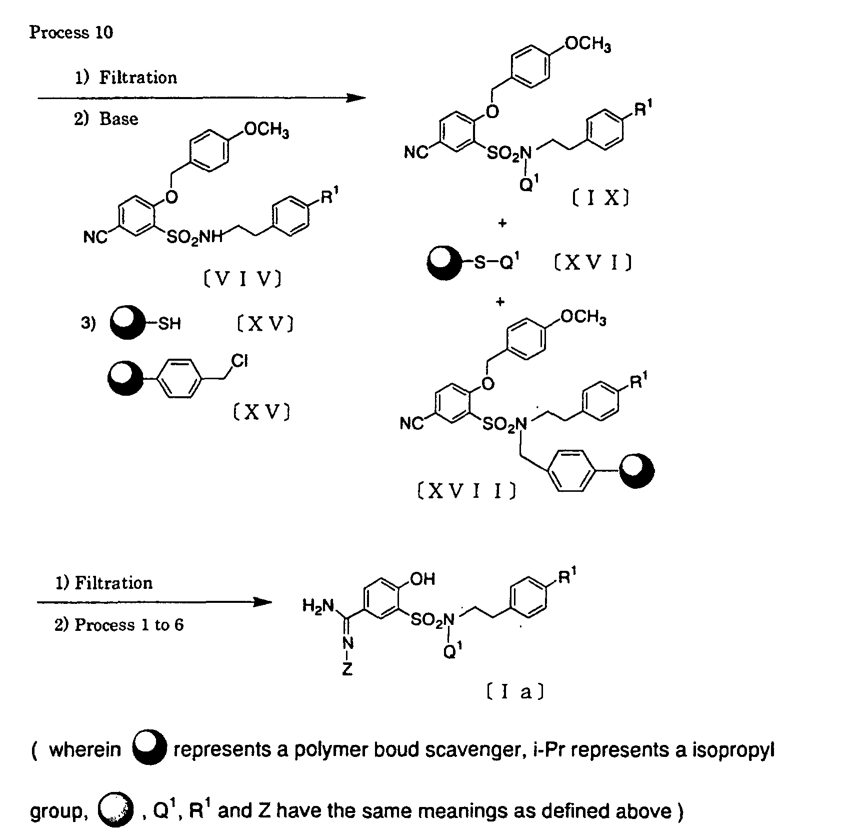

### Process 9

The alcohol derivative represented by the general formula (VII) is allowed to react with methanesulfonyl chrolide in the presence of the polymer bound reagent represented by the general formula (XI) at usually 0 ºC to room temperature to give the compound represented by the general formula (XII). The appropriate solvent of the reaction is dichloromethane, tetrahydrofuran or these combined solvent. Although the reaction time change depends on the starting materials and solvents, it is usually for 0.5 to 24hours.

### Process 10

After the filtration of the polymer bound reagents represented by the general formula (XI) and (XIII), the compound represented by the general formula (XII) is allowed to react with the compound represented by the general formula (VI) in the presence of an appropriate base such as potassium carbonate or cesium carbonate at usually 0 to 80 ºC to give the compound represented by the general formula (IX). The appropriate solvent of the reaction is *N,N*-dimethylformamide, tetrahydrofuran, acetone or these combined solvent. Although the reaction time change depends on the starting materials and solvents, it is usually for 3 to 24hours. After above reaction finished, the remaining starting materials can be scavenged by the addition of the polymer bound scavengers represented by the general formula (XIV) and (XV). After the process 10, the polymer bound scavengers represented by the general formula (XIV), (XV), (XVI) and (XVII) are removed by the filtration and the resulting compound represented by the general formula (IX) can be converted into the compound represented by the general formula (Ia) by subjecting to the process 1 to 6 described above appropriately.

Of the compounds represented by the general formula (I) in this invention, compound represented by the general formula (Ib) described below can be produced by the following method.

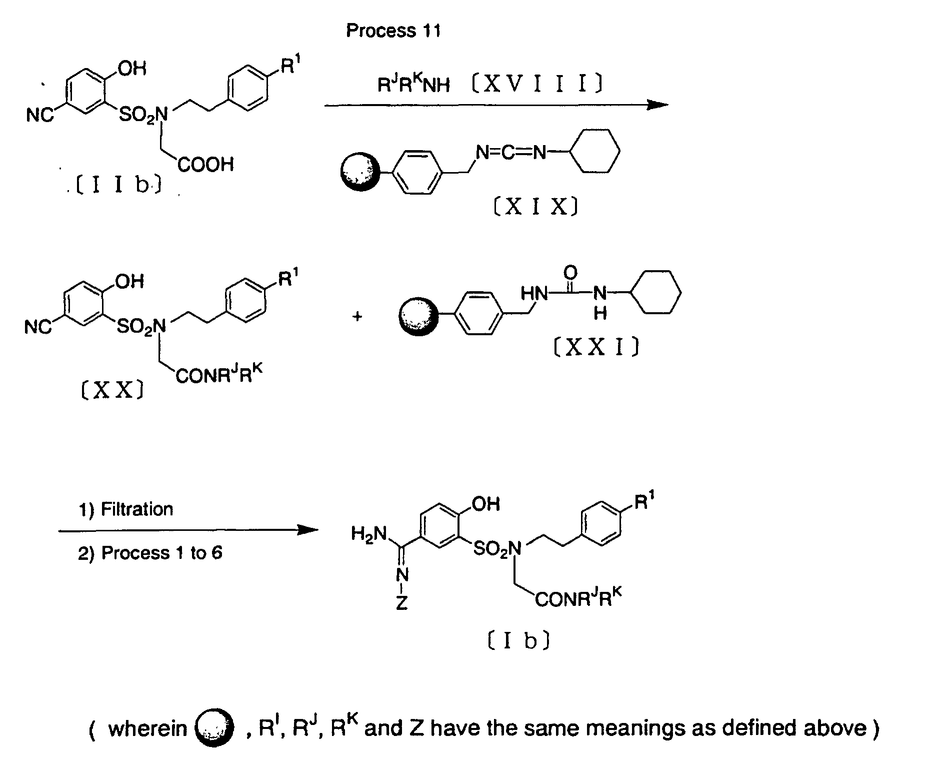

### Process 11

The compound represented by the general formula(IIb) is allowed to react with the amine compound represented by the general formula(XVIII) in the presence of the polymer bound reagent represented by the general formula (XIX) at usually 0 to 80 ºC to give the compound represented by the general formula (XX). The appropriate solvent of the reaction is dichloromethane, *N*,*N*-dimethylformamide, tetrahydrofuran or these combined solvent. Although the reaction time change depends on the startingmaterials and solvents, it is usually for 0.5 to 24hours.

After the process 11, the polymer bound reagents represented by the general formula (XIX) and (XXI) are removed by the filtration and the resulting compound represented by the general formula (XX) can be converted into the compound represented by the general formula (Ib) by subjecting to the process 1 to 6 described above appropriately.

5-cyano-2-hydroxybenzenesulfonamide derivatives represented by the general formula (II), (IIa) and (IIb) utilized as a starting material in the preceding production processes can be for example produced according to the following method. 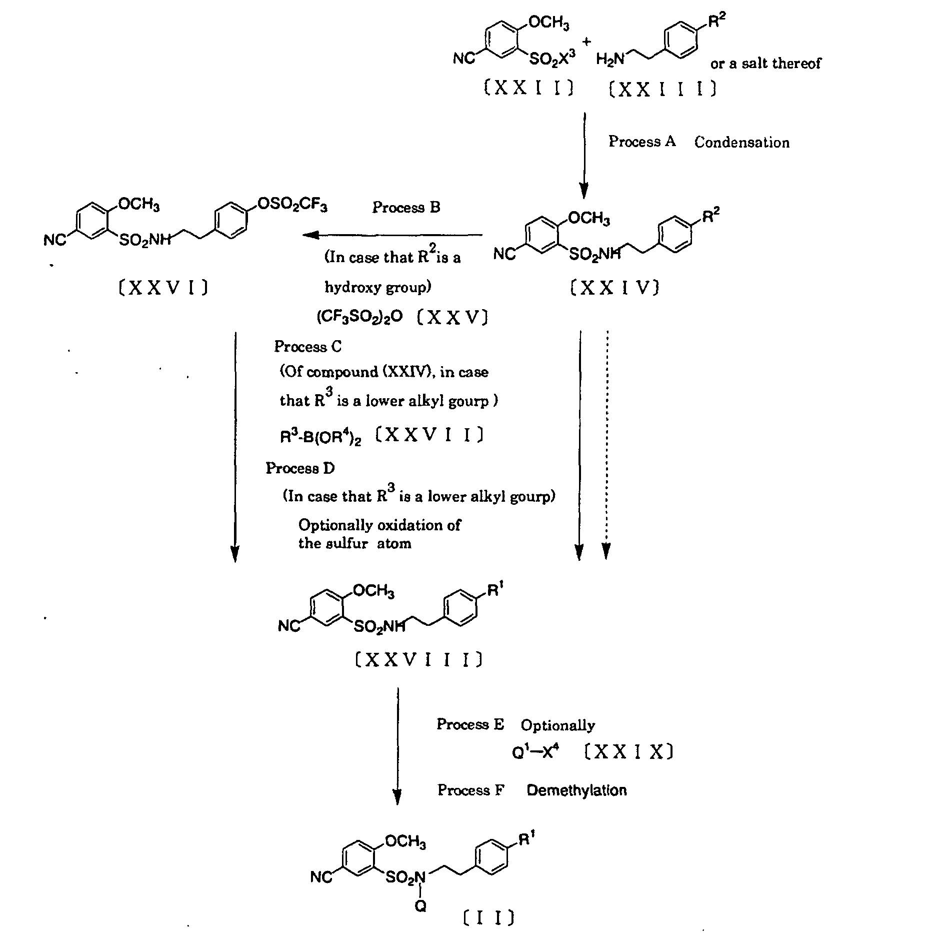 wherein R² represents a halogen atom, a hydroxy group, a lower alkyl group which may have -COOR^{A} in which R^{A} has the same meaning as defined above, a lower alkoxy group which may have -COOR^{A} in which R^{A} has the same meaning as defined above, a lower alkenyl group which may have -COOR^{A} in which R^{A} has the same meaning as defined above, a 3 to 10-membered cycloalkyl group, a lower acyl group, -COORA in which R^{A} has same meaning as defined above, -CONRBRC in which R^{B} and R^{C} are independently a hydrogen atom or a lower alkyl group, or -NR^{B}R^{C} forms a cyclic amino group, an amino group, a mono or di(lower alkyl)amino group, a cyclic amino group which may have an oxo group, a 3 to 10-membered heterocycloalkyl group which may have an oxo group;
R3 represents a 6 to 10-membered aryl group which may have one to three substituents selected from the above group (A) or a 5 to 10-membered aromatic heterocyclic group which may have a substituent selected from the above group (B);
R⁴ represents a hydrogen atom or a lower alkyl group, or both of R⁴ bind to form a lower alkylene group;
X³ represents a leaving group such as a chlorine atom, a bromine atom or an iodine atom;
X⁴ represents a leaving group such as a chlorine atom, a bromine atom, an iodine atom, a methanesulofonyloxy group or a p-toluenesulfonyloxy group;
Q, Q¹ and R¹ have the same meanings as defined above.

### Process A

A compound represented by the above general formula (XXIV) can be prepared by condensing a benzenesulfonyl halide derivative represented by the above general formula (XXII) with a phenethylamine derivative represented by the above general formula (XXIII) or a salt thereof in the presence or absence of a base such as triethylamine or potassium carbonate in a polar solvent such as tetrahydrofuran, *N*,*N*-dimethylformamide, or a mixed solvent of such solvent with water at usually 0°C to room temperature.

### Process B

A compound represented by the above general formula (XXVI) can be prepared by condensing a phenol derivative represented by the above general formula (XXIV) wherein R² is a hydroxy group with tifluoromethanesulfonic anhydride represented by the above formula (XXV) in the presence of a base such as *N,N*-dimethylaminopyridine in a solvent such as dichloromethane or tetrahydrofuran at usually 0°C to room temperature.

### Process C

A compound represented by the below general formula (XXXII) can be prepared by condensing a compound represented by the above general formula (XXIV) or a compound wherein R⁷ is a halogen atom represented by the above general formula (XXIV) with a borane compound represented by the above general formula (XXVII) in the presence of a catalyst such as tetrakis (triphenylphosphine)-palladium(0), palladium(II) acetate or [1,1'-bis(diphenylphosphino)ferrocene]chloronickel(II) and a base such as sodium carbonate, sodium hydrogen carbonate, potassium phosphate or triethylamine in the presence or absence of a phase-transfer catalyst such as tetrabutylammonium bromide in a solvent such as toluene, tatrahydrofuran, *N*,*N*-dimethylformamide or water, or a mixed solvent thereof at usually room temperature to reflux temperature.

### Process D

A compound wherein R³ is a (lower alkyl)thioaryl group represented by the above general formula (XXVII) prepared by the above process C can be converted into a corresponding sulfonyl compound represented by the above general formula (XXVIII) by treating it with an oxidizing agent such as oxone (trademark) or *m*-chloroperbenzoic acid in a solvent such as acetone or dichloromethane, or a mixed solvent of such solvent with water at usually 0°C to reflux temperature.

### Process E

A corresponding *N*-alkylated compound can be prepared by subjecting a compound represented by the above general formula (XXVIII) to *N*-alkylation using an alkylating agent represented by the above general formula (XXIX) in the presence of a base such as triethylamine or potassium carbonate in a solvent such as *N,N*-dimethylformamide at usually -20°C to reflux temperature.

### Process F

A benzenesulfonamide derivative represented by the above general formula (II) can be prepared by subjecting a compound of the above general formula (XXVIII) or a compound *N*-alkylated by Process E to demethylation under heating in the presence of lithium chloride in a solvent such as *N*,*N*-dimethylformamide or *N,N*-dimethylacetamide at usually 100°C to reflux temperature.

In the aforementioned production process, for example, a compound represented by the below general formula (XXXII) can be also prepared by the following method: 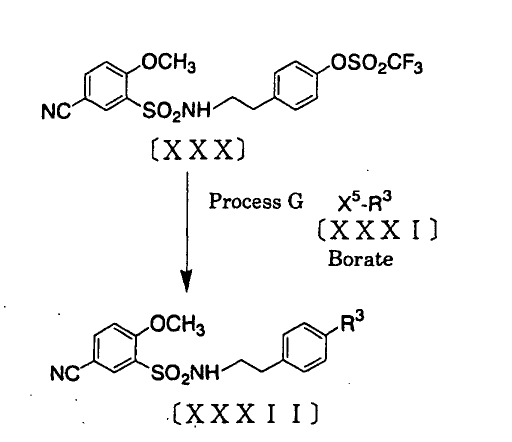 wherein X⁵ represents a bromine atom, a chlorine atom or an iodine atom; R³ has the same meaning as defined above.

### Process G

A benzenesulfonamide derivative represented by the above general formula (XXXII) can be prepared by condensing a benzenesulfonamide derivative represented by the above general formula (XXX) with a halide compound represented by the above general formula (XXXI) in the presence of a borate such as bis(pinacolato)diborane and a catalyst such as [1,1'-bis-(diphenylphosphino)ferrocene]palladium(II) dichloride dichloromethane complex in a solvent such as dioxane at usually room temperature to reflux temperature.

For example, the compound represented by the above general formula (V) in the aforementioned production process is commercially available or can be prepared by methods described in literature or the like (Michael Folkmann, Synthesis, 1159 (1990); Jose Alxander, J.Med.Chem., 318-322, 31 (1988)).

The compounds of the present invention obtained by the above production process can be easily isolated and purified by conventional separation means such as fractional recrystallization, precipitation, purification using column chromatography, solvent extraction and the like.

The 5-amidino-2-hydroxybenzenesulfonamide derivatives represented by the above general formula (I) of the present invention can be converted into their pharmaceutically acceptable salts in the usual way. Examples of the such salts include acid addition salts with mineral acids (e.g., hydrochloric acid, hydrobromic acid, hydroiodic acid, sulfuric acid, nitric acid, phosphoric acid and the like), acid addition salts with organic acids (e.g., formic acid, acetic acid, methanesulfonic acid, benzenesulfonic acid, *p*-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like), salts with organic amines (e.g., morpholine, pyrrolidine, piperidine, piperazine, lysine and the like), and salts with inorganic bases such as a sodium salt, a potassium salt and a calcium salt.

In addition, the compounds represented by the above general formula (I) of the present invention also include its hydrates and solvates with pharmaceutically acceptable solvents (e.g., ethanol).

Of the compounds represented by the above general formula (I) of the present invention, compounds having an asymmetric carbon atom exist in two optical isomer forms of (*R*) configuration and (*S*) configuration. Either one of the isomers or a mixture thereof can be employed in the present invention. In the compounds represented by the above general formula (I) of the present invention, when geometrical isomers or tautomers exist, the present invention includes all of the geometrical isomers and tautomers.

The compounds represented by the above general formula (I) of the present invention are compounds having a potent inhibitory activity on activated blood coagulation factor X and anti-coagulation activity. The compounds represented by the above general formula (I) of the present invention also have an extremely weak inhibitory activity on thrombin and therefore are highly selective activated blood coagulation factor X inhibitors.

The compounds represented by the above general formula (I) of the present invention are selective activated blood coagulation factor X inhibitors. In consequence, the compounds of the present invention are extremely useful as agents for the prevention or treatment of cerebral infarction, cerebral thrombosis, cerebral embolism, transient cerebral ischemic attack (TIA), subarachnoid hemorrhage-induced cerebral vasospasm, alzheimer's disease, myocardial infarction, unstable angina, heart failure, thrombosis followed by atrial fibrillation, pulmonary infarction, pulmonary embolism, acute respiratory distress syndrome (ARDS), Berger disease, peripheral arterial obstruction, deep vein thrombosis, disseminated intravascular coagulation syndrome, atherosclerosis, behcet's disease, diabetic neuropathy, diabetic nephropathy, diabetic retinopathy, diabetic thrombotic complications, acute progressive glomerulonephritis, chronic glomerulonephritis, IgA nephropathy, nephritic syndrome, focal segmental glomerulosclreosis, membranous nephropathy, membranoproliferative glomerulonephritis, crescentic glomerulonephritis, lupus nephritis, purpura nephritis, interplanting rejection, systemic inflammatory response syndrome (SIRS), dialysis- or operation-induced thrombocytopenia, thrombus formation after artificial blood vessel operation or after artificial valve replacement, restenosis and reocculusion after coronary intervention such as percutaneous transluminal coronary angioplasty (PTCA) or percutaneous transluminal coronary recanalization (PTCR) surgery and the like, thrombus formation and the like at the time of extracorporeal circulation and the like, agents for the prevention of blood coagulation at the time of insertion of blood vessel catheter, and agents for the prevention or treatment of influenza virus infection based on the activity to inhibit growth of influenza virus.

In addition, the compounds of the present invention can be used suitably in combination with at least one of the drug with the exception of the activated blood coagulation factor X inhibitors. As examples of drugs which can be used in combination with the compound in the present invention, adrenocortical hormone, platelet aggregation inhibitors, adenylate cyclase activators, PGF2α antagonists, cyclooxygenase inhibitors, adenosine antagonists, GPIIb/IIIa antagonists, anticoagulants and fibrinolytic drugs, antithrombin drugs, free-radical scavengers, immunosuppressant drugs, erythropoietin, fish oil, angiotensin-converting enzyme inhibitors, angiotensin II receptor antagonists, glycation inhibitors, protein kinase C inhibitors, aldose reductase inhibitors, endothelin receptor antagonists, endothelin-converting enzyme inhibitors, neutral endopeptidase inhibitors, thromboxane A₂ synthetase inhibitors, thromboxane A₂ receptor antagonists, PGI₂ agonists and the like can be illustrated.

In case of uses of the compound of the above general formula (I) in combination with the one or more drugs selected from the above groups, the present invention includes either dosage forms of a single preparation or separated preparations for simultaneous administration in way of same or different administration route, or dosage forms separated preparation for administration at different administration intervals in way of same or different administration route. A pharmaceutical composition comprising the compound of the above general formula (I) in combination with the above drugs includes both dosage forms as a single preparation and separated preparations for combination as mentioned above.

It can obtain more advantageous effects beyond additive effects in the prevention or treatment of the above diseases by using the compound of the present invention in combination with suitable one or more drugs selected from the above group. And it can be decrease the administration dose in comparison with administration of the compound of the present invention alone, and can be avoided or declined the adverse effects of co-administrated drugs too.

Example compounds as the above drugs used for combination are listed as follows. However, the present invention is not limited thereto, and it also included a compound of free form and pharmaceutically acceptable salts thereof.

As adrenocortical hormone, cortisone acetate, prednisolone, prednisolone sodium succinate, prednisolone sodium phosphate, methylprednisolone, methylprednisolone acetate, triamcinolone, dexamethasone, dexamethasone metasulfobenzoate sodium, dexamethasone sodium phosphate, betamethasone, betamethasone sodium phosphate, prasterone, KSR-592 and the like are illustrated.

As platelet aggregation inhibitors, dilazep dihydrochloride, dipyridamole, cilostazol, alprostadil, iloprost, cloricromene, triflusal, TA-993 and the like are illustrated.

As adenylate cyclase activators, ticlopidine hydrochloride, colforsin daropate hydrochloride, glucagons, PACAP-38 and the like are illustrated.

As PGF2α antagonists, trimetazidine hydrochloride and the like are illustrated.

As cyclooxygenase inhibitors, aspirin, ketoprofen, tiaprofenic acid, alminoprofen, ibuprofen piconol, flurbiprofen, zaltoprofen, pirprofen, tenoxicam, loxoprofen sodium, oxaprozin, suprofen, fenoprofen, tolfenamic acid, pranoprofen, droxicam, amtolmetin guacil, piroxicam succinate, nabmetone, mofezolac, indobufen, lornoxicam, eltenac, ketorolac trometamol, bromfenac sodium hydrate, aceclofenac, diclofenac sodium, cizolirtine citrate, licofelone, S-14080, D-1158, NMI-377, NMI-172, NMI-246, NMI-267, DP-103, MX-1094 and the like are illustrated.

As adenosine antagonists, clopidogrel sulfate, E-3080 and the like are illustrated.

As GPIIb/IIIa antagonists, abciximab, tirofiban hydrochloride, eptifibatide, sibrafiban, roxifiban acetate, gantofiban, cromafiban, elarofiban, YM-337, T-250, DMP-802, UR-3216, YM-68128, HMR-1794, TAK-024, CRL-42796 and the like are illustrated.

As anticogulants, heparin, warfarin and the like are illustrated.

As thrombolitic drugs, urokinase, streptokinase, t-PA(tissue-type plasminogen activator; tisokinase, alteprase, nateplase, monteplase, pamiteplase, nasaruplase and the like) and the like are illustrated.

As antithrombin drugs, hirudin, argatroban, melagatran, ximelagatran, napsagatran, efegatran, CJC-1004, BIBR-1048, TRI-50B, CX-397, LU-57291 and the like are illustrated.

As free-radical scavengers, edaravone and the like are illustrated.

As immunosuppressant drugs, azathioprine, cyclophosphamide, mizoribine, ciclosporin, tacrolimus hydrate, chlorambucil, lobenzarit disodium, auranofin, alprostadil, gusperimus hydrochloride, biosynsorb, muromonab, alefacept, pentostatin, daclizumab, sirolimus, mycophenolate mofetil, leflonomide, basiliximab, dornase α, bindarid, cladribine, pimecrolimus, ilodecakin, cedelizumab, efalizumab, everolimus, anisperimus, gavilitnomab, faralimomab, clofarabine, siplizumab, saireito, LDP-03, CD4, SR-43551, SK&F-106615, IDEC-114, IDEC-131, FTY-720, TSK-204, LF-080299, A-86281, A-802715, GVH-313, HMR-1279, ZD-7349, IPL-423323, CBP-1011, MT-1345, CNI-1493, CBP-2011, J-695, LJP-920, L-732531, ABX-RB2, AP-1903, IDPS, BMS-205820, BMS-224818, CTLA4-lg, ER-49890, ER-38925, ISAtx-247, RDP-58, PNU-156804, LJP-1082, TMC-95A, TV-4710, PTR-262-MG, AGI-1096 and the like are illustrated.

As angiotensin-converting enzyme inhibitors, lisinopril, ramipril, fosinopril, enalaprilmaleate, captopril, alacepril, delapril hydrochloride, benzapril hydrochloride, quinaprilat, imidapril hydrochloride,zofenopril calcium,fosinopril sodium, cilazapril, temocaprilhydrochloride, spiraprilhydrochloride, quinapril hydrochloride, perindopril erbumine, moexipril hydrochloride, trandolapril, MDL-100240, SA-7060, E-4030, GW-660511 and the like are illustrated.

As angiotensin II receptor antagonists. losartan potassium, valsartan, irbesartan, candesartan cilexetil, eprosartan mesilate, telmisartan, olmesartan, Dup753, PD123177, EXP-3174, EXP-3312, KT-3-671, RU-64276, GA-0113, CS-088 and the like are illustrated.

As glycation inhibitors, pimagedine hydrochloride, ALT-711, EXO-226, KGR-1380, ALT-711andthelikeareillustrated.

As protein kinase C inhibitors, midostaurin, perifosine, LY333531, KW-2401, ISIS-3521, ISIS-5132 and the like are illustrated.

As aldose reductase inhibitors, epalrestat, risarestat, fidarestat, tolrestat, zopolrestat, minalrestat, ascorbyl gamolenate, lindolrestat, AD-5467, AS-3201, NZ-314, SG-210, IDD-598, JTT-811 and the like are illustrated.

As endothelin receptor antagonists, bosentan, sitaxsentan sodium, darusentan, atrasentan, tezosentan sodium, ambrisentan, BMS-207940, BMS-193884, S-0139, BQ-610, TA-0201, SB-215355, SB-234551, SB-247083, J-104132, RO-61-1790, PD-180988, LU-302872, TBC-3214, TBC-3711, RPR-118031A, ABT-546, ATZ-1993, YM-598 and the like are illustrated.

As endothelin-converting enzyme inhibitors, SLV-306, CGS-35066, SM-19712 and the like are illustrated.

As neutral endopeptidase inhibitors, omapatrilat, fasidotril, ecadotril, sampatrilat, MDL-100240, SA-7060, SLV-306, E-4030, GW-660511X and the like are illustrated.

As thromboxane A₂ synthetase inhibitors, sodium ozagrel, ozagrel hydrochloride, isbogrel, terbogrel, imitrodast sodium, imidazol salicylate, NM-702, S-32080, NIK-639 and the like are illustrated.

As thromboxane A₂ receptor antagonists, egualen sodium, seratrodast, ramatroban, epoprostenol sodium, domitroban calcium hydrate, ibudilast, phthalazinol, KT-2-962, Z-335, S-18204, YM-158, S-32080, S-36496, S-35120 and the like are illustrated.

As PGI₂ agonists, beraprost sodium, iloprost, clinprost, pimilprost, TY-11223 and the like are illustrated.

When the 5-amidino-2-hydroxybenzenesulfonamide derivatives represented by the above general formula (I) of the present invention and pharmaceutically acceptable salts thereof are employed in the practical treatment, they are administered orally or parenterally in the form of appropriate pharmaceutical compositions such as tablets, powders, fine granules, granules, capsules, injections, solutions, adhesive preparations, ointments, inhalants, suppositories and the like. These pharmaceutical compositions can be formulated in accordance with pharmaceutically conventional methods using conventional pharmaceutical carriers, excipients and other additives. In case of the use in combination with the drugs other than the 5-amidino-2-hydroxybenzenesulfonamide derivative represented by the above general f ormula (I), they can be prepared by f ormulate each active ingredient together or individually.

When the pharmaceutical compositions of the present invention are employed in the practical treatment, the dosage of the 5-amidino-2-hydroxybenzenesulfonamide derivatives represented by the above general formula (I) or pharmaceutically acceptable salts thereof is appropriately decided depending on the sex, age, body weight, degrees of symptoms and treatment of each patient, which is approximately within the range of from 1 to 5,000 mg per day per adult human in case of oral administration and approximately within the range of from 0.01 to 500 mg per day per adult human in case of parenteral administration such as injection, and the daily dose can be divided into one to several doses per day. Also, in case of the use in combination with the another drugs, the dosage of the 5-amidino-2-hydroxybenzenesulfonamide derivatives represented by the above general formula (I) or pharmaceutically acceptable salts thereof can be decreased appropriately and occasionally depending on the dosage of the another drugs.

### Examples

The present invention is further illustrated in more detail by way of the following Reference Examples, Examples and Test Examples. However, the present invention is not limited thereto.

### Reference Example 1

### (4-Isopropylphenyl)acetonitrile

To a stirred solution of 100 g of 4-isopropylbenzyl chloride in 1500 mL of *N,N*-dimethylformamide was added 32.0 g of sodium cyanide under ice-cooling. The mixture was stirred at 70 ºC for 4 hours, and to the reaction mixture was added water. The mixture was extracted with ethyl acetate, and the organic layer was washed with water, and brine, and dried over anhydrous magnesium sulfate. The solvent was removed under reduced pressure to give 96.5 g of (4-isoopropylphenyl)acetonitrile.
¹H-NMR (CDCl₃) δ ppm:
1.24 (6H, d, J=6.9Hz), 2.91 (1H, sept, J=6.9Hz), 3.70 (2H, s), 7.22-7.27 (4H, m)

### Reference Example 2

The following compounds were prepared according to a similar manner to that described in Example 1

### (4-Cyanomethyl)benzoic acid

¹H-NMR (DMSO-d₆) δ ppm:
4.15 (2H, s), 7.45 (2H, d, J=8.2Hz), 7.95 (2H, d, J=8.2Hz), 12.90 (1H, br s)

### Reference Example 3

### 2-(4-Isopropylphenyl)ethylamine hydrochloride

To a stirred 1000 mL of 1.0 mol/L borane-tetrahydrofuran complex was added a solution of 79.6 g of (4-isopropylphenyl)acetonitrile in 400 mL of tetrahydrofuran under ice-cooling, and the mixture was stirred at room temperature for 2 hours. To the stirred reaction mixture was added 500 mL of methanol over 30 minutes under ice-cooling, and the mixture was stirred at the same temperature for 20 minutes. The reaction mixture was concentrated under reduced pressure, and to the residue were added isopropanol and 500 mL of 2 mol/L hydrochloric acid. The solvent was removed under reduced pressure, the residue was recrystallized from isopropanol-diisopropyl ether to give 41.5 g of 2-(4-isopropylphenyl)ethylamine hydrochloride.
¹H-NMR (DMSO-d₆) δ ppm:
1.18 (6H, d, J=6.9Hz), 2.81-2.92 (3H, m), 2.96-3.05 (2H, m), 7.14-7.26 (4H, m), 8.05 (3H, br s)

### Reference Example 4

### Ethyl 4-(ethoxycarbonylmethyl)benzoate

A solution of 680 mg of 4-(cyanomethyl)benzoic acid and 8.0 mL of 5 mol/L sodium hydroxide solution in 8.0 mL of dimethylsulfoxide was stirred at 110 ºC for 4.5 hours. After the reaction mixture was washed with ethyl acetate, the aqueous layer was acidified with 25 mL of 2 mol/L hydrochloric acid, and the mixture was extracted with ethyl acetate. After the organic layer was washed with brine, dried over anhydrous magnesium sulfate, and the solvent was removed under reduced pressure. To a solution of the obtained residue in 30 mL of ethanol was added 4 drops of concentrated hydrochloric acid. After the mixture was refluxed for 14 hours, the solvent was removed under reduced pressure. The residue was dissolved in ethyl acetate, and the mixture was washed with saturated aqueous sodium bicarbonate solution, and brine. The mixture was dried over anhydrous magnesium sulfate, and the solvent was removed under reduced pressure to give 710 mg of ethyl 4-(ethoxycarbonylmethyl)benzoate.
¹H-NMR (CDCl₃) δ ppm:
1.25 (3H, t, J=6.9Hz), 1.39 (3H, t, J=6.9Hz), 3.68 (2H, s), 4.16 (2H, q, J=6.9Hz), 4.38 (2H, q, J=6.9Hz), 7.36 (2H, d, J=8.2Hz), 8.01 (2H, d, J=8.2Hz)

### Reference Example 5

### Ethyl 4-(carboxymethyl)benzoate

To a solution of 700 mg of 4-(ethoxycarbonylmethyl)benzoate in 4.0 mL of ethanol was added 3.4 mL of 1 mol/L sodium hydroxide solution, and the mixture was stirred at room temperature for 40 minutes. To the reaction mixture was added water, and the mixture was washed with ethyl acetate. The aqueous layer was acidified with 2 mol/L hydrochloric acid, and extracted with ethyl acetate. After the organic layer was washed with brine, dried over anhydrous magnesium sulfate, and the solvent was removed under reduced pressure to give 550 mg of ethyl 4-(carboxymethyl)benzoate.
¹H-NMR (DMSO-d₆) δ ppm:
1.32 (3H, t, J=6.9Hz), 3.68 (2H, s), 4.31 (2H, q, J=6.9Hz), 7.41 (2H, d, J=8.2Hz), 7.90 (2H, d, J=8.2Hz), 12.45 (1H, br s)

### Reference Example 6

### Ethyl 4-(2-hydroxyethyl)benzoate

To a stirred solution of 1.74 g of ethyl 4-(carboxymethyl)benzoate in 70 mL of tetrahydrofuran were added successively 1.4 mL of triethylamine and isobutyl chlorofomate under ice-cooling, and the mixture was stirred at the same temperature for 10 minutes. The insoluble was removed by filtration, and washed with 10 mL of tetrahydrofuran. To the filtrate were added 1.83 g of sodium borohydride, 35 mL of water, and 15 mL of ethanol at room temperature, and the mixture was stirred at 50 ºC for 7.4 hours. To the reaction mixture was added water, and the mixture was extracted with ethyl acetate. The organic layer was washed with aqueous citric acid solution, saturated aqueous sodium bicarbonate solution, and brine, and dried over anhydrous magnesium sulfate. The solvent was removed under reduced pressure to give 1.37 g of ethyl 4-(2-hydroxyethyl)benzoate.
¹H-NMR (CDCl₃) δ ppm:
1.39 (3H, t, J=6.9Hz), 2.93 (2H, t, J=6.6Hz), 3.89 (2H, t, J=6.6Hz), 4.37 (2H, q, J=6.9Hz), 7.30 (2H, d, J=8.2Hz), 7.99 (2H, d, J=8.2Hz)

### Reference Example 7

### Ethyl 4-(2-phtalimidoethyl)benzoate

To a stirred suspension of 1.74 g of ethyl 4-(2-hydroxyethyl)benzoate, 1.32 g of phtalimide, and 3.54 g of triphenylphosphine in 30 mL of tetrahydrofuran was dropped a solution of 5.53 g of 40 % diethyl diazenediyldicarboxylate-toluene solution in 3 mL of tetrahydrofuran at room temperature, and the mixture was stirred at the same temperature under argon atmosphere for 21 hours. The reaction mixture was concentrated under reduced pressure, and the residue was purified by column chromatography on silica gel (eluent: ethyl acetate-hexane) to give 1.84 g of ethyl 4-(2-phtalimidoethyl)benzoate.
¹H-NMR (CDCl₃) δ ppm:
1.38 (3H, t, J=6.9Hz), 3.03-3.09 (2H, m), 3.92-3.98 (2H, m), 4.35 (2H, q, J=6.9Hz), 7.31 (2H, d, J=8.2Hz), 7.68-7.74 (2H, m), 7.80-7.85 (2H, m), 7.96 (2H, d, J=8.2Hz)

### Reference Example 8

### 4-(2-Chloroethyl)-N,N-dimethylaniline hydrochloride

To a stirred solution of 0.50 g of 2-(4-dimethylaminophenyl)ethanol in 15 mL of dichloromethane was added 1.10 mL of thionyl chloride under ice-cooling, and the mixture was stirred at room temperature for 22 hours. After the reaction mixture was concentrated under reduced pressure, to the residue was added ethyl acetate, and the precipitate was collected by filtration to give 0.679 g of 4-(2-chloroethyl)-*N,N*-dimethylaniline hydrochloride.
¹H-NMR (CDCl₃) δ ppm:
3.11 (2H, t, J=6.9Hz), 3.16 (6H, s), 3.73 (2H, t, J=6.9Hz), 7.36-7.41 (2H, m), 7.71-7.76 (2H, m)

### Reference Example 9

### N-[2-(4-Dimethylaminophenyl)ethyl]phtalimide

A mixture of 50 mg of 4-(2-chloroethyl)-*N,N*-dimethylaniline hydrochloride and 46 mg of potassium phtalimide in 1.1 mL of *N,N*-dimethylformamide was stirred at 50 ºC for 3 hours. To the mixture was added 46 mg of potassium phtalimide, and the mixture was stirred at the same temperature for 4 days. To the reaction mixture was added water, and the mixture was extracted with ethyl acetate. The organic layer was washed with water, and brine, and dried over anhydrous magnesium sulfate. After the solvent was removed under reduced pressure, to the residue was added ethanol, and the precipitate was collected by filtration to give 21 mg of *N*-[2-(4-dimethylaminophenyl)-ethyl]phtalimide.
¹H-NMR (CDCl₃) δ ppm:
2.84-2.92 (8H, m), 3.84-3.90 (2H, m), 6.65-6.70 (2H, m), 7.11-7.16 (2H, m), 7.67-7.72 (2H, m), 7.80-7.86 (2H, m)

### Reference Example 10

### Ethyl 4-(2-aminoethyl)benzoate

A suspension of 1.88 g of ethyl 4-(2-phtalimidoethyl)-benzoate and 3.1 mL of hydrazine monohydrate in 100 mL of ethanol was stirred at 70 ºC for 45 minutes. The insoluble was removed by filtration, and the filtrate was concentrated under reduced pressure. To the residue were added ethyl acetate, 1 mol/L hydrochloric acid, and a small amount of ethanol. To the separated aqueous layer was added sodium chloride, and the mixture was extracted with dichloromethane. After the organic layers were combined, dried over anhydrous magnesium sulfate, and the solvent was removed under reduced pressure to give 0.91 g of ethyl 4-(2-aminoethyl)benzoate.
¹H-NMR (CDCl₃) δ ppm:
1.30-1.43 (3H, m), 2.73-2.88 (2H, m), 2.91-3.08 (2H, m), 4.30-4.35 (2H, m), 7.19-7.35 (2H, m), 7.90-8.05 (2H, m)

### Reference Example 11

The following compound was prepared according to a similar manner to that described in Reference Example 10

### 4-(2-Aminoethyl)-N,N-dimethylaniline

¹H-NMR (CDCl₃) δ ppm:
2.00-2.40 (2H, br), 2.69 (2H, t, J=6.9Hz), 2.92 (6H, s), 2.95 (2H, t, J=6.9Hz), 6.68-6.73 (2H, m), 7.05-7.11 (2H, m)

### Reference Example 12

### 5-Carbamoyl-2-methoxybenzenesulfonyl chloride

To 1733 g of chlorosulfonic acid was added in small portions 150 g of 4-methoxybenzamide under ice-cooling with stirring during 15 minutes, and the mixture was stirred at room temperature for 14 hours. After being stirred at 50 ºC for additional 1.5 hours, the reaction mixture was dropped into 7 kg of ice. The precipitate was collected by filtration, washed with water and hexane to give 230 g of 5-carbamoyl-2-methoxybenzenesulfonyl chloride.
¹H-NMR (DMSO-d₆) δ ppm:
3.81 (3H, s), 7.00 (1H, d, J=8.5Hz), 7.10 (1H, br s), 7.84 (1H, dd, J=8.5, 2.5Hz), 7.87 (1H, br s), 8.23 (1H, d, J=2.5Hz)

### Reference Example 13

### 5-Cyano-2-methoxybenzenesulfonyl chloride

5-Carbamoyl-2-methoxybenzenesulfonyl chloride (150 g) was suspended in 1800 mL of ethyl acetate. After 219 mL of thionyl chloride was dropped to the stirred suspension under ice-cooling, 2.3 mL of *N*,*N*-dimethylformamide was added to the mixture. After being stirred at 55 ºC for 3 hours, the reaction mixture was concentrated under reduced pressure. To the residue were added ethyl acetate and water, and the separated organic layer was washed with water, saturated aqueous sodium bicarbonate solution, and brine, and dried over anhydrous magnesium sulfate. The solvent was removed under reduced pressure, and the obtained crude product was recrystallized from ethyl acetate-hexane to give 86.8 g of 5-cyano-2-methoxybenzenesulfonyl chloride.
¹H-NMR (CDCl₃) δ ppm:
4.16 (3H, s), 7.24 (1H, d, J=8.8Hz), 7.96 (1H, dd, J=8.8, 2.2Hz), 8.28 (1H, d, J=2.2Hz)

### Reference Example 14

### 5-Cyano-N-[2-(4-dimethylaminophenyl)ethyl]-2-methoxybenzenesulfonamide

To a stirred solution of 79 mg of 4-(2-aminoethyl)-*N,N*-dimethylaniline and 66 mg of potassium carbonate in tetrahydrofuran (0.87 mL) -water (0.44 mL) was added 0.101 g of 5-cyano-2-methoxybenzenesulfonyl chloride under ice-cooling, and the mixture was stirred at room temperature for 2 hours. To the reaction mixture were added water and ethyl acetate, and the organic layer was separated. After the aqueous layer was extracted with ethyl acetate, the organic layers were combined, and washed with water, and brine. The mixture was dried over anhydrous magnesium sulfate, and the residue was purified by column chromatography on silica gel (eluent: ethyl acetate-hexane) to give 0.131 g of 5-cyano-*N*-[2-(4-dimethylaminophenyl)ethyl]-2-methoxybenzenesulfonamide.
¹H-NMR (CDCl₃) δ ppm:
2.69 (2H, t, J=6.3Hz), 2.92 (6H, s), 3.14 (2H, t, J=6.3Hz), 3.73 (3H, s), 4.77 (1H, t, J=6.3Hz), 6.61-6.60 (2H, m), 6.89-6.95 (2H, m), 6.98 (1H, d, J=8.5Hz), 7.78 (1H, dd, J=8.5, 2.2Hz), 8.18 (1H, d, J=2.2Hz)

### Reference Example 15

### N-(2-Phenylethyl)-2,2,2-trifluoroacetamide

To a stirred solution of 2.42 g of phenethylamine and 3.65 mL of triethylamine in 40 mL of dichloromethane was added 4.5 mL of trifluoroacetic anhydride under ice-cooling, and the mixture was stirred at room temperature for 41 hours. After the solvent was removed under reduced pressure, the residue was dissolved in ethyl acetate. The solution was washed successively with 1 mol/L hydrochloric acid, saturated aqueous sodium bicarbonate solution, and brine, and dried over anhydrous magnesium sulfate. The solvent was removed under reduced pressure to give 4.3 g of N-(2-phenylethyl)-2,2,2-trifluoroacetamide.
¹H-NMR (CDCl₃) δ ppm:
2.89 (2H, t, J=6.7Hz), 3.63 (2H, t, J=6.7Hz), 6.27 (1H, br s), 7.16-7.23 (2H, m), 7.25-7.30 (1H, m), 7.31-7.40 (2H, m)

### Reference Example 16

### N-[2-(4-Acetylphenyl)ethyl]-2,2,2-trifluoroacetamide

To a stirred solution of 1.65 g of *N*-(2-phenylethyl)-2,2,2-trifluoroacetamide and 0.72 mL of acetyl chloride in 40 mL of dichloromethane was added in small portions 3.14 g of aluminum chloride under ice-cooling, and the mixture was stirred at room temperature for 24 hours. To the reaction mixture was added ice under ice-cooling, and the organic layer was separated. The organic layer was washed with 1 mol/L hydrochloric acid, and brine, and dried over anhydrous magnesium sulfate. After the solvent was removed under reduced pressure, and the residue was purified by column chromatography on silica gel (eluent: ethyl acetate-hexane) to give 1.0 g of *N*-[2-(4-acetylphenyl)ethyl]-2,2,2-trifluoroacetamide.
¹H-NMR (CDCl₃) δ ppm:
2.59 (3H, s), 2.97 (2H, t, J=6.6Hz), 3.66 (2H, q, J=6.6Hz), 6.41 (1H, brs), 7.29 (2H, d, J=8.2Hz), 7.92 (2H, d, J=8.2Hz)

### Reference Example 17

### Ethyl 3-[4-[2-(2,2,2-trifluoroacetylamino)ethyl]phenyl]but-2-enoate

To a stirred suspension of 0.23 g of sodium hydride in 20 mL of tetrahydrofuran was dropped 1.15 mL of ethyl (diethoxyphosphoryl)acetate at room temperature, and the mixture was stirred for 2 minutes. To the mixture was added a solution of 1.0 g of *N*-[2-(4-acetylphenyl)ethyl]-2,2,2-trifluoroacetamide in 15 mL of tetrahydrofuran, and the mixture was stirred at 70 ºC for 3.5 hours. To the reaction mixture was added water, and the mixture was extracted with ethyl acetate. The organic layer was washed with brine, and dried over anhydrous magnesium sulfate. After the solvent was removed under reduced pressure, the residue was purified by column chromatography on silica gel (eluent: ethyl acetate-hexane) to give 780 mg of ethyl 3-[4-[2-(2,2,2-trifluoroacetylamino)ethyl]phenyl]-but-2-enoate.
¹H-NMR (CDCl₃) δ ppm:
1.32 (3H, t, J=6.9Hz), 2.57 (3H, s), 2.86-2.97 (2H, m), 3.63 (2H, q, J=6.6Hz), 4.22 (2H, q, J=6.9Hz), 6.09-6.16 (1H, m), 6.34 (1H, br s), 7.20 (2H, d, J=8.2Hz), 7.45 (2H, d, J=8.2Hz)

### Reference Example 18

### 3-Chlorosulfonyl-4-methoxybenzoic acid

To 4 mL of chlorosulfonic acid was added slowly 1.35 mL of ethyl 4-methoxybenzoate under argon atmosphere and ice-cooling, and the mixture was stirred at 80 ºC for 1 hour. To the reaction mixture was added ice-water, and the precipitate was collected by filtration. The obtained solid was washed successively with water, and hexane, and dried to give 1.2 g of 3-chlorosulfonyl-4-methoxybenzoic acid.
¹H-NMR (DMSO-d₆) δ ppm:
3.83 (3H, s), 7.06 (1H, d, J=8.6Hz), 7.90 (1H, dd, J=8.6, 2.6Hz), 8.30 (1H, d, J=2.6Hz), 13.78 (1H, br s)

### Reference Example 19

### 3-[N-[2-[4-(2-Ethoxycarbonyl-1-methylvinyl)phenyl]ethyl]sulfamoyl]-4-methoxybenzoic acid

A suspension of 800 mg of ethyl 3-[4-[2-(2,2,2-trifluoroacetylamino)ethyl]phenyl]but-2-enoate and 2.7 mL of 2 mol/L sodium hydroxide solution in 2.8 mL of ethanol was stirred at room temperature for 55 minutes. To the reaction mixture was added 2.7 mL of 2 mol/L hydrochloric acid, and the mixture was concentrated under reduced pressure. To the residue was added ethanol, and the insoluble was removed by filtration. After the filtrate was concentrated under reduced pressure, the residue was dissolved in water. To the mixture was added sodium bicarbonate to alkalify, and the mixture was extracted with ethyl acetate. The organic layer was washed with brine, dried over anhydrous magnesium sulfate, and the solvent was removed under reduced pressure. To a stirred solution of 500 mg of the obtained residue and 0.665 mL of triethylamine in tetrahydrofuran (6 mL)-water (9 mL) was added in small portions 550 mg of 3-chlorosulfonyl-4-methoxybenzoic acid under ice-cooling, and the mixture was stirred at room temperature for 16 hours. To the reaction mixture was added 1 mol/L hydrochloric acid, and the mixture was extracted with ethyl acetate. The organic layer was washed with brine, and dried over anhydrous magnesium sulfate. The solvent was removed under reduced pressure to give 760 mg of 3-[*N*-[2-[4-(2-ethoxycarbonyl-1-methylvinyl)phenyl]-ethyl]sulfamoyl]-4-methoxybenzoic acid.
¹H-NMR (DMSO-d₆) δ ppm:
1.24 (3H, t, J=6.9Hz), 2.48 (3H, d, J=1.3Hz), 2.66-2.73 (2H, m), 2.98-3.11 (2H, m), 3.92 (3H, s), 4.14 (2H, q, J=6.9Hz), 6.11 (1H, d, J=1.3Hz), 7.15 (2H, d, J=8.2Hz), 7.27 (1H, d, J=8.5Hz), 7.44 (2H, d, J=8.2Hz), 7.48 (1H, t, J=5.7Hz), 8.12 (1H, dd, J=8.5, 1.9Hz), 8.26 (1H, d, J=1.9Hz), 13.06 (1H, br s)

### Reference Example 20

### 3-[N-[2-(4-Ethoxycarbonylphenyl)ethyl]sulfamoyl]-4-methoxybenzoic acid

To a stirred suspension of 0.90 g of ethyl 4-(2-aminoethyl)benzoate and 1.4 mL of triethylamine in tetrahydrofuran (8 mL)-water (8 mL) was added in small portions 1.22 g of 3-chlorosulfonyl-4-methoxybenzoic acid, and the mixture was stirred at room temperature for 18 hours. To the reaction mixture was added 1 mol/L hydrochloric acid, and the mixture was extracted with ethyl acetate. The organic layer was washed with brine, and dried over anhydrous magnesium sulfate. The solvent was removed under reduced pressure, and to the residue was added ethyl acetate. The mixture was triturated, and the precipitate was collected by filtration to give 0.67 g of 3-[*N*-[2-(4-ethoxycarbonylphenyl)ethyl]sulfamoyl]-4-methoxybenzoic acid.
¹H-NMR (DMSO-d₆) δ ppm:
1.31 (3H, t, J=7.1Hz), 2.75 (2H, t, J=6.9Hz), 3.05-3.15 (2H, m), 3.93 (3H, s), 4.29 (2H, q, J=7.1Hz), 7.22-7.30 (3H, m), 7.53 (1H, t, J=5.7Hz), 7.79 (2H, d, J=8.3Hz), 8.10 (1H, dd, J=8.2, 2.2Hz), 8.23 (1H, d, J=2.2Hz), 13.04 (1H, br s)

### Reference Example 21

### Ethyl 3-[4-[2-(5-carbamoyl-2-methoxybenzenesulfonylamino)ethyl]phenyl]but-2-enoate

To a suspension of 750 mg of 3-[*N*-[2-[4-(2-ethoxycarbonyl-1-methylvinyl)phenyl]ethyl]sulfamoyl]-4-metoxybenzoic acid, 385 mg of 1-hydroxybenzotirazole monohydrate, and 352 mg of ammonium chloride in 21 ml of *N,N*-dimethylformamide were added successively 0.935 mL of triethylamine, and 482 mg of 1-[3-(dimethylamino)propyl]-3-ethylcarbodiimide hydrochloride, and the mixture was stirred at room temperature for 19 hours. To the reaction mixture was added water, and the mixture was stirred at 10 minutes. After the mixture was extracted with ethyl acetate, the organic layer was washed with 1 mol/L hydrochloric acid, water, and brine, and dried over anhydrous magnesium sulfate. The solvent was removed under reduced pressure to give 620 mg of ethyl 3-[4-[2-(5-carbamoyl-2-methoxybenzenesulfonylamino)ethyl]phenyl]-but-2-enoate.
¹H-NMR (DMSO-d₆) δ ppm:
1.23 (3H, t, J=6.9Hz), 2.48 (3H, d, J=1.6Hz), 2.65-2.74 (2H, m), 2.96-3.08 (2H, m), 3.90 (3H, s), 4.13 (2H, q, J=6.9Hz), 6.12 (1H, d, J=1.6Hz), 7.15 (2H, d, J=8.2Hz), 7.24 (1H, d, J=8.5Hz), 7.31-7.41 (2H, m), 7.46 (2H, d, J=8.2Hz), 8.05 (1H, brs), 8.10 (1H, dd, J=8.5, 2.2Hz), 8.26 (1H, d, J=2.2Hz)

### Reference Example 22

The following compound was prepared according to a similar manner to that described in Reference Example 21

### Ethyl 4-[2-(5-carbamoyl-2-methoxybenzenesulfonylamino)ethyl]benzoate

¹H-NMR (DMSO-d₆) δ ppm:
1.31 (3H, t, J=7.1Hz), 2.72-2.78 (2H, m), 3.02-3.10 (2H, m), 3.91 (3H, s), 4.29 (2H, q, J=7.1Hz), 7.23 (1H, d, J=8.8Hz), 7.26 (2H, d, J=8.5Hz), 7.36 (1H, brs), 7.42(1H, t, J=6.0Hz), 7.80-7.84 (2H, m), 8.05 (1H, br s), 8.09 (1H, dd, J=8.2, 2.2Hz), 8.25 (1H, d, J=2.2Hz)

### Reference Example 23

### 3-[N-[2-(4-Bromophenyl)ethyl]sulfamoyl]-4-methoxybenzamide

To a suspension of 4.89 g of 2-(4-bromophenyl)ethylamine and 6.76 mL of triethylamine in 25 mL of *N*,*N*-dimethylformamide was added in small portions 6.1 g of 5-carbamoyl-2-methoxybenzenesulfonamide at room temperature, and the mixture was stirred at the same temperature for 1 hour. To the reaction mixture was added water, and the mixture was stirred at room temperature for 30 minutes. The precipitate was collected by filtration, and washed with water to give 7.31 g of 3-[*N*-[2-(4-bromophenyl)ethyl]sulfamoyl]-4-methoxybenzamide.
¹H-NMR (CDCl₃) δ ppm:
2.75 (2H, t, J=6.9Hz), 3.09-3.19 (2H, m) , 3.85 (3H, s), 5.74-5.84 (1H, m), 6.12 (1H, br s), 6.96-7.01 (2H, m), 7.03 (1H, d, J=8.8Hz), 7.35-7.40 (2H, m), 7.53 (1H, br s), 8.17 (1H, dd, J=8.8, 2.2Hz), 8.40 (1H, d, J=2.2Hz)

### Reference Example 24

The following compound was prepared according to a similar manner to that described in Reference Example 23

### 3-[N-[2-(4-isopropylphenyl)ethyl]sulfamoyl]-4-methoxybenzamide

¹H-NMR (CDCl₃) δ ppm:
1.23 (6H, d, J=6.9Hz), 2.76 (2H, t, J=6.3Hz), 2.88 (1H, sept, J=6.9Hz), 3.11-3.17 (2H, m), 3.68 (3H, s), 4.91 (1H, t, J=6.0Hz), 5.50-6.50 (2H, br), 6.98-7.02 (3H, m), 7.15 (2H, d, J=8.2Hz), 8.16 (1H, dd, J=8.8, 2.2Hz), 8.26 (1H, d, J=2.2Hz)

### Reference Example 25

### N-tert-Butylbenzenesulfonamide

To a stirred suspension of 4.0 mL of *tert*-butylamine and 5.77 g of potassium carbonate in tetrahydrofuran (9.0 mL)-water (20 mL) was added dropwise a solution of 4 . 85mL of benzenesulfonyl chloride in 1.0 mL of tetrahydrofuran over 3 minutes under ice-cooling, and the mixture was stirred at the same temperature for 1.2 hours. To the mixture was added 0.5 mL of tert -butylamine, and the mixture was stirred for additional 40 hours. To the reaction mixture was added ethyl acetate, and the organic layer was separated. The organic layer was washed successively with water, saturated aqueous sodium bicarbonate solution, 1mol/L hydrochloric acid, and brine, and dried over anhydrous magnesium sulfate. The solvent was removed under reduced pressure to give 7.90 g of *N*-*tert*-butylbenzenesulfonamide.
¹H-NMR (CDCl₃) δ ppm:
1.23 (9H, s), 4.59 (1H, br s), 7.45-7.57 (3H, m), 7.87-7.93 (2H, m)

### Reference Example 26

### 2-(tert-Butylsulfamoyl)phenyl(dihydroxy)borane

To a stirred solution of 0.30 g of N-(tert-butyl)-benzenesulfonamide in 5.0 mL of tetrahydrofuran was added dropwise 2.21 mL of 1.5 mol/L n-butyl lithium-hexane solution over 4 minutes under ice-cooling and argon atmosphere. The temperature of the reaction mixture was turned to room temperature gradually, and stirred for 1. 5 hours. To the stirred reaction mixture were added 0.40 mL of triisopropyl borate and 2.0 mL of tetrahydrofuran, and the mixture was stirred at the same temperature for 2.5 hours. To the reaction mixture was added 2.3 mL of 1 mol/L hydrochloric acid, and the mixture was stirred at the same temperature for 30 minutes. To the reaction mixture was added water, and the mixture was extracted with diethyl ether. The organic layer was extracted with 1 mol/L sodium hydroxide solution, and the aqueous layer was acidified by addition of 6 mol/L hydrochloric acid. After the mixture was extracted with ethyl acetate, the organic layer was washed with brine, and dried over anhydrous magnesium sulfate. The solvent was removed under reduced pressure to give 0.180 g of 2-(tert-butylsulfamoyl)phenyl(dihydroxy)borane.
¹H-NMR (CDCl₃) δ ppm:
1.19 (9H, s), 5.01 (1H, br s), 6.03 (2H, br s), 7.52 (1H, td, J=7.3, 1.3Hz), 7.56 (1H, td, J=7.3, 1.3Hz), 7.85 (1H, dd, J=7.3, 1.3Hz), 8.02 (1H, dd, J=7.3, 1.3Hz)

### Reference Example 27

### 3-[N-[2-(2'-tert-Butylsulfamoylbiphenyl-4-yl)ethyl]sulfamoyl]-4-methoxybenzamide

A suspension of 0.50 g of 2-(*tert*-butylsulfamoyl)phenyl(dihydroxy)borane, 0.802 g of 3-[*N*-[2-(4-bromophenyl)ethyl]sulfamoyl]-4-methoxybenzamide, 0.224 g of tetrakis(triphenylphosphine)palladium(0), 62 mg of tetrabutylammonium bromide, and 0.441 g of sodium carbonate in water (1.9 mL)-benzene (12 mL) was stirred at 90 ºC for 8 hours. To the mixture was added 0.205 g of sodium carbonate, and the mixture was stirred at the same temperature for 4 hours. To the reaction mixture were added 1 mol/L hydrochloric acid and a small amount of methanol, and the mixture was extracted with ethyl acetate. The organic layer was washed with water, and brine, and dried over anhydrous magnesium sulfate. The solvent was removed under reduced pressure, and the residue was purified by column chromatography on silica gel (eluent: ethyl acetate - hexane) to give 0.641 g of 3-[*N*-[2-(2'-tert-butylsulfamoylbiphenyl-4-yl)ethyl]sulfamoyl]-4-methoxybenzamide.
¹H-NMR (CDCl₃) δ ppm:
1.01 (9H, s), 2.87 (2H, t, J=6.6Hz), 3.23 (2H, q, J=6.6Hz), 3.68 (1H, br s), 3.95 (3H, s), 4.96 (1H, t, J=6.6Hz), 5.30-6.50 (2H, br), 7.10 (1H, d, J=8.5Hz), 7.20 (2H, d, J=8.2Hz), 7.25-7.29 (1H, m), 7.44 (2H, d, J=8.2Hz), 7.49 (1H, td, J=7.6, 1.3Hz). 7.56 (1H, td, J=7.6, 1.3Hz), 8.17 (1H, dd, J=7.6, 1.3Hz), 8.19 (1H, dd, J=8.5, 2.2Hz), 8.27 (1H, d, J=2.2Hz)

### Reference Example 28

### 2-Chloroethyl [4-(2-hydroxyethyl)phenyl]carbamate

To a stirred solution of 10 g of 2-(4-aminophenyl)ethanol and 6.43 g of sodium bicarbonate in dichloromethane (140 mL)-water (110 mL) was added 7.93 mL of 2-chloroethyl chloroformate under ice-cooling. The mixture was stirred at the same temperature for 10 minutes, and at room temperature for 5 hours. To the reaction mixture was added water, and the mixture was extracted with dichloromethane. The organic layer was washed with water, and brine, and dried over anhydrous magnesium sulfate. The solvent was removed under reduced pressure, and the residue was purified by column chromatography on silica gel (eluent: ethyl acetate-hexane) to give 17.6 g of 2-chloroethyl [4-(2-hydroxyethyl)phenyl]carbamate.
¹H-NMR (DMSO-d₆) δ ppm:
2.61-2.68 (2H, m), 3.50-3.58 (2H, m), 3.83-3.89 (2H, m), 4.30-4.36 (2H, m), 4.56-4.62 (1H, m), 7.08-7.14 (2H, m), 7.32-7.40 (2H, m), 9.70 (1H, br s)

### Reference Example 29

### 3-[4-(2-Hydroxyethyl)phenyl]-1,3-oxazolidin-2-one

A solution of 5.0 g of 2-chloroethyl [4-(2-hydroxyethyl)-phenyl]carbamate and 4.45 g of potassium hydroxide in 100 mL of ethanol was stirred at 50 ºC for 2 hours. After being cooled to room temperature, the reaction mixture was stirred for 30 minutes, and to the mixture was added water. After the mixture was extracted with ethyl acetate, the organic layer was washed with water, and brine, and dried over anhydrous magnesium sulfate. The solvent was removed under reduced pressure, and the residue was purified by column chromatography on silica gel (eluent: ethyl acetate) to give 4.14 g of 3-[4-(2-hydroxyethyl)phenyl]-1,3-oxazolidin-2-one.
¹H-NMR (DMSO-d₆) δ ppm:
2.65-2.73 (2H, m), 3.54-3.61 (2H, m), 3.98-4.05 (2H, m), 4.38-4.44 (2H, m), 7.19-7.25 (2H, m), 7.42-7.47 (2H, m)

### Reference Example 30

### 2-[4-(2-Oxo-1,3-oxazolidin-3-yl)phenyl]ethyl methanesulfonate

A suspension of 1.14 g of 3-[4-(2-hydroxyethyl)phenyl]-1,3-oxazolidin-2-one, 0.639 mL of methanesulfonyl chloride, and 4.4 g of 4-(diisopropylaminomethyl)polystylene (3.75 mmol/g) in 13 mL of dichloromethane was stirred at room temperature for 1.5 hours. The insoluble material was removed by filtration thorough celite, and washed with dichloromethane. The filtrate was concentrated under reduced pressure to give 0.515 g of 2-[4-(2-oxo-1,3-oxazolidin-3-yl)phenyl]ethyl methanesulfonate.
¹H-NMR (CDCl₃) δ ppm:
2.89 (3H, s), 3.01-3.07 (2H, m), 4.03-4.09 (2H, m), 4.37-4.43 (2H, m), 4.46-4.52 (2H, m), 7.23-7.28 (2H, m), 7.49-7.53 (2H, m)

### Reference Example 31

### 4-Methoxy-3-sulfamoylbenzamide

To a stirred solution of 3.0 mL of 28 % aqueous ammonia solution in tetrahydrofuran (35 mL)-water (15 mL) was added in small portions 5.0 g of 5-carbamoyl-2-methoxybenzenesulfonyl chloride under ice-cooling, and the mixture was stirred at room temperature for 1.5 hours. After to the reaction mixture was added 100 mL of water, the insoluble was removed by filtration, and washed successively with water, and hexane to give 3.84 g of 4-methoxy-3-sulfamoylbenzamide.
¹H-NMR (DMSO-d₆) δ ppm:
3.96 (3H, s), 7.13 (2H, br s), 7.26 (1H, d, J=8.5Hz), 7.32 (1H, br s), 8.03 (1H, br s), 8.09 (1H, dd, J=8.5, 2.2Hz), 8.29 (1H, d, J=2.2Hz)

### Reference Example 32

### 4-Methoxy-3-[N-[2-[4-(2-oxo-1,3-oxazolidin-3-yl)phenyl]ethyl]sulfamoyl]benzamide

A suspension of 1.357 g of 4-methoxy-3-sulfamoylbenzamide, 1.766 g of 2-[4-(2-oxo-1,3-oxazolidin-3-yl)phenyl]ethyl methanesulfonate, and 2.209 g of cesium carbonate in 20 mL of *N,N*-dimethylformamide was stirred 75 ºC for 12 hours. To the reaction mixture was added water, and the mixture was extracted with ethyl acetate. The organic layer was washed with water. and brine, and dried over anhydrous magnesium sulfate. The solvent was removed under reduced pressure to give 4-methoxy-3-[*N*-[2-[4-(2-oxo-1,3-oxazolidin-3-yl)phenyl]ethyl]sulfamoyl]benzamide.
¹H-NMR (DMSO-d₆) δ ppm :
2.61-2.67 (2H, m), 2.96-3.05 (2H, m), 3.91 (3H, s), 3.97-4.05 (2H, m), 4.37-4.46 (2H, m), 7.09-7.57 (6H, m), 8.01-8.14 (3H, m), 8.24 (1H, d, J=2.2Hz)

### Reference Example 33

### Ethyl 3-[4-[2-(5-cyano-2-methoxybenzenesulfonylamino)ethyl]phenyl]but-2-enoate

To a suspension of 600 mg of ethyl 3-[4-[2-(5-carbamoyl-2-methoxybenzenesulfonylamino)ethyl]phenyl]but-2-enoate and 0.75 mL of triethylamine in 10 mL of dichloromethane was added 0.38 mL of trifluoroacetic anhydride, and the mixture was stirred at room temperature for 5.5 hours. To the mixture was added 0.11 mL of methanol, and the mixture was stirred at the same temperature for 30 minutes. The solvent was removed under reduced pressure, and the residue was dissolved in ethyl acetate. The solution was washed with 1 mol/L hydrochloric acid, and brine, and dried over anhydrous magnesium sulfate. The solvent was removed under reduced pressure to give 470 mg of ethyl 3-[4-[2-(5-cyano-2-methoxybenzenesulfonylamino)ethyl]phenyl ]but-2-enoate.
¹H-NMR (CDCl₃) δ ppm:
1.32 (3H, t, J=6.9Hz), 2.54-2.57 (3H, m), 2.82 (2H, t, J=6.9Hz), 3.22 (2H, q, J=6.9Hz), 3.78 (3H, s), 4.22 (2H, q, J=6.9Hz), 4.81-4.90 (1H, m), 6.10 (1H, d, J=1.3Hz), 7.01 (1H, d, J=8.8Hz), 7.10 (2H, d, J=8.2Hz), 7.40 (2H, d, J=8.2Hz), 7.81 (1H, dd, J=8.8, 2.2Hz), 8.20 (1H, d, J=2.2Hz)

### Reference Example 34

The following compounds were prepared according to a similar manner to that described in Reference Example 33

### 5-Cyano-N-[2-(4-isopropylphenyl)ethyl]-2-methoxybenzenesulfonamide

¹H-NMR (CDCl₃) δ ppm:
1.23 (6H, d, J=6.9Hz), 2.76 (2H, t, J=6.3Hz), 2.89 (1H, sept, J=6.9Hz), 3.14-3.21 (2H, m), 3.71 (3H, s), 4.75-4.85 (1H, m), 6.96-7.02 (3H, m), 7.15 (2H, d, J=8.2Hz), 7.80 (1H, dd, J=8.5, 2.2Hz), 8.21 (1H, d, J=2.2Hz)

### Ethyl 4-[2-(5-cyano-2-methoxybenzenesulfonylamino)ethyl]benzoate

¹H-NMR (CDCl₃) δ ppm:
1.41 (3H, t, J=7.1Hz), 2.87 (2H, t, J=6.6Hz), 3.20-3.30 (2H, m), 3.79 (3H, s), 4.39 (2H, q, J=7.1Hz), 4.83 (1H, t, J=6.0Hz), 7.01 (1H, d, J=8.7Hz), 7.16 (2H, d, J=8.2Hz), 7.80 (1H, dd, J=8.7, 2.2Hz), 7.94 (2H, d, J=8.2Hz), 8.18 (1H, d, J=2.2Hz)

### 5-Cyano-2-methoxy-N-[2-[4-(2-oxo-1,3-oxazolidin-3-yl)phenyl]ethyl]benzenesulfonamide

¹H-NMR (DMSO-d₆) δ ppm:
2.60-2.69 (2H, m), 3.05-3.13 (2H, m), 3.94 (3H, s), 3.98-4.07 (2H, m), 4.38-4.47 (2H, m), 7.08-7.14 (2H, m), 7.33 (1H, d, J=8.5Hz), 7.36-7.42 (2H, m) , 7.62 (1H, br s), 7.97 (1H, d, J=2.2Hz), 8.04 (1H, dd, J=8.5, 2.2Hz)

*N*-tert-Butyl-4'-[2-(5-cyano-2-methoxybenzenesulfonylamino)ethyl]biphenyl-2-sulfonamide
¹H-NMR (DMSO-d₆) δ ppm:
0.96 (9H, s), 2.70-2.77 (2H, m), 3.05-3.13 (2H, m), 3.99 (3H, s), 6.34 (1H, s), 7.16 (2H, d, J=7.9Hz), 7.23-7.30 (3H, m), 7.38-7.43 (1H, m), 7.54 (1H, td, J=7.6, 1.3Hz), 7.61 (1H, td, J=7.6, 1.3Hz), 7.66 (1H, t, J=5.7Hz), 8.03 (1H, dd, J=7.6, 1.3Hz), 8.06-8.11 (2H, m)

### Reference Example 35

### 4-[2-(5-Cyano-2-methoxybenzenesulfonylamino)ethyl]benzoic acid

A suspension of 330 mg of ethyl 4-[2-(5-cyano-2-methoxybenzenesulfonylamino)ethyl]benzoate and 1.9 mL of 1 mol/L sodium hydroxide solution in 1.9 mL of ethanol was stirred at room temperature for 2 hours, and at 55 ºC for 3 hours. To the reaction mixture was added 0.15 mL of 1 mol/L sodium hydroxide solution, and the mixture was stirred at the same temperature for 30 minutes. After the solvent was removed under reduced pressure, to the residue was added 1 mol/L hydrochloric acid to acidify, and the mixture was extracted with dichloromethane. The organic layer was washed with brine, and dried over anhydrous magnesium sulfate. The solvent was removed under reduced pressure to give 0.26 g of 4-[2-(5-cyano-2-methoxybenzenesulfonylamino)ethyl]benzoic acid.
¹H-NMR (DMSO-d₆) δ ppm:
2.74 (2H, t, J=6.9Hz), 3.08-3.17 (2H, m), 3.94 (3H, s), 7.23 (2H, d, J=8.2Hz), 7.34 (1H, d, J=8.8Hz), 7.68 (1H, t, J=5.7Hz), 7.77-7.82 (2H, m), 8.01 (1H, d, J=2.2Hz), 8.04 (1H, dd, J=8.8, 2.2Hz), 12.80 (1H, br s)

### Reference Example 36

### 5-Cyano-2-methoxy-N-[2-[4-(pyrrolidin-1-ylcarbonyl)phenyl]ethyl]benzenesulfonamide

To a stirred solution of 0.122 g of 4-[2-(5-cyano-2-methoxybenzenesulfonylamino)ethyl]benzoic acid, 0.078 g of 1-hydroxybenzotirazole monohydrate, and 0.065 mL of pyrrolidine in 2 ml of *N,N*-dimethylformamide were added successively 0.109 mL of triethylamine, and 0.097 g of 1-[3-(dimethylamino)-propyl]-3-ethylcarbodiimide hydrochloride at room temperature, and the mixture was stirred at the same temperature for 4.6 hours. To the reaction mixture was added water, and the mixture was extracted with ethyl acetate. The organic layer was washed with 1 mol/L hydrochloric acid, saturated aqueous sodium bicarbonate solution, and brine, and dried over anhydrous magnesium sulfate. The solvent was removed under reduced pressure to give 0.085 g of 5-cyano-2-methoxy-*N*-[2-[4-(pyrrolidin-1-ylcarbonyl)phenyl]ethyl]benzenesulfonamide.
¹H-NMR (CDCl₃) δ ppm:
1.85-2.02 (4H, m), 2.84 (2H, t, J=6.6Hz), 3.17-3.26 (2H, m), 3.41 (2H, t, J=6.6Hz), 3.64 (2H, t, J=6.9Hz), 3.77 (3H, s), 4.72-4.76 (1H, m), 7.01 (1H, d, J=8.8Hz), 7.10 (2H, d, J=8.2Hz), 7.40-7.44 (2H, m), 7.80 (1H, dd, J=8.8, 2.2Hz), 8.18 (1H, d, J=2.2Hz)

### Reference Example 37

### Ethyl [(5-cyano-2-methoxybenzenesulfonyl)-[2-(4-isopropylphenyl)ethyl]amino]acetate

To a stirred solution of 0.20 g of 5-cyano-*N*-[2-(4-isopropylphenyl)ethyl]-2-methoxybenzenesulf onamide in 1.7 mL of *N,N*-dimethylformamide were added successively 85 mg of potassium carbonate and 0.068 mL of ethyl bromoacetate under ice-cooling, and the mixture was stirred at room temperature for 17 hours. To the reaction mixture was added water, and the mixture was extracted with ethyl acetate. The organic layer was washed with water, and brine, and dried over anhydrous magnesium sulfate. The solvent was removed under reduced pressure to give 236 mg of ethyl [(5-cyano-2-methoxybenzenesulfonyl)-[2-(4-isopropylphenyl)ethyl]amino]acetate.
¹H-NMR (CDCl₃) δ ppm:
1.21 (3H, t, J=7.3Hz), 1.23 (6H, d, J=6.9Hz), 2.78-2.91 (3H, m), 3.56-3.64 (2H, m), 3.91 (3H, s), 4.06-4.12 (4H, m), 6.99 (1H, d, J=8.5Hz), 7.04 (2H, d, J=8.2Hz), 7.11 (2H, d, J=8.2Hz), 7.76 (1H, dd, J=8.5, 2.2Hz), 8.23 (1H, d, J=2.2Hz)

### Reference Example 38

The following compound was prepared according to a similar manner to that described in Reference Example 37

### 5-Cyano-N-[2-(4-isopropylphenyl)ethyl]-2-methoxy-N-methylbenzenesulfonamide

¹H-NMR (CDCl₃) δ ppm:
1.23 (6H, d, J=6.9Hz), 2.81-2.91 (6H, m), 3.37-3.42 (2H, m), 3.95 (3H, s), 7.02 (1H, d, J=8.5Hz), 7.05-7.15 (4H, m), 7.76 (1H, dd, J=8.5, 2.2Hz), 8.22 (1H, d, J=2.2Hz)

### Reference Example 39

### 1,3-Thiazol-2-ylmethanol

To a stirred solution of 1.00 g of 1,3-thiazole-2-carboxaldehyde in 10 mL of tetrahydrofuran was added 0.334 g of sodium borohydride at room temperature. To the mixture was dropped 1 mL of methanol, and the mixture was stirred at the same temperature for 30 minutes. To the reaction mixture was added water, and the mixture was extracted with ethyl acetate. The organic layer was washed with brine, and dried over anhydrous magnesium sulfate. The solvent was removed under reduced pressure to give 0.344 g of 1,3-thiazol-2-ylmethanol. ¹H-NMR (CDCl₃) δ ppm:
3.11 (1H, br s), 4.97 (2H, s), 7.32 (1H, d, J=3.2Hz), 7.75 (1H, d, J=3.2Hz)

### Reference Example 40

### 5-Cyano-N-[2-(4-isopropylphenyl)ethyl]-2-methoxy-N-(1,3-thiazol-2-ylmethyl)benzenesulfonamide

To a stirred solution of 0.454 g of 1,3-thiazol-2-ylmethanol, 1.41 g of 5-cyano-2-methoxy-*N*-[2-(4-isopropylphenyl)ethyl]-benzenesulfonamide, and 1.55 g of triphenylphosphine in 30 mL of tetrahydrofuran was added dropwise 3.08 mL of 40 % diisopropyl diazenediyldicarboxylate - toluene solution under ice-cooling, and the mixture was stirred at room temperature for 4 hours. To the reaction mixture was added saturated aqueous sodium bicarbonate solution, and the mixture was extracted with ethyl acetate. The organic layer was washed with brine, and dried over anhydrous magnesium sulfate. After the solvent was removed under reduced pressure, to the residue was added diluted hydrochloric acid to acidify, and the solvent was removed under reduced pressure. The residue was suspended with ethyl acetate, and the insoluble material was collected by filtration. After the obtained solid was suspended with ethyl acetate, to the mixture was added saturated aqueous sodium bicarbonate solution, and the mixture was extracted with ethyl acetate. The organic layer was washed with brine, and dried over anhydrous magnesium sulfate. The solvent was removed under reduced pressure to give 0.80 g of 5-cyano-*N*-[2-(4-isopropylphenyl)ethyl]-2-methoxy-*N*-(1,3-thiazol-2-ylmethyl)benzenesulfonamide.
¹H-NMR (DMSO-d₆) δ ppm:
1.16 (6H, d, J=6.9Hz), 2.61-2.71 (2H, m), 2.81 (1H, sept, J=6.9Hz), 3.41-3.54 (2H, m), 3.92 (3H, s), 4.87 (2H, s), 6.97 (2H, d, J=7.9Hz), 7.07 (2H, d, J=7.9Hz), 7.35 (1H, d, J=8.8Hz), 7.71 (1H, d, J=3.2Hz), 7.75 (1H, d, J=3.2Hz), 8.09 (1H, dd, J=8.8, 2.2Hz), 8.13 (1H, d, J=2.2Hz)

### Reference Example 41

### 5-Cyano-N-(2-hydroxyethyl)-N-[2-(4-isopropylphenyl)ethyl]-2-methoxybenzenesulfonamide

To a stirred solution of 230 mg of Ethyl [(5-cyano-2-methoxybenzenesulfonyl)-[2-(4-isopropylphenyl)e thyl]amino]acetate in 2.5 mL of ethanol was added 59 mg of sodium borohydride under ice-cooling, and the mixture was stirred at room temperature for 2 days. To the stirred reaction mixture was added 1 mol/L hydrochloric acid under ice-cooling, and the mixture was extracted with ethyl acetate. The organic layer was washed with 1 mol/L hydrochloric acid, water, and brine, and dried over anhydrous magnesium sulfate. The solvent was removed under reduced pressure, and the residue was purified by column chromatography on silica gel (eluent: ethyl acetate-hexane) to give 105 mg of 5-cyano-*N*-(2-hydroxyethyl)-*N*-[2-(4-isopropylphenyl)ethyl]-2-methoxybenzenesulfonamide.
¹H-NMR (CDCl₃) δ ppm:
1.22 (6H, d, J=6.9Hz), 1.85 (1H, t, J=5.7Hz), 2.81-2.91 (3H, m), 3.45-3.53 (4H, m), 3.74 (2H, q, J=5.7Hz), 3.95 (3H, s), 7.00-7.04 (3H, m), 7.11 (2H, d, J=8.2Hz), 7.78 (1H, dd, J=8.5, 2.2Hz), 8.27 (1H, d, J=2.2Hz)

### Reference Example 42

### [(5-Cyano-2-methoxybenzenesulfonyl)-[2-(4-isopropylphenyl)ethyl]amino]acetic acid

To a solution of 15.14 g of Ethyl [(5-cyano-2-methoxybenzenesulfonyl)-[2-(4-isopropylphenyl)ethyl]amino]acetate in 180 mL of tetrahydrofuran was added 85 mL of 2 mol/L sodium hydroxide solution at room temperature, and the mixture was stirred at 50 ºC for 1 hour. To the reaction mixture was added 270 mL of 1 mol/L hydrochloric acid, and the mixture was extracted with ethyl acetate. The organic layer was washed with water, and brine, and dried over anhydrous magnesium sulfate. The solvent was removed under reduced pressure to give 12.48 g of [(5-cyano-2-methoxybenzenesulfonyl)-[2-(4-isopropylphenyl)ethyl]amino]acetic acid.
¹H-NMR (DMSO-d₆) δ ppm:
1.16 (6H, d, J=6.9Hz), 2.65-2.74 (2H, m), 2.76-2.86 (1H, m), 3.41-3.50 (2H, m), 3.90 (3H, s), 4.10 (2H, s), 6.96-7.03 (2H, m), 7.04-7.10 (2H, m), 7.33 (1H, d, J=8.8Hz), 8.05 (1H, dd, J=8.8, 2.2Hz), 8.10 (1H, d, J=2.2Hz), 12.75 (1H, br s)

### Reference Example 43

### 5-Cyano-N-[2-(4-isopropylphenyl)ethyl]-2-methoxy-N-(2-morpholin-4-yl-2-oxoethyl)benzenesulfonamide

To a stirred solution of 11.93 g of [(5-cyano-2-methoxybenzenesulfonyl)-[2-(4-isopropylphenyl)e thyl]amino]acetic acid, 4.386 g of 1-hydroxybenzotirazole monohydrate, and 3.747 mL of morpholine in 140 ml of *N,N*-dimethylformamide was added 10.18 g of 1-[3-(dimethylamino)propyl]-3-ethylcarbodiimide hydrochloride under ice-cooling, and the mixture was stirred at room temperature for 6 hours. To the reaction mixture was added water, and the mixture was extracted with ethyl acetate. The organic layer was washed with water, and brine, and dried over anhydrous magnesium sulfate. The solvent was removed under reduced pressure, and the residue was purified by column chromatography on silica gel (eluent: ethyl acetate-hexane) to give 13.49 g of 5-cyano-*N*-[2-(4-isopropylphenyl)ethyl]-2-methoxy-*N*-(2-morpholin-4-yl-2-oxoethyl)benzenesulfonamide.
¹H-NMR (DMSO-d₆) δ ppm:
1.16 (6H, d, J=6.9Hz), 2.65-2.75 (2H, m), 2.77-2.86 (1H, m), 3.32-3.41 (4H, m), 3.42-3.49 (2H, m), 3.50-3.60 (4H, m), 3.90 (3H, s), 4.25 (2H, s), 6.98-7.04 (2H, m), 7.05-7.11 (2H, m), 7.30 (1H, d, J=8.8Hz), 8.04 (1H, dd, J=8.8, 2.2Hz), 8.10 (1H, d, J=2.2Hz)

### Example 1

### 5-Cyano-2-hydroxy-N-[2-(4-isopropylphenyl)ethyl]-N-(1,3-thiazol-2-ylmethyl)benzenesulfonamide (Compound 1)

A suspension of 286 mg of 5-cyano-*N*-[2-(4-isopropylphenyl)ethyl]-2-methoxy-*N*-(1,3-thiazol-2-ylmethyl)benzenesulfonamide and 45 mg of lithium chloride in 8 mL of *N,N*-dimethylformamide was stirred at 140 ºC for 4 hours. The solvent was removed under reduced pressure, and to the residue was added diluted hydrochloric acid. The insoluble was collected by filtration to give 226 mg of 5-cyano-2-hydroxy-*N*-[2-(4-isopropylphenyl)ethyl]-*N*-(1,3-thiazol-2-ylmethyl)benzenesulfonamide.
¹H-NMR (DMSO-d₆) δ ppm:
1.15 (6H, d, J=6.9Hz), 2.62-2.71 (2H, m), 2.81 (1H, sept, J=6.9Hz), 3.41-3.53 (2H, m), 4.88 (2H, s), 6.99 (2H, d, J=7.9Hz), 7.07-7.13 (3H, m), 7.70 (1H, d, J=3.2Hz), 7.74 (1H, d, J=3.2Hz), 7.88 (1H, dd, J=8.5, 2.2Hz), 8.07 (1H, d, J=2.2Hz), 12.20 (1H, br s)

### Example 2

The following compounds were prepared according to a similar manner to that described in Example 1

### 5-Cyano-2-hydroxy-N-[2-[4-(pyrrolidin-1-ylcarbonyl)phenyl]ethyl]benzenesulfonamide (Compound 2)

¹H-NMR (CDCl₃) δ ppm:
1.84-2.15 (4H, m), 2.79 (2H, t, J=6.3Hz), 3.11-3.19 (2H, m), 3.41 (2H, t, J=6.6Hz), 3.66 (2H, t, J=6.9Hz), 5.10 (1H, br s), 7.02 (1H, d, J=8.5Hz) , 7.08 (2H, d, J=8.2Hz) , 7.36 (2H, d, J=8.2Hz), 7.62 (1H, dd, J=8.5, 2.2Hz), 8.03 (1H, d, J=2.2Hz), 10.40-11.10 (1H, br)

### Ethyl 3-[4-[2-(5-cyano-2-hydroxybenzenesulfonylamino)ethyl]phenyl]but-2-enoate (Compound 3)

¹H-NMR (CDCl₃) δ ppm:
1.31 (3H, t, J=6.9Hz), 2.55 (3H, s), 2.82 (2H, t, J=6.9Hz), 3.32 (2H, q, J=6.9Hz), 4.22 (2H, q, J=6.9Hz), 4.66-4.72 (1H, m), 6.12 (1H, s), 7.05-7.15 (3H, m), 7.40 (2H, d, J=8.2Hz), 7.69 (1H, dd, J=8.8, 2.2Hz), 7.90 (1H, d, J=2.2Hz), 9.23 (1H, br s)

### 5-Cyano-2-hydroxy-N-[2-[4-(2-oxo-1,3-oxazolidin-3-yl)phenyl]ethyl]benzenesulfonamide (Compound 4)

¹H-NMR (DMSO-d₆) δ ppm:
2.62-2.70 (2H, m), 3.01-3.12 (2H, m), 3.98-4.07 (2H, m), 4.38-4.47 (2H, m), 7.09 (1H, d, J=8.7Hz), 7.12-7.18 (2H, m), 7.39-7.45 (2H, m), 7.46-7.53 (1H, m), 7.84 (1H, dd, J=8.7, 2.2Hz), 7.93 (1H, d, J=2.2Hz), 11.90-12.03 (1H, br)

### 5-Cyano-2-hydroxy-N-[2-(4-isopropylphenyl)ethyl]-N-(2-morpholin-4-yl-2-oxoethyl)benzensulfonamide (Compound 5)

¹H-NMR (DMSO-d₆) δ ppm:
1.16 (6H, d, J=6.9Hz), 2.65-2.75 (2H, m), 2.76-2.86 (1H, m), 3.30-3.60 (10H, m), 4.25 (2H, s), 7.00-7.06 (2H, m), 7.06-7.14 (3H, m), 7.84 (1H, dd, J=8.5, 2.2Hz), 8.03 (1H, d, J=2.2Hz), 12.02 (1H, br s)

### 5-Cyano-N-[2-(4-dimethylaminophenyl)ethyl]-2-hydroxybenzene sulfonamide (Compound 6)

¹H-NMR (DMSO-d₆) δ ppm:
2.53-2.58 (2H, m), 2.83 (6H, s), 2.95-3.01 (2H, m), 6.61 (2H, d, J=8.5Hz), 6.93 (2H, d, J=8.5Hz), 7.11 (1H, d, J=8.5Hz), 7.38-7.43 (1H, m), 7.85 (1H, dd, J=8.5, 2.2Hz), 7.93-7.97 (1H, m), 11.70-12.40 (1H, br)

### N-tert-Butyl-4'-[2-(5-cyano-2-hydroxybenzenesulfonylamino)ethyl]biphenyl-2-sulfonamide (Compound 7)

¹H-NMR (DMSO-d₆) δ ppm:
0.95 (9H, s), 2.72-2.78 (2H, m), 3.05-3.13 (2H, m), 6.34 (1H, br s), 7.13 (1H, d, J=8.5Hz), 7.18 (2H, d, J=7.9Hz), 7.26-7.31 (3H, m), 7.51-7.58 (2H, m), 7.61 (1H, td, J=7.6, 1.3Hz), 7.87 (1H, dd, J=8.5, 2.2Hz), 8.00-8.05 (2H, m), 12.02 (1H, br s)

### Ethyl [(5-cyano-2-hydroxybenzenesulfonyl)-[2-(4-isopropylphenyl)ethyl]amino]acetate (Compound 8)

¹H-NMR (CDCl₃) δ ppm:
1.15-1.30 (9H, m), 2.80-2.93 (3H, m), 3.41-3.47 (2H, m), 4.04 (2H, s), 4.07-4.18 (2H, m), 7.03 (2H, d, J=7.9Hz), 7.08 (1H, d, J=8.8Hz), 7.14 (2H, d, J=7.9Hz), 7.68 (1H, dd, J=8.8, 1.9Hz), 7.99 (1H, d, J=1.9Hz), 9.30-9.80 (1H, br)

### 5-Cyano-2-hydroxy-N-[2-(4-isopropylphenyl)ethyl]-N-methylbenzenesulfonamide (Compound 9)

¹H-NMR (DMSO-d₆) δ ppm:
1.18 (6H, d, J=6.6Hz), 2.72-2.78 (2H, m), 2.80 (3H, s), 2.84 (1H, sept, J=6.6Hz), 7.08-7.17 (5H, m), 7.87 (1H, dd, J=8.5, 2.2Hz), 8.02 (1H, d, J=2.2Hz), 12.00 (1H, br s)

### 5-Cyano-2-hydroxy-N-(2-hydroxyethyl)-N-[2-(4-isopropylphenyl)ethyl]benzenesulfonamide (Compound 10)

¹H-NMR (DMSO-d₆) δ ppm:
1.17 (6H, d, J=6.6Hz), 2.70-2.77 (2H, m), 2.83 (1H, sept, J=6.6Hz), 3.40-3.70 (4H, m), 4.50-5.00 (1H, br), 7.06 (2H, d, J=8.2Hz), 7.10 (1H, d, J=8.5Hz), 7.13 (2H, d, J=8.2Hz), 7.87 (1H, dd, J=8.5, 2.2Hz), 8.06 (1H, d, J=2.2Hz), 12.03 (1H, br s)

### Example 3

### 5-Cyano-N-hydrazinocarbonylmethyl-2-hydroxy-N-[2-(4-isopropylphenyl)ethyl]benzenesulfonamide (Compound 11)

To a solution of 0.463 g of ethyl [(5-cyano-2-hydroxybenzenesulfonyl)-[2-(4-isopropylphenyl)ethyl]amino]-acetate in 5 mL of ethanol was added 0.156 mL of hydrazine monohydrate at room temperature, and the mixture was refluxed for 16 hours. The solvent was removed under reduced pressure to give 0.448 g of 5-cyano-*N*-hydrazinocarbonylmethyl-2-hydroxy-*N*-[2-(4-isopropylphenyl)ethyl]benzenesulfonamide.
¹H-NMR (DMSO-d₆) δ ppm:
1.15 (6H, d, J=6.9Hz), 2.61-2.71 (2H, m), 2.74-2.89 (1H, m), 2.96-3.09 (2H, m), 3.91 (2H, br s), 5.76 (2H, s), 6.32 (1H, d, J=9.1Hz), 6.91-6.96 (2H, m), 7.07-7.13 (2H, m), 7.28 (1H, dd, J=9.1, 2.5Hz), 7.74 (1H, d, J=2.5Hz), 8.80-9.20 (1H, m), 10.80-11.10 (1H, br)

### Example 4

### 5-Cyano-2-hydroxy-N-[2-(4-isopropylphenyl)ethyl]-N-(5-oxo-4,5-dihydro-1,3,4-oxadiazol-2-ylmethyl)benzenesulfonamide (Compound 12)

To a stirred solution of 0.470 g of 5-cyano-*N*-hydrazinocarbonylmethyl-2-hydroxy-*N*-[2-(4-isopropylphenyl)ethyl]benzenesulfonamide in 10 mL of tetrahydrofuran was added 0.335 g of triphosgene under ice-cooling, and the mixture was stirred at room temperature for 30 minutes. To the reaction mixture was added water, and the mixture was extracted with ethyl acetate. The organic layer was washed with brine, and dried over anhydrous magnesium sulfate. The solvent was removed under reduced pressure, and the residue was purified by column chromatography on silica gel (eluent: ethyl acetate) to give 0.37 g of 5-cyano-2-hydroxy-*N*-[2-(4-isopropylphenyl)ethyl]-*N*-(5-oxo-4,5-dihydro-1,3,4-oxadiazol-2-ylmethyl)benzenesulfonamide.
¹H-NMR (DMSO-d₆) δ ppm:
1.17 (6H, d, J=6.9Hz), 2.66-2.90 (3H, m), 3.41-3.56 (2H, m), 4.50 (2H, s), 6.99-7.17 (5H, m), 7.88 (1H, dd, J=8.5, 2.2Hz), 8.04 (1H, d, J=2.2Hz), 12.15-12.32 (2H, m)

### Reference example 44

### N-[2-(Morpholin-4-yl)ethoxycarbonyloxy]succinimide

To a solution of 1.65 g of 2-(morpholin-4-yl)ethanol and 0.275 mL of triethylamine in 3 mL of dichloromethane was added 0.505 g of bis-(2,5-dioxopyrrolidin-1-yl) carbonate one portion at room temperature, and the mixture was stirred at the same temperature for 1.4 hours. The solvent was removed under reduced pressure, and the residue was dissolved in ethyl acetate. The resulting solution was washed with water, and dried over anhydrous magnesium sulfate. The solvent was removed under reduced pressure to give 0.43 g of *N*-[2-(morpholin-4-yl)ethoxycarbonyloxy]succinimide.
¹H-NMR (CDCl₃) δ ppm:
2.49-2.57 (4H, m), 2.72 (2H, t, J=5.7Hz), 2.84 (4H, s), 3.67-3.75 (4H, m), 4.43 (2H, t, J=5.7Hz)

### Reference example 45

### 4-Chloromethoxycarbonyloxy-1-nitrobenzene

To a solution of 5.0 g of 4-nitrophenol and 2.90 mL of pyridine in dichloromethane was added 3.50 mL of chloromethyl chloroformate under ice-cooling with stirring, and the mixture was stirred at room temperature for 61 hours. The reaction mixture was washed with water, aqueous sodium hydroxide (0.125 mol/L) andwater successively, and dried over anhydrous magnesium sulfate. The solvent was removed under reduced pressure to give 7.92 g of 4-chloromethoxycarbonyloxy-1-nitorobenzene.
¹H-NMR (CDCl₃) δ ppm:
5.85 (2H, s), 7.41-7.46 (2H, m), 8.29-8.35 (2H, m)

### Reference example 46

### 4-Iodomethoxycarbonyloky-1-nitrobenzene

A suspension of 5.45 g of 4-chloromethoxycarbonyloxy-1-nitrobenzene and 7.07 g of sodium iodide in 30 mL of acetone was stirred at 45 ºC for 7.5 hours. The insoluble material was removed by filtration, and washed with diethyl ether. The filtrate was concentrated under reduced pressure to give 7.54 g of 4-iodomethoxycarbonyloky-1-nitrobenzene.
¹H-NMR (CDCl₃) δ ppm:
6.07 (2H, s), 7.40-7.45 (2H, m), 8.28-8.33 (2H, m)

### Reference example 47

### 4-Nitrophenoxycarbonyloxymethyl 2-acetoxy-2-methylpropionate

To 20 mL of acetyl chloride was added in small portions 10.3 g of 2-hydroxy-2-methylpropionic acid under ice-cooling with stirring, and refluxed for 1 hour, and then the reaction mixture was concentrated under reduced pressure. A suspension of 10.4 g of the residue, 20.2 g of 4-iodomethoxycarbonyloxy-1-nitrobenzene and 11.9 g of silver carbonate in 135 mL of toluene was stirred at 80 ºC for 1.2 hours. The insoluble material was removed by filtration, and washed with ethyl acetate. The filtrate was removed under reduced pressure, and the residue was purified by column chromatography on silica gel (eluent: ethyl acetate-hexane) to give 6.7 g of 4-nitrophenoxycarbonyloxymethyl 2-acetoxy-2-methylpropionate.
¹H-NMR (CDCl₃) δ ppm:
1.54 (6H, s), 2.03 (3H, s), 5.87 (2H, s), 7.42-7.47 (2H, m), 8.27-8.33 (2H, m)

### Reference example 48

### O-Chloromethyl S-ethylthiocarbonate

To a solution of 99 g of chloromethy chloroformate in 1.6 L of diethyl ether was added by drop wise a solution of 56.9 mL of ethanethiol and 107 mL of triethylamine in 400 mL of diethyl ether during 110 minuets under ice-cooling with stirring, and then stirred at room temperature for 22 hours. The formed precipitate was removed by filtration through a celite column, and the filtrate was concentrated under reduced pressure to give 94.3 g of O-chloromethyl S-ethylthiocarbonate.
¹H-NMR (CDCl₃) δ ppm:
1.35 (3H, t, J=7.3Hz), 2.93 (2H, q, J=7.3Hz), 5.77 (2H, s)

### Reference example 49

### Ethylthiocarbonyloxymethyl 2,2-dimethylpropionate

5.0 g of 2,2-dimethylpropionic acid was dissolved in a solution of 127 mL of 10% aqueous tetrabutylammmonium hydroxide solution in 50 mL of tetrahydrofuran, and stirred at room temperature for 10 minutes. The reaction mixture was concentrated under reduced pressure, and the residue was dissolved in 130 mL of tetrahydrofuran. To the resulting solution was added 6.88 g of O-chloromethyl S-Ethylthiocarbonate, and the mixture was stirred at the same temperature for 14 hours. To the reaction mixture was added water, and extracted with ethyl acetate. The oerganic layer was washed with water and brine, and dried over anhydrous magnesium sulfate. The solvent was removed under reduced pressure, and the residue was purified by column chromatography on silica gel (eluent: ethyl acetate - hexane) to give 9.89 g of ethylthiocarbonyloxymethyl 2,2-dimethylpropionate.
¹H-NMR (CDCl₃) δ ppm:
1.22 (9H, s), 1.33 (3H, t, J=7.3Hz), 2.89 (2H, q, J=7.3Hz), 5.81 (2H, s)

### Reference example 50

### Chlorocarbonyloxymethyl 2,2-dimethylpropionate

To 1.46 mL of sulfulyl chloride 2.00 g of ethylcarbonyloxymethyl 2,2-dimethylpripoionate was added under ice-cooling with stirring, and stirred at the same temperature for 1.5 hours. The reaction mixture was concentrated under reduced pressure to give 1.75 g of chlorocarbonyloxymethyl 2,2-dimethylpropionate.
¹H-NMR (CDCl₃) δ ppm:
1.25 (9H, s), 5.83 (2H, s)

### Reference example 51

### 4-Nitrophenoxycarbonyloxymethyl 2,2-dimethylpropionate

To a solution of 1.75 g of chlorocarbonyloxymethyl 2,2-dimethylpropionate and 0.762 mL of pyridine in 36 mL of tetrahydrofuran was added 1.31 g of 4-nitrophenol under ice-cooling with stirring, and stirred at the same temperature for 30 minutes. To the reaction mixture was added water, and extracted with ethyl acetate. The organic layer was washed with water and brine, dried over anhydrous magnesium sulfate. The solvent was removed under reduced pressure, and the residue was purified by column chromatography on silica gel (eluent: ethyl acetate - hexane) to give 1.836 g of 4-nitrophenoxycarbonyloxymethyl 2,2-dimethylpropionate.
¹H-NMR (CDCl₃) δ ppm:
1.26 (9H, s), 5.89 (2H, s), 7.38-7.43 (2H, m), 8.27-8.32 (2H, m)

### Reference example 52

### 2-Benzyloxyethyl 2,2-dimethylpropionate

To a solution of 15.2 g of 2-benzyloxyethanol and 15.3 mL of triethylamine in 300 mL of dichloromethane was added 13.5 mL of 2,2-dimethylpropionyl chloride under ice-cooling with stirring, and stirred at room temperature for 26 hours. The reaction mixture was concentrated under reduced pressure, to the residue were added water and diethyl ether, and the organic layer was separated. The aqueous layer was extracted with diethyl ether, and the combined organic layer was washed with hydrochloric acid (1 mol/L), water, saturated sodium hydrogen carbonate solution, water and brine successively, and dried over anhydrous magnesium sulfate. The solvent was removed under reduced pressure, and the residue was purified by column chromatography on silica gel (eluent: ethyl acetate - hexane) to give 20.7 g of 2-benzyloxyethyl 2,2-dimethylpropionate.
¹H-NMR (CDCl₃) δ ppm:
1.22 (9H, s), 3.65-3.70 (2H, m), 4.23-4.27 (2H, m), 4.57 (2H, s), 7.26-7.38 (5H, m)

### Reference example 53

### 2-Hydoroxyethyl 2,2-dimethylpropionate

A suspension of 2.06 g of 2-benzyoxyethyl 2,2-dimethylpropionate and 4.12 g of 10 % palladium on carbon in 260 mL of ethanol was stirred under a hydrogen atmosphere at room temperature for 1.5 hours. The insoluble material was removed by filtration through Celite, and the filtrate was concentrated under reduced pressure to give 12.5 g of 2-Hydoroxyethyl 2,2-dimethylpropionate.
¹H-NMR (CDCl₃) δ ppm:
1.21 (9H, s), 2.15 (1H, br s), 3.77-3.86 (2H, m), 4.17-4.23 (2H, m)

### Reference example 54

### 2-Benzyloxyethyl chloroformate

To a solution of 1.0 mL of 2-benzyloxyethanol and 1.15 mL of triethylamine in 20 mL of dichloromethane was added in small portions 0.84 g of triphosgene under ice-cooling with stirring, and stirred at the same temperature for 15 minutes, and stirred at room temperature for 1 hour. To the reaction mixture was bubbled an argon gas for 2 minutes, and the solvent of the reaction mixture was removed under reduced pressure. To the residue were added diethyl ether and anhydrous magnesium sulfate, and the insoluble material was removed by filtration. The filtrate was concentrated under reduced pressure to give 1.35 g of 2-benzyloxyethyl chloroformate.
¹H-NMR (CDCl₃) δ ppm:
3.71-3.76 (2H, m), 4.45-4.50 (2H, m), 4.58 (2H, s), 7.27-7.41 (5H, m)

### Reference example 55

The following compounds were prepared according to a similar manner to that described in Reference Example 54.

### Tetrahydrofuran-2-ylmethyl chloroformate

¹H-NMR (CDCl₃) δ ppm:
1.60-1.71 (1H, m), 1.85-2.10 (3H, m), 3.77-3.85 (1H, m), 3.86-3.95 (1H, m), 4.15-4.30 (2H, m), 4.32-4.38 (1H, m)

### Methyl 2-chlorocarbonyloxypropionate

1.60 (3H, d, J=7.3Hz), 3.81 (3H, s), 5.18 (1H, q, J=7.3Hz)

### 2-(Chlorocarbonyloxy)ethyl 2,2-dimethylpropionate

¹H-NMR (CDCl₃) δ ppm:
1.21 (9H, s), 4.31-4.38 (2H, m), 4.50-4.55 (2H, m)

### Example 5

### 5-Carbamimidoyl-2-hydroxy-N-[2-[4-(2-oxooxazolidin-3-yl)phenyl]ethyl]benzenesufonamide (Compound 13)

5-Cyano-2-hydroxy-*N*-[2-[4-(2-oxooxazolidin-3-yl)phenyl[ethyl]benzenesulfonamide(0.142 g) was dissolved in 5.8 g of 40% hydrogen chloride-ethanol solution, and stirred at room temperature for 4 hours. The reaction mixture was concentrated under reduced pressure, to the residue were added 4 mL of ethanol and 0.22 g of ammounium acetate, and stirred at room temperature for 14 hours. To the reaction mixture were added 12 mL of water 2 mL of hexane under ice-cooling with stirring, and the mixture was stirred for 1 hour. The formed precipitate was collected by filtration to give 28 mg of 5-carbamimidoyl-2-hydroxy-*N*-[2-[4-(2-oxooxazolidin-3-yl)phenyl]ethyl]benzenesufonamide.
¹H-NMR (DMSO-d₆) δ ppm:
2.64-2.71 (2H, m), 2.78-2.85 (2H, m), 3.98-4.06 (2H, m), 4.37-4.45 (2H, m), 6.29 (1H, d, J=9.1Hz), 6.60-7.00 (1H, br), 7.14-7.20 (2H, m), 7.40-7.47 (2H, m), 7.51 (1H, dd, J=9.1, 3.2Hz), 7.72 (2H, br s), 7.96 (1H, d, J=3.2Hz), 8.35-8.60 (2H, br)

### Example 6

The following compounds were prepared according to a similar manner to that described in Example 5.

### 5-Carbamimidoyl-2-hydroxy-N-[2-(4-isopropylphenyl)ethyl]-N-(2-morpholin-4-yl-2-oxoethyl)benzenesulfonamide (Compound 14)

¹H-NMR (DMSO-d₆) δ ppm:
1.15 (6H, d, J=6.6Hz), 2.61-2.70 (2H, m), 2.76-2.86 (1H, m), 3.19-3.63 (10H, m), 4.30 (2H, br s), 6.21 (1H, d, J=9.1Hz), 6.94-7.00 (2H,m), 7.05-7.13 (2H,m), 7.45 (1H, dd, J=9.1, 2.8Hz), 7.81 (2H, br s), 8.05 (1H, d, J=2.8Hz), 8.44 (2H, br s)

### 5-Carbamimidoyl-2-hydroxy-N-[2-(2'-sulfamoylbiphenyl-4-yl)ethyl]benzenesulfnamide (Compound 15)

¹H-NMR (DMSO-d₆) δ ppm:
2.72-2.80 (2H, m), 2.83-2.91 (2H, m), 6.39 (1H, d, J=9.1Hz), 6.64-6.78 (1H, br), 6.80-7.75 (12H, m), 7.95 (1H, d, J=2.8Hz), 8.05 (1H, dd, J=7.8, 1.2Hz), 8.26-8.66 (2H, br)

### Ethyl [(carbamimidoyl-2-hydroxybenzenesulfonyl)-[2-(4-isopropylphenyl)ethyl]amino]acetate (Compound 16)

¹H-NMR (DMSO-d₆) δ ppm:
1.15 (6H, d, J=6.9Hz), 1.20 (3H, t, J=7.3Hz), 2.60-2.70 (2H, m), 2.80 (1H, sept, J=6.9Hz), 3.15-3.22 (2H, m), 4.09 (2H, q, J=7.3Hz), 4.36 (2H, s), 6.16 (1H, d, J=9.1Hz), 7.01 (2H, d, J=7.9Hz), 7.09 (2H, d, J=7.9Hz), 7.42 (1H, dd, J=9.1, 3.2Hz), 7.60-7.90 (2H, br), 8.01 (1H, d, J=3.2Hz), 8.20-8.60 (2H, br)

### 5-Carbamimidoyl-2-hydroxy-N-[2-(4-isopropylphenyl)ethyl]-N-methylbenzenesulfonamide (Compound 17)

¹H-NMR (DMSO-d₆) δ ppm:
1. 17 (6H, d, J=6.9Hz), 2.68-2.74 (5H, m), 2.83 (1H, sept, J=6.9Hz), 3.25-3.32 (2H, m), 6.14 (1H, d, J=9.1Hz), 7.07-7.16 (4H, m), 7.41 (1H, dd, J=9.1, 3.1Hz), 7.50-7.94 (2H, br), 7.99 (1H, d, J=3.1Hz), 8.15-8.58 (2H, br)

### Example 7

### Ethyl 3-[4-[2-(5-carbamimidoyl-2-hydroxybenzenesulfonylamino)ethyl]phenyl]but-2-enoate hydrochloride (Compound 18)

Ethyl 3-[4-[2-(5-cyano-2-hydroxybenzenesulfonnylamino)ethyl]phenyl]but-2-enoate(0.44 g) was dissolved in 8.0 mL of 38% hydrogen chloride-ethanol solution, and stirred at room temperature for 5 hours. The reaction mixture was concentrated under reduced pressure, to the residue were added 4 mL of ethanol and 0.22 g of ammonium acetate, and stirred at room temperature for 21 hours. The reaction mixture was concentrated under reduced pressure, to the residue were added 10 mL of ethanol and 0.4 mL of 2 mol/L hydrochloric acid, and the insoluble material was removed by filtration. The filtrate was concentrated under reduced pressure, and the residue was purified by column chromatography on octadesyl-silica gel (eluent: water -acetonitrile). The obtained compound was dissolved in hydrochloric acid (1 mol/L) and ethanol, and concentrated under reduced pressure to give 0.19 g of ethyl 3-[4-[2-(5-carbamimidoyl-2-hydroxybenzenesulfonylamino)ethyl]phenyl]but-2-enoate hydrochloride.
¹H-NMR (DMSO-d₆) δ ppm:
1.24 (3H, t, J=6.9Hz), 2.48 (3H, d, J=1.3Hz), 2.66-2.76 (2H, m), 2.97-3.12 (2H, m) , 4.14 (2H, q, J=6.9Hz), 6.12 (1H, d, J=1.3Hz), 7.14-7.20 (2H,m), 7.40-7.50 (3H,m), 7.87 (1H, dd, J=8.8, 2.2Hz), 8.14 (1H, d, J=2.2Hz), 8.84 (2H, br s), 9.26 (2H, br s), 12.02 (1H, br s)

### Example 8

The following compounds were prepared according to a similar manner to that described in Example 7.

### 5-Carbamimidoyl-2-hydroxy-N-[2-[4-(pyrrolidin-1-ylcarbonyl) phenyl]ethyl]benzenesulfonamide hydrochloride (Compound 19)

¹H-NMR (DMSO-d₆) δ ppm:
1.72-1.89 (4H, m), 2.73 (2H, t, J=7.3Hz), 3.02-3.10 (2H, m), 3.35 (2H, t, J=6.3Hz), 3.44 (2H, t, J=6.6Hz), 7.16-7.22 (3H, m), 7.38 (2H, d, J=7.9Hz), 7.48 (1H, t, J=5.7Hz), 7.86 (1H, dd, J=8.5, 2.5Hz), 8.12 (1H, d, J=2.5Hz), 8.90 (2H, br s), 9.28 (2H, br s), 12.06 (1H, br s)

### 5-Carbamimidoyl-2-hydroxy-N-[2-(4-isopropylphenyl)ethyl]-N-(5-oxo-4,5-dihydro-1,3,4-oxadiazol-2-ylmethyl)benzenesulfonamide hydrochloride (Compound 20)

¹H-NMR (DMSO-d₆) δ ppm:
1.16 (6H, d, J=6.9Hz), 2.65-2.75 (2H, m), 2.77-2.87 (1H, m), 3.35-3.47 (2H, m), 4.53 (2H, s), 7.04 (2H, d, J=7.9Hz), 7.11 (2H, d, J=7.9Hz), 7.24 (1H, d, J=8.8Hz), 7.92 (1H, dd, J=8.8, 2.2Hz), 8.21 (1H, d, J=2.2Hz), 9.01 (2H, br s), 9.33 (2H, br s), 12.10-12.54 (2H, m)

### 5-Carbamimidoyl-2-hydroxy-N-[2-(4-isopropylphenyl)ethyl]-N-(1,3-thiazol-2-ylmethyl)benzenesulfonamide hydrochloride (Compound 21)

¹H-NMR (DMSO-d₆) δ ppm:
1.15 (6H, d, J=6.9Hz), 2.60-2.70 (2H, m), 2.81 (1H, sept, J=6.9Hz), 3.42-3.51 (2H, m), 4.89 (2H, s), 6.99 (2H, d, J=7.9Hz), 7.07-7.12 (2H, m), 7.26 (1H, d, J=8.5Hz), 7.71 (1H, d, J=3.2Hz), 7.76 (1H, d, J=3.2Hz), 7.92 (1H, dd, J=8.5, 2.2Hz), 8.23 (1H, d, J=2.2Hz), 9.02 (2H, br s), 9.33 (2H, br s), 12.34 (1H, br s)

### 5-Carbamimidoyl-N-[2-(4-dimethylaminophenyl)ethyl]-2-hydroxybenzenesulfonamide dihydrochloride (Compound 22)

¹H-NMR (DMSO-d₆) δ ppm:
2.71-2.78 (2H, m) , 2.83 (6H, s), 6.28 (1H, d, J=9.1Hz), 6.60-6.65 (2H, m), 6.70-6.90 (1H, br), 6.92-6.98 (2H, m), 7.50 (1H, dd, J=9.1, 2.8Hz), 7.70-7.90 (2H, br), 7.95 (1H, d, J=2.8Hz), 8.30-8.60 (2H, br)

### 5-Carbamimidoyl-2-hydroxy-N-(2-hydroxyethyl)-N-[2-(4-isopropylpheyl)ethyl]benzenesulfonamide hydrochloride (Compound 23)

¹H-NMR (DMSO-d₆) δ ppm:
1.17 (6H, d, J=6.6Hz), 2.70-2.77 (2H, m), 2.83 (1H, sept, J=6.6Hz), 3.41-3.55 (4H, m), 4.65-4.85 (1H, br), 7.06 (2H, d, J=8.2Hz), 7.13 (2H, d, J=8.2Hz), 7.17 (1H, d, J=8.5Hz), 7.87 (1H, dd, J=8.5, 2.5Hz), 8.20 (1H, d, J=2.5Hz), 8.85 (2H, br s), 9.29 (2H, br s), 12.05 (1H, s)

### Example 9

### 3-[4-[2-(5-Carbamimidoyl-2-hydroxybenzenesulfonylamino)ethyl]phenyl]but-2-enoic acid hydrochloride (Compound 24)

A solution of 40 mg of ethyl 3-[4-[2-(5-carbamimidoyl-2-hydroxybenzenesulfonyl)ethyl]phenyl]but-2-enoate hydrochloride and 2.2 mL of hydrochloric acid (0.5 mol/L) in 1.0 mL of acetonitrile was stirred at 85°C for 22 hours. To the reaction mixture was added 0.5 mL of hydrochloric acid (1 mol/L), and stirred at the same temperature for 17 hours. The reaction mixture was concentrated under reduced pressure, and the residue was purified by column chromatography on octadecyl-silica gel (eluent: water -acetonitrile) to give 6 mg of 3-[4-[2-(5-carbamimidoyl-2-hydroxybenzenesulfonylamino)ethyl]phenyl]but-2-enoic acid hydrochloride
¹H-NMR (DMSO-d₆) δ ppm:
2.46 (3H, d, J=1.3Hz), 2.72 (2H, t, J=7.6Hz), 2.98-3.07 (2H, m), 6.07 (1H, d, J=1.3Hz), 7.12-7.20 (3H, m), 7.41-7.50 (3H, m), 7.86 (1H, dd, J=8.5, 2.2Hz), 8.13 (1H, d, J=2.2Hz), 8.77 (2H, br s), 9.24 (2H, br s), 11.98 (1H, br s), 12.20 (1H, br s)

### Example 10

The following compound was prepared allowing to react by a similar manner to that described in Example9, and optionally to transform to free form by usual method.

### [(5-Carbamimidoly-2-hydroxybenzenesulfonyl)-[2-(4-isopropylphenyl)ethyl]amino]acetic acid hydrochloride (Compound 25)

¹H-NMR (DMSO-d₆) δ ppm:
1.16 (6H, d, J=6.9Hz), 2.65-2.75 (2H, m), 2.82 (1H, sept, J=6.9Hz), 3.38-3.48 (2H, m), 4.16 (2H, s), 7.04 (2H, d, J=7.9Hz), 7.10 (2H, d, J=7.9Hz), 7.19 (1H, d, J=8.8Hz), 7.88 (1H, dd, J=8.8, 2.5Hz), 8.19 (1H, d, J=2.5Hz), 8.94 (2H, br s), 9.30 (2H, br s), 11.90-13.00 (2H, br)

### Example 11

### 2-Hydroxy-5-(N-hydroxycarbamimidoyl)-N-[2-(4-isopropylphenyl)ethyl]-N-(2-morpholin-4-yl-2-oxoethyl)benzenesulfonamide (Compound 26)

97 mg of 5-cyano-2-hydroxy-*N*-[2-(4-isopropylphenyl)ethyl]-*N*-(2-morpholin-4-yl-2-oxoethyl)benzenesulfonamide was dissolved in 10 mL of 35 % hydrogen chloride in ethanol, and stirred at room temperature for 4 hours. The reaction mixture was concentrated unde reduced pressure, and the residue was dissolved in 10 mL of ethanol. To the resulting mixture were added 72 mg of hydroxylamine hydrochloride and 0.20 mL of triethylamine successively, and stirred at room temperature for 17 hours. The reaction mixture was concentrated under reduced pressure, and to the residue was added water. The insoluble material was collected by filtration, and washed with water to give 82 mg of 2-hydroxy-5-(*N*-hydroxycarbamimidoyl)-*N*-[2-(4-isopropylphenyl)ethyl]-*N*-(2-morpholin-4-yl-2-oxoethyl)benzenesulfonamide.
¹H-NMR (DMSO-d₆) δ ppm:
1.16 (6H, d, J=6.9Hz), 2.61-2.70 (3H, m), 2.75-2.87 (2H, m), 3.35-3.46 (4H, m), 3.50-3.61 (4H, m), 4.22 (2H, s), 5.81 (2H, br s), 6.94 (1H, d, J=8.5Hz), 6.99 (2H, d, J=8.2Hz), 7.11 (2H, d, J=8.2Hz), 7.70 (1H, dd, J=8.5, 2.2Hz), 8.04 (1H, d, J=2.2Hz), 9.54 (1H, br s), 10.65-11.25 (1H, br)

### Reference example 56

### 5-[Amino(tetrahydrofuran-2-ylmethoxycarbonylimino)methyl]N-[2-(4-isopropylophenyl)ethyl]-N-methyl-2-(tetrahydrofuran-2-ylmethoxycarbonyloxy)benzenesulfonamide

To a solution of 0.12 g of 5-carbamimidoyl-2-hydroxy-*N*-[2-(4-isopropylphenyl)ethyl]-*N*-methylbenzenesulfonamide and 0.134mL of triethylamine in 1.6 mL of *N,N*-dimethylformamide was added 0.158 g of tetrahydrofuran-2-ylmethyl chloroformate under ice-cooling with stirring, aned stirred at the same temperature for 1.5 hours. To the reaction mixture was added water, and extracted with ethyl acetate. The organic layer was washed with water and brine, and dried over anhydrous magnesium sulfate. The solvent was removed under reduced pressure, and the residue was purified by column chromatography on silica gel (eluent: ethyl acetate-hexane) to give 0.124 g of 5-[amino(tetrahydrofuran-2-ylmethoxycarbonylimino)methyl]-*N*-[2-(4-isopropylphenyl)ethyl]-*N*-methyl-2-(tetrahydrofuran-2-ylmethoxycarbonyloxy)benzenesulfonamide.
¹H-NMR (DMSO-d₆) δ ppm:
1.17 (6H, d, J=6.9Hz), 1.50-1.64 (2H, m), 1.70-2.00 (6H, m), 2.70-2.90 (6H, m), 3.33-3.42 (2H, m), 3.54-3.84 (4H, m), 3.96-4.26 (6H, m), 7.10-7.18 (4H, m), 7.65 (1H, d, J=8.5Hz), 8.29 (1H, dd, J=8.5, 2.2Hz), 8.44 (1H, d, J=2.2Hz), 9.24 (2H, br s)

### Reference example 57

The following compounds were prepared according to a similar manner to that described in Reference Example 56.

### 5-[Amino(butoxycarbonylimino)methyl]-2-butoxycarbonyloxy-N-[2-(4-isopropylphenyl)ethyl]-N-methylbenzenesulfonamide

¹H-NMR (DMSO-d₆) δ ppm:
0.85-0.95 (6H, m), 1.18 (6H, d, J=6.9Hz), 1.28-1.41 (4H, m), 1.55-1.65 (4H, m), 2.70-2.90 (6H, m), 3.30-3.42 (2H, m), 4.00-4.08 (2H, m), 4.21 (2H, t, J=6.3Hz), 7.10-7.18 (4H, m), 7.64 (1H, d, J=8.5Hz), 8.27 (1H, dd, J=8.5, 1.9Hz), 8.42 (1H, d, J=1.9Hz), 9.25 (2H, br s)

### 5-[Amino-(2,2,2-trichloroethoxycarbonylimino)methyl]-N-[2-(4-isopropylphenyl)ethyl]-N-methyl-2-(2,2,2-trichloroethoxy carbonyloxy)benzenesulfonamide

¹H-NMR (DMSO-d₆) δ ppm:
1.17 (6H, d, J=6.9Hz), 2.70-2.88 (6H, m), 3.36-3.45 (2H, m), 4.91 (2H, s), 5.06 (2H, s), 7.13 (4H, br s), 7.73 (1H, d, J=8.8Hz), 8.35 (1H, dd, J=8.8, 2.2Hz), 8.47 (1H, d, J=2.2Hz), 9.33 (1H, br s), 9.61 (1H, br s)

### 5-[Amino-(2-benzyloxyethoxycarbonylimino)methyl]-2-(2-benzyloxyethoxycarbonyloxy)-N-[2-(4-isopropylphenyl)ethyl]-N-methylbenzensulfonamide

¹H-NMR (CDCl₃) δ ppm:
1.21 (6H, d, J=6.9Hz), 2.80-2.91 (6H, m), 3.36-3.42 (2H, m), 3.71-3.75 (2H, m), 3.75-3.80 (2H, m), 4.34-4.39 (2H, m), 4.43-4.47 (2H, m), 4.55 (2H, s), 4.60 (2H, s), 6.10-6.80 (1H, br), 7.07-7.14 (4H, m), 7.25-7.39 (11H, m), 8.24 (1H, dd, J=8.5, 2.2Hz), 8.31 (1H, d, J=2.2Hz), 9.10-9.80 (1H, br)

### 5-[Amino-(2-methoxyethoxycarbonylimino)methyl]-2-(2-methoxy ethoxycarbonyloxy)-N-[2-(4-isopropylphenyl)ethyl]-N-methylbenzenesulfonamide

¹H-NMR (CDCl₃) δ ppm:
1. 22 (6H, d, J=6.9Hz), 2.80-2.93 (6H, m), 3.37 (3H, s), 3.38-3.44 (5H, m), 3.63-3.66 (2H, m), 3.67-3.71 (2H, m), 4.31-4.35 (2H, m), 4.39-4.43 (2H, m), 6.00-7.00 (1H, br), 7.09-7.17 (4H, m), 7.39 (1H, d, J=8.5Hz), 8.26 (1H, dd, J=8.5, 2.2Hz), 8.32 (1H, d, J=2.2Hz), 9.00-10.00 (1H, br)

### 5-[Amino(phenoxycarbonylimino)methyl]-N-[2-(4-isopropylphenyl)ethyl]-N-methyl-2-(phenoxycarbonyloxy)benzenesulfonamide

¹H-NMR (CDCl₃) δ ppm:
1.21 (6H, d, J=6.9Hz), 2.80-2.91 (3H, m), 2.95 (3H, s), 3.44-3.57 (2H, m), 6.55-6.80 (1H, br), 7.11-7.32 (10H, m), 7.38-7.44 (4H, m), 7.52 (1H, d, J=8.5Hz), 8.31 (1H, dd, J=8.5, 2.2Hz), 8.38 (1H, d, J=2.2Hz), 9.40-9.80 (1H, br)

### 5-[Amino(methoxycarbonylimino)methyl]-N-[2-(4-isopropylphenyl)ethyl]2-(methoxycarbonyloxy)-N-methylbenzenesulfonamide

¹H-NMR (CDCl₃) δ ppm:
1.23 (6H, d, J=6.9Hz), 2.81-2.90 (6H, m), 3.36-3.43 (2H, m), 3.81 (3H, s), 3.92 (3H, s), 6.20-6.80 (1H, br), 7.08-7.16 (4H, m), 7.41 (1H, d, J=8.5Hz), 8.25 (1H, dd, J=8.5, 2.2Hz), 8.31 (1H, d, J=2.2Hz), 9.20-9.90 (1H, br)

### Methyl 2-[amino-[3-[[2-(4-isopropylphenyl)ethyl](methyl)sulfamoyl]-4-[1-(methoxycarbonyl)ethoxycarbonyloxy]phenyl]methylenecarbamoyloxy]propionate

¹H-NMR (DMSO-d₆) δ ppm:
1.22 (6H, d, J=6.9Hz), 1.48-1.62 (6H, m), 2.75-2.95 (6H, m), 3.35-3.55 (2H, m), 3.78 (3H, s), 3.80 (3H, s), 5.05-5.25 (2H, m), 7.05-7.20 (4H, m), 7.45 (1H, d, J=8.5Hz), 8.25-8.40 (2H, m)

### 2-[Amino-[4-[2-(2,2-dimethylpropionyloxy)ethoxycarbonyloxy]-3-[[2-(4-isopropylphenyl)ethyl]-(2-morpholin-4-yl-2-oxoethyl)sulfamoyl]phenyl]methylenecarbamoyloxy]ethyl 2,2-dimethylpropionate

¹H-NMR (DMSO-d₆) δ ppm:
1:19-1.24 (24H, m), 2.83-2.96 (3H, m), 3.18-3.25 (2H, m), 3.46-3.52 (2H, m), 3.55-3.71 (6H, m), 4.06 (2H, br s), 4.32-4.42 (6H, m), 4.47-4.52 (2H, m), 7.10-7.18 (4H, m), 7.34-7.46 (2H, m), 8.36 (1H, dd, J=8.5, 2.2Hz), 8.81 (1H, d, J=2.2Hz), 9.59 (1H, br s)

### Amino-[4-(2,2-dimethylpropionyloxymethoxycarbonyloxy)-3-[[2-(4-isopropylphenyl)ethyl]-(2-morgholin-4-yl-2-oxoethyl) sulfamoyl]phenyl]methylenecarbamoyloxymethyl 2,2-dimethylpropionate

¹H-NMR (DMSO-d₆) δ ppm:
1.20-1.29 (24H, m), 2.82-2.96 (3H, m), 3.18-3.25 (2H, m), 3.45-3.52 (2H, m), 3.57-3.68 (6H, m), 4.05 (2H, br s), 5.866 (2H, s), 5.872 (2H, s), 7.10-7.18 (4H, m), 7.37 (1H, d, J=8.5Hz), 7.41 (1H, br s), 8.36 (1H, dd, J=8.5, 2.5Hz), 8.78 (1H, d, J=2.5Hz), 9.61 (1H, br s)

### Example 12

### Methylammonium 4-[amino-[2-(2,2-dimethylgropionyloxy)ethoxycarbonylimino]methyl]-2-[[2-(4-isopropylphenyl)ethyl]-(2-morpholin-4-yl-2-oxoethyl)sulfamoyl]phenoxide (Compound 27)

To a solution of 0.19 g of 2-[amino-[4-[2,2-dimethylpropionyloxy]ethoxycarbonyloxy]-3-[[2-(4-isopropylphenyl)ethyl]-(2-morpholin-4-yl-2-oxoethyl)sulfamoyl]phenyl]methylenecarbamoyloxy]ethyl 2,2-dimethylpropionate in 1.1 mL of tetrahydrofuran was added 0.053 mL of 40 % aqueous methylamine solution, and stirred at the same temperature for 1 hours. To the reaction mixture was added diethyl ether, and the precipitate was collected by filtration to give 0.135 g of methylammonium 4-[amino-[2-(2,2-dimethylpropionyloxy)ethoxycarbonylimino]methyl]-2-[[2-(4-isopropylphenyl)ethyl]-(2-morpholin-4-yl-2-oxoethyl)sulfamoyl]phenoxide.
¹H-NMR (DMSO-d₆) δ ppm:
1.12-1.16(15H, m), 2.37 (3H, s), 2.61-2.70 (2H, m), 2.75-2.85 (1H, m), 3.18-3.62 (10H, m), 4.16-4.30 (6H, m), 6.34 (1H, br s), 6.93-6.99 (2H, m), 7.05-7.11 (2H, m), 7.69-7.77 (1H, m), 8.30 (1H, d, J=2.8Hz), 8.58 (1H, br s), 9.13 (1H, br s)

### Example 13

The following compounds were prepared according to a similar manner to that described in Example12 allowing to the compound to react, and optionally to transform to free form by usual method.

### Methylammonium 4-[amino-(2,2-dimethylpropionyloxymethyloxycarbonylimino)methyl]-2-[[2-(4-isopropylphenyl)ethyl]-(2-mo rpholin-4-yl-2-oxoethyl)sulfamoyl]phenoxide (Compound 28)

¹H-NMR (DMSO-d₆) δ ppm:
1.12-1.16 (15H, m), 2.36 (3H, s), 2.60-2.69 (2H, m), 2.75-2.85 (1H, m), 3.18-3.64 (10H, m), 4.27 (2H, s), 5.69 (2H, s), 6.20 (1H, d, J=8.8Hz), 6.93-7.00 (2H, m), 7.05-7.11 (2H, m), 7.35 (3H, br s), 7.69 (1H, dd, J=8.8, 2.8Hz), 8.30 (1H, d, J=2.8Hz), 8.67 (1H, br s), 9.10 (1H, br s)

### Methylammonium 4-[amino-[1-(methoxycarbonyl)ethoxycarbonylimino]methyl]-2-[[2-(4-isopropylphenyl)ethyl](methyl)-sulfamoyl]phenoxide (Compound 29)

¹H-NMR (DMSO-d₆) δ ppm:
1.23 (6H, d, J=7.0Hz), 1.57 (3H, d, J=6.9Hz), 2.46 (3H, s), 2.78-2.93 (6H, m), 3.31-3.38 (2H, m), 3.77 (3H, s), 5.07 (1H, q, J=6.9Hz), 6.20-6.80 (1H, br), 7.08-7.12 (3H, m), 7.14-7.18 (2H, m), 8.00 (1H, d, J=2.5Hz), 8.09 (1H, dd, J=8.8, 2.5Hz), 9.00-10.00 (1H, br)

### Methylammonium 4-[amino(butoxycarbonylimino)methyl]-2-[[2-(4-isopropylphenyl)ethyl](methyl)sulfamoyl]phenoxide (Compound 30)

¹H-NMR (DMSO-d₆) δ ppm:
0.90 (3H, t, J=7.3Hz), 1.17 (6H, d, J=6.9Hz), 1.30-1.42 (2H, m), 1.50-1.65 (2H, m), 2.35 (3H, br s), 2.65-2.90 (6H, m), 3.15-3.50 (5H, m), 3.96 (2H, t, J=6.9Hz), 6.26 (1H, d, J=8.8Hz), 7.00-7.20 (4H, m), 7.68 (1H, dd, J=8.8, 2.2Hz), 8.24 (1H, d, J=2.2Hz), 8.44 (1H, br s), 9.14 (1H, br s)

### Methylammonium 4-[amino-(2,2,2-trichloroethoxycarbonylimino)methyl]-2-[[2-(4-isopropylphenyl)ethyl](methyl)sulfamoyl]phenoxide (Compound 31)

¹H-NMR (DMSO-d₆) δ ppm:
1.17 (6H, d, J=6.9Hz), 2.36 (3H, s), 2.67-2.75 (5H, m), 2.78-2.87 (1H, m), 3.20-3.45 (5H, m), 4.83 (2H, s), 6.27 (1H, d, J=8.8Hz), 7.08-7.17 (4H, m), 7.73 (1H, dd, J=8.8, 2.5Hz), 8.29 (1H, d, J=2.5Hz), 8.73 (1H, br s), 9.08 (1H, br s)

### Methylammonium 4-[amino[(tetrahydrofuran-2-yl)methoxycarbonylimino]methyl]-2-[[2-(4-isopropylphenyl)ethyl](methyl)sulfamoyl]phenoxide (Compound 32)

¹H-NMR (DMSO-d₆) δ ppm:
1.17 (6H, d, J=6.9Hz), 1.52-1.62 (1H, m), 1.74-1.98 (3H, m), 2.35 (3H, s), 2.67-2.76 (5H, m), 2.78-2.87 (1H, m), 3.25-3.34 (2H, m), 3.60-3.67 (1H, m), 3.71-3.80 (1H, m), 3.89-3.96 (2H, m), 3.98-4.07 (1H, m), 6.28 (1H, d, J=8.8Hz), 7.06-7.16 (4H, m), 7.69 (1H, dd, J=8.8, 2.8Hz), 8.25 (1H, d, J=2.8Hz), 8.48 (1H, br s), 9.09 (1H, br s)

### Methylammonium 4-[amino-(2-benzyloxyethoxycarbanylimino)methyl]-2-[[2-(4-isopropylphenyl)ethyl](methyl)sulfamoyl]phenoxide (Compound 33)

¹H-NMR (DMSO-d₆) δ ppm:
1.16 (6H, d, J=6.9Hz), 2.36 (3H, s), 2.67-2.74 (5H, m), 2.82 (1H, sept, J=6.9Hz), 3.29-3.33 (2H, m), 3.63-3.67 (2H, m), 4.11-4.15 (2H, m), 4.52 (2H, s), 6.31 (1H, t, J=8.8Hz), 7.06-7.15 (4H, m), 7.25-7.31 (1H, m), 7.32-7.37 (4H, m), 7.71 (1H, dd, J=8.8, 2.5Hz), 8.27 (1H, d, J=2.5Hz), 8.30-9.40 (2H, br)

### Methyloammonium 4-[amino-(2-methoxyethoxycarbonylimino)methyl]-2-[[2-(4-isopropylphenyl)ethyl](methyl)sulfamoyl]phenoxide (Compound 34)

¹H-NMR (DMSO-d₆) δ ppm:
1.17 (6H, d, J=6.9Hz), 2.35 (3H, s), 2.66-2.74 (5H, m), 2.83 (1H, sept, J=6.9Hz), 3.26-3.32 (5H, m), 3.51-3.55 (2H, m), 4.05-4.09 (2H, m), 6.26 (1H, d, J=9.1Hz), 7.08-7.15 (4H, m), 7.68 (1H, dd, J=9.1, 2.5Hz), 8.26 (1H, d, J=2.5Hz), 8.30-8.70 (1H, br), 8.80-9.30 (1H, br)

### Methylammonium 4-[amino(methoxycarbanylimino)methyl]-2-[[2-(4-isopropylphenyl)ethyl](methyl)sulfamoyl]phenoxide (Compound 35)

¹H-NMR (DMSO-d₆) δ ppm:
1.17 (6H, d, J=6.9Hz), 2.36 (3H, s), 2.68-2.74 (5H, m), 2.83 (1H, sept, J=6.9Hz), 3.55 (3H, s), 6.26-6.37 (1H, m), 7.07-7.16 (4H, m), 7.70 (1H, dd, J=8.8, 2.8Hz), 8.26 (1H, d, J=2.8Hz), 8.30-9.30 (2H, br)

### 5-[Amino(phenoxycarbonylimino)methyl]-2-hydroxy-N-[2-(4-iso propylphenyl)ethyl]-N-methylbenzenesulfonamide (Compound 36)

¹H-NMR (DMSO-d₆) δ ppm:
1.42 (6H, d, J=6.9Hz), 2.72-2.78 (2H, m), 2.80 (3H, s), 2.84 (1H, sept, J=6.9Hz), 7.05 (1H, d, J=8.8Hz), 7.10-7.24 (7H, m), 7.37-7.42 (2H, m), 8.10 (1H, dd, J=8.8, 2.5Hz), 8.43 (1H, d, J=2.5Hz), 9.15-9.40 (2H, br), 11.55 (1H, br s)

### Example 14

### 5-[Amino-(2-morpholin-4-ylethoxycarbonylimino)methyl]-2-hydroxy-N-[2-(4-isopropylphenyl)ethyl]-N-methylbenzenesulfonamide (Compound 37)

A solution of 0.12 g of 5-carbamimidoyl-2-hydroxy-*N*-[2-(4-isopropylphenyl)ethyl]-*N*-methylbenzenesulfonamide and 0.27 g of *N*-[2-(morpholin-4-yl)ethoxycarbonyloxy]succinimide in 1. 0 mL of *N,N*-dimethylformamide was stirred at room temperature for 1.7 hours. To the reaction mixture was added water, and extracted with ethyl acetate. The organic layer was washed with water and brine, dried over anhydrous magnesium sulfate. The solvent was removed under reduced pressure, and the residue was purified by column chromatography on silica gel (eluent: ethyl acetate-ethanol) to give 67 mg of 5-[amino-(2-morpholin-4-ylethoxycarbonylimino)methyl]-2-hydroxy-*N*-[2-(4-isopropylphenyl)ethyl]-*N*-methylbenzenesulfonamide.
¹H-NMR (CDCl₃) δ ppm:
1.23 (6H, d, J=6.9Hz), 2.50-2.60 (4H, m), 2.72 (2H, t, J=6.0Hz), 2.78-2.93 (6H, m), 3.29-3.38 (2H, m), 3.66-3.77 (4H, m), 4.29 (2H, t, J=6.0Hz),6.08-6.89 (1H, br), 7.07-7.13 (3H, m), 7.16 ( 2H, d, J=7.9Hz), 7.98-8.02(1H, m), 8.06 (1H, dd, J=8.5, 2.5Hz), 8.90-10.13 (1H, br)

### Example 15

### Amino-[4-hydroxy-3-[[2-(4-isopropylphenyl)ethyl]-(2-morpholin-4-yl-2-oxoethyl)sulfamoyl]phenyl]methylenecarbamoyloxymethy 2-acetoxy-2-methylpropionate (Compound 38)

To a solution of 0.069 mL of 5-carbamimidoyl-2-hydroxy-*N*-[2-(4-isopropylphenyl)ethyl]-*N*-(2-morpholin-4-yl-2-ox oethyl)benzensulfonamide in *N,N*-dimethylformamide was added 136 mg of 4-nitrophenoxycarbonyloxymethyl 2-acetoxy-2-methylpropionate under ice-cooling with stirring, and the reaction mixture was stirred at the same temperature for 1 hour. To the reaction mixture was added ethyl acetate, and washed with water and brine, and dried over anhydrous magnesium sulfate. The solvent was removed under reduced pressure, and the residue was purified by preparative thin layer chromatography on silica gel (developing solvent: ethyl acetate) to give 32 mg of amino-[4-hydroxy-3-[[2-(4-isopropylphenyl)ethyl]-(2-morpholin-4-yl-2-oxoethyl)sulfamoyl]phenyl]methylenecarbamoyloxymethy 2-acetoxy-2-methylpropionate.
¹H-NMR (DMSO-d₆) δ ppm:
1.15 (6H, d, J=7.0Hz), 1.47 (6H, s), 1.99 (3H, s), 2.68 (2H, t, J=7.6Hz), 2.75-2.85 (1H, m), 3.20-3.60 (10H, m), 4.25 (2H, s), 5.73 (2H, s), 6.95-7.15 (5H, m), 8.07 (1H, dd, J=8.5, 2.1Hz), 8.43 (1H, d, J=2.1Hz), 9.15-9.40 (2H, m), 11.60-12.00 (1H, br)

### Reference example 58

### 5-Cyano-2-hydroxy-N-[2-(4-isopropylphenyl)ethyl]benzenesulfonamide

A suspension of 4.33 g of 5-cyano-N-[2-(4-isopropylphenyl)ethyl]-2-methoxybenzenesulfonamide and 1.54 g of lithium chloride in 50 mL of *N,N*-dimethylformamide was stirred at 140°C for 6 hours, and then stirred at room temperature for 36 hours. To the reaction mixture was added hydrochloric acid (1 mol/L), and extracted with ethyl acetate. The organic layer was washed with hydrochloric acid and brine, dried over anhydrous magnesium sulfate. The solvent was removed under reduced pressure to give 4.84 g of 5-Cyano-2-hydroxy-*N*-[2-(4-isopropylphenyl)ethyl]benzenesulfonamide as yellow solid.
¹H-NMR (CDCl₃) δ ppm:
1.20-1.30 (6H, m), 2.70-2.95 (3H, m), 3.25-3.35 (2H, m), 4.60-4.80 (1H, br), 6.95-7.05 (2H, m), 7.10-7.20 (3H, m), 7.65-7.75 (1H, m), 7.90 (1H, d, J=2.0Hz)

### Reference example 59

### tert-Butyl N-(5-cyano-2-hydroxybenzenesulfonyl)-N-[2-(4-isopropylphenyl)ethyl]carbamate

To a solution of 4.16 g of 5-cyano-2-hydroxy-*N*-[2-(4-isopropylphenyl)ethyl]-*N*-benzenesulfonamide in dichloromethane were added 0.30 g of N,N-dimethylaminopyridine, 2.77 g of tert-butyl carbonate and 6.16 mL of diisopropylethylamine at room temperature with stirring, the reaction mixture was stirred for 12 hours. The solvent was removed under reduced pressure, the residue was dissolved in ethyl acetate. The organic layer was washed with phosphate buffer, diluted hydrochloric acid and brine successively, and dried over anhydrous magnesium sulfate. The solvent was removed under reduced pressure, and the residue was purified by medium pressure liquid column chromatography on silica gel (eluent: ethyl acetate-hexane) to give 3.56 g of *tert*-butyl *N*-(5-cyano-2-hydroxybenzenesulfonyl)-*N*-[2-(4-isopropylphenyl)ethyl]carbamate.
¹H-NMR (CDCl₃) δ ppm:
1.24 (6H, d, J=6.9Hz), 1.36 (9H, s), 2.80-2.95 (1H, m), 2.99 (2H, t, J=7.6Hz), 4.01 (2H, t, J=7.6Hz), 7.11 (1H, d, J=8.8Hz), 7.15-7.25 (4H, m),7.70 (1H, dd, J=8.8, 2.1Hz), 7.85 (1H, d, J=2.1Hz), 9.50 (1H, br s)

### Reference example 60

### tert-Butyl N-[5-cyano-2-(4-methoxybenzyloxy)benzenesulfonyl-N-[2-(4-isopropylphenyl)ethyl]carbamate

To a solution of 3.55 g of *tert*-butyl *N*-(5-cyano-2-hydroxybenzenesulfonyl)-*N*-[2-(4-isopropylphenyl)ethyl]carbamate, 5.20 g of cesium carbonate and 0.24 g of sodium iodide in *N,N*-dimethylformamide was added 1.63 mL of 4-methoxybenzyl chloride under ice-cooling with stirring, and the mixture was stirred for 10 minutes, then heated at 50°C for 1.5 hours. To the reaction mixture was added diluted hydrochloric acid, and extracted with ethyl acetate. The organic layer was washed with diluted hydrochloric acid and brine, and dried over anhydrous magnesium sulfate. The solvent was remived under reduced pressure, the formed crystal was suspended in petroleum ether, and collected by filtration to give 4.34 g of *tert*-butyl *N*-[5-cyano-2-(4-methoxybenzyloxy)benzenesulfonyl-N-[2-(4-isopropylphenyl)ethyl]-carbamate.
¹H-NMR (CDCl₃) δ ppm:
1.24 (6H, d, J=6.9Hz), 1.27 (9H, s), 2.80-2.95 (3H, m), 3.55-3.70 (2H, m), 3.74 (3H, s), 5.14 (2H, s), 6.81 (2H, d, J=8.7Hz), 7.03 (2H, d, J=8.1Hz), 7.10-7.20 (3H, m), 7.20-7.30 (2H, m), 7.78 (1H, dd, J=8.7, 2.2Hz), 8.32 (1H, d, J=2.2Hz)

### Reference example 61

### 5-Cyano-N-[2-(4-isopropylphenyl)ethyl]-2-(4-methoxybenzyloxy)benzenesulfonamide

The solution of 4.34 g of tert-Butyl *N*-(5-cyano-2-(4-methoxybenzyloxy)benzenesulfonyl]-*N*-[2-(4-isopropylphenyl)-ethyl]carbamete in 40 mL of *N,N*-dimethylformanide was heated at 140°C for 2 hours. After the reaction mixture was cooling, to the reaction mixture was added diluted hydrochloric acid, and extracted with ethyl acetate. The organic layer was washed with water and brine, and dried over anhydrous magnesium sulfate. The solvent was removed under reduced pressure, and the residue was purified by medium pressure liquid column chromatography on amin-propylated silica gel (eluent: ethyl acetate-hexane) to give 1.25 g of 5-cyano-*N*-[2-(4-isopropylphenyl)ethyl]-2-(4-methoxybenzyloxy)benzenesulfonamide.
¹H-NMR (CDCl₃) δ ppm:
1.02 (6H, d, J=6.9Hz), 2.66 (1H, t, J=6.8Hz), 2.80-2.95 (1H, m), 3.16 (2H, q, J=6.8Hz), 3.82 (3H, s), 4.70-4.85 (1H, m), 5.03 (2H, s), 6.85-6.95 (4H, m), 7.06 (1H, d, J=8.6Hz), 7.09 (2H, d, J=8.1Hz), 7.24 (2H, d, J=8.6Hz), 7.76 (1H, dd, J=8.6, 2.2Hz), 8.21 (1H, d, J=2.2Hz)

### Example 16

### Ethyl [(5-cyano-2-hydroxybenzenesulfonyl)-[2-(4-isopropylphenyl)ethyl]amino]acetate (Compound 8)

A suspension of 6.20g of Ethyl [*N*-(5-cyano-2-methoxybenzenesulfonyl)-*N*-[2-(4-isopropylphenyl)ethyl]amino]acetat e and 1.77 g of lithium chloride in 70 mL of *N,N*-dimethylsulfonamide was heated at 140°C for 5 hours. After the reaction mixture was cooled, to the reaction mixture were added hydrochloric acid (1mol/mL) and ethyl acetate, and the mixture was stirred at room temperature for 5 hours. After the organic layer was separated, the aqueous layer was extracted with ethyl acetate, the combied organic layer was washed with water and brine, and dried over anhydrous magnesium sulfate. The solvent was removed under reduced pressure, and the residue was purified by medium pressure liquid column chromatography on silica gel (eluent: dichloromethane -methanol) to give 1. 31 g of Ethyl [(5-cyano-2-hydroxybenzenesulfonyl)-[2-(4-isopropylphenyl)ethyl]amino]acetate.
¹H-NMR (CDCl₃) δ ppm:
1.15-1.35 (9H, m), 2.75-2.95 (3H, m), 3.44 (2H, t, J=7.6Hz), 4.04 (2H, s), 4.15 (2H, q, J=4.15Hz), 7.03 (2H, d, J=8.0Hz), 7.08 (1H, d, J=8.7Hz), 7.15 (2H, d, J=8.0Hz), 7.68 (1H, dd, J=8.7, 2.1Hz), 7.99 (1H, d, J=2.1Hz), 9.53 (1H, s)

### Example 17

### [[5-Cyano-2-hydroxybenzensulfonyl]-[2-(4-isopropylpheny)ethyl]amino]acetic acid (compound 39)

To the ethanol(15mL) solution of ethyl [(5-cyano-2-hydroxybenzenesulfonyl)-[2-(4-isopropylphenyl)ethyl]amino]acetate(1.30g) was added sodium hydroxide solution (2mol/L, 4.5mL) under room temperature with stirring, and made to reaction for 12 hours. To the reaction mixture was added hydrochloric acid(2mol/L, 4.5mL) under ice cooling, extracted with ethyl acetate. The organic layer was extracted with ethyl acetate. After the organic layer was washed with brine, The extract was dried over anhydrous magnesium sulfate to give [(5-cyanao-2-hydroxybenzenesulfonamide(1.23g).
¹H-NMR (CDCl₃) δ ppm:
1.23 (6H, d, d=6.9Hz), 2.80-2.95 (3H, m) , 3.45 (2H, t, J=7.6Hz), 4.11 (2H, s), 7.04 (2H, d, J=8.0Hz), 7.08 (1H, d, J=8.7Hz), 7.15 (2H, d, J=8.0Hz), 7.68 (1H, dd, J=8.7, 2.0Hz), 7.97 (1H, d, J=2.0Hz)

### Example 18

### 5-Amidino-2-hydroxy-N-[2-(4-isopropylphenyl)ethyl]-N-(pyridin-4-ylmethyl)benzenesufonamide (compound 40)

To the Diphenylphosphino-polystyrene (65mg, 0.19mmol, Fluka, 200-400mesh, crosslinked with 2% DVB, 3.0mmol phosphine/g resin) was added at room temperature a solution of 5-cyano-*N*-[2-(4-isopropylphenyl)ethyl]-2-(4-methoxybenzyl)benzenesulfonamide (0.065mmol) in tetrahydrofuran (0.323M, 0.2mL) and a solution of 4-pyridylmethanol (0.194mmol) in tetrahydrofuran (0.969M, 0.2ml) followed by a solution of diisopropylazodicarboxylate (0.194mmol) in toluene (1.938M, 0.1ml). The mixture was agitated at room temperature for 7hours. To the mixture was added an additional solution of diisopropylazodicarboxylate (0.064mmol) in toluene (1.938M, 0.034ml) and agitating for 8 hours. The resin was removed by the filtration and the filtrate was concentrated under reduced pressure. To the residue was added 38% HCl-EtOH (0.65ml) and agitated at room temperature for 15 hours. The solvent was removed under reduced pressure. To the residue was added a solution of ammonium acetate in ethanol (0.8M, 0.65ml) and agitated at room temperature for 8 hours. To the reaction mixture was added ethyl acetate (2ml) and treated with NH₂ ion exchange cartridge (Varian, 500mg, pre-conditioned with ethyl acetate, washing with ethyl acetate, eluting with ethanol) and concentrated under reduced pressure. The residue was dissolved in methanol (2ml) and treated with SCX ion exchange cartridge (Varian, 500mg, pre-conditioned with methanol, washing with methanol, eluting with 2mol/L-ammonia in methanol). The filtrate was concentrated under reduced pressure to give 5-amidino-2-hydroxy-*N*-[2-(4-isopropylphenyl)ethyl]-*N*-(pyridine-4-ylmethyl)benzenesulfonamide.
MS(m/e):453(M+H)

### Example 19

The following compounds were prepared in a similar manner to that described in Example 18 using 2-methoxyethanol, 2-(2-methoxyethoxy)ethanol, 3-pyridylmethanol, 2-ethoxyethanol, allylalcohol, propargylalcohol, 2-isopropoxyethanol, 2-(thiophen-2-yl)ethanol, 2-allyloxyethanol, 2-propoxyethanol, 3-(3-methoxypropyloxy)propanol, 2-butyn-1-ol, 2-(2-ethoxyethoxy)ethanol, (S)-5-hydroxymethyl-2-pyrrolidinone or 4-methoxybutanol respectively instead of 4-pyridylmethanol.

### 5-Amidino-2-hydroxy-N-[2-(4-isopropylphenyl)ethyl]-N-(2-methoxyethyl)benzenesulfonamide (compound41)

MS(m/e):420(M+H)

### 5-Amidino-2-hydroxy-N-[2-(4-isopropylphenyl)ethyl]-N-[2-(2-methoxyethoxy)ethyl]benzensulfonamide (compound 42)

MS(m/e):464(M+H)

### 5-Amidino-2-hydroxy-N-[2-(4-isopropylphenyl)ethyl]-N-(pyridin-3-ylmethyl9benzenesulfonamide (compound 43)

MS(m/e):453(M+H)

### 5-Amidino-N-(2-ethoxyethyl)-2-hydroxy-N-[2-(4-isopropylphenyl)ethyl]benzenesulfonamide (compound 44)

MS(m/e):434(M+H)

### N-Allyl-5-amidino-2-hydroxy-N-[2-(4-isopropylphenyl)ethyl]benzenesulfonamide (compound 45)

MS(m/e):402(M+H)

### 5-Amidino-2-hydroxy-N-[2-(4-isopropylphenyl)ethyl]-N-propargylbenzenesulfonamide (compound 46)

MS(m/e):400(M+H)

### 5-Amidino-2-hydroxy-N-(2-isopropoxyethyl)-N-[2-(4-isopropyl phenyl)ethyl]benzenesulfonamide (compound 47)

MS(m/e):448(M+H)

### 5-Amidino-2-hydroxy-N-[2-(4-isopropylphenyl)ethyl]-N-[2-(thiophen-2-yl)ethyl]benzenesulfonamide (compound 48)

MS(m/e):472(M+H)

### N-(2-Allyloxyethyl)-5-amidino-2-hydroxy-N-[2-(4-isopropylphenyl)ethyl]benzenesulfonamide (compound 49)

MS(m/e):446(M+H)

### 5-Amidino-2-hydroxy-N-[2-(4-isopropylphenyl)ethyl]-N-(2-propoxyethyl)benzenesulfonamide (compound 50)

MS(m/e):448(M+H)

### 5-Amidino-2-hydroxy-N-[2-(4-isopropylphenyl)ethyl]-N-[3-(3-methoxypropyloxy)benzenesulfonamide (compound 51)

MS(m/e):492(M+H)

### 5-Amidino-N-(2-butyn-1-yl)-2-hydroxy-N-[2-(4-isopropylphenyl)ethyl]benzenesulfonamide (compound 52)

MS(m/e):414(M+H)

### 5-Amidino-N-[2-(2-ethoxyethoxy)ethyl]-2-hydroxy-N-[2-(4-isopropylpheny)ethyl]benzenesulfonamide (compound 53)

MS(m/e):478(M+H)

### (S)-5-Amidino-2-hydroxy-N-[2-(4-isopropylphenyl)ethyl]-N-(2-pyrroridinon-5-ylmethyl)benzenesulfonamide (compound 54)

MS(m/e):459(M+H)

### 5-Amidino-2-hydroxy-N-[2-(4-isopropylphenyl)ethyl]-N-(4-methoxybutyl)benzenesulfonamide (compound 55)

MS(m/e):448(M+H)

### Reference example 62

### Thiophenol resin

To a suspension of [3-(3-mercaptophenyl)propyl]aminomethylpolystyrene (10g, 14.3mmol, Argonaut, 100-200 mesh, crosslinked with 1% DVB, 1.43mmol SH/g resin) in the solution (100ml, 95:5 tetrahydrofuran/water) was added n-tributylphosphinie (17.6mL) and agitated at room temperature for 1.5 hours and the resin was washed with tetrahydrofuran (deoxygenated) several times. The resulting resin was dried in vacuo to give polymer bound scavenger thiophenol resin.

### Example 20

To each of the 5 columns in a 48 well polypropylene reaction block (Robins Flexchem) was added *N,N*-(diisopropyl)aminomethylporystyrene (435mg, 1.5mmol, Argonaut, 100-200 mesh, crosslinked with 2% DVB, 3.45mmol diisopropylmetylamine/g resin) and primary alcohol (0.50mmol) of 5-methylisoxazole-3-methanol, 6-methylpyridine-2-methanol, 2-methylthiazole-4-methanol, 3-fluorobenzylalcohol or 3-methoxycarbonylbenzylaocohol, one alcohol per column, as a stock solution in dichloromethane (0.417M, 1.2mL) and the methanesulfonyl chloride(0.058mL) was added to each columns. The plate was sealed and agitated at room temperature for 2 hours. The product solutions were filtered through the frits in the bottom of the wells directly into 48 deep well plate. The solvent was removed under reduced pressure to give methanesulfonic acid 5-methyl-isoxazol-3-ylmethyl ester, methanesulfonic acid 6-methyl-pyridin-2-ylmethyl ester, methanesulfonic acid 2-methyl-thiazol-4-ylmethyl ester, methanesulfonic acid 3-fluoro-benzyl ester or methanesulfonic acid 3-methoxycarbonyl-benzyl ester respectively. Each of resulting 5 methane sulfonic acid esters was treated one by one as follows. Cesium carbonate (84mg) was placed in the reaction vessel and a solution of a methane sulfonic acid ester described above (0.129mmol) in *N*,*N*-dimethylfolmamide (0.645M, 0.2mL) and a solution of 5-Cyano-*N*-[2-(4-isopropylphenyl)ethyl]-2-(4-methoxybenzyloxy)benzenesulfonamide (0.043mmol) in tetrahydrofuran (0.215M, 0.2mL) were added at room temperature then agitated for 12 hours. To the mixture was added a suspension of chloromethylpolystyrene (143mg, 0.129mmol, Watanabe, 100-200 mesh, crosslinked with 2% DVB, 0.9mmol Cl/g resin) in *N,N*-dimethylformamide (1mL) and agitated at 50? for 5 hours. Subsequently to the mixture was added a suspension of thiophenol resin (262mg, 0.387mmol) in the solution of ethanol (1ml) and tetrahydrofuran (1ml) and reacted at room temperature for 12 hours. After the resins were filtered, the filtrate was concentrated. To the resulting residue was added ethyl acetate (1mL) and water (1mL) then the mixture was shaken. The mixture was added onto a diatomaceous earth column (Varian, chemelute, pre-conditioned with 1mL water, eluting with ethyl acetate) and concentrated under reduced pressure.

To the residue was added 33% HCl-EtOH (0.50ml) and agitated at room temperature for 4 hours. The solvent was removed under the reduced pressure. To the residue was added a solution of ammonium acetate in ethanol (0.8M, 0.50ml) and agitated at room temperature for 12 hours. To the reaction mixture was added ethyl acetate (2ml) and treated with NH2 ion exchange cartridge (Varian, 500mg, pre-conditioned with ethyl acetate, washing with ethyl acetate and water, eluting with ethanol) and concentrated under reduced pressure to give following compounds.

### 5-Amidino-2-hydroxy-N-[2-(4-isopropylphenyl)ethyl]-N-(5-methylisoxazol-3-ylmethyl)benzenesulfonamide (compound 56)

MS(m/e):467(M+H)

### 5-Amidino-2-hydroxy-N-[2-(4-isopropyl)ethy-N-(6-methylpyridin-2-ylmethyl)benenesulfonamide (compound 57)

MS(m/e):467(M+H)

### 5-Amidino-2-hydroxy-N-[2-(4-isopropylpheneyl)ethyl]-N-(2-methylthiazol-4-ylmethyl)benzenesulfonamide (compound 58)

MS(m/e):473(M+H)

### 5-Amidino-N-(3-fluorobenzyl)-2-hydroxy-N-[2-(4-isopropylphenyl)ethyl]benzenesulfonamide (compound 59)

MS(m/e):470(M+H)

### Methyl 3-[[(5-amidino-2-hydroxybenzenesulfonyl)-[2-(4-isopropylphenyl)ethyl]amino]methyl]benzoate (Compound 60)

MS(m/e):510(M+H)

### Example 21

The following compounds were prepared in a similar manner to that described in Example 20 using *N*-(2-hydroxyethyl)-2-pyrrolidinone, 2-phenoxyethanol, 2-(4-methylthiazol-5-yl)ethanol, 2-(2-pyridyl)ethanol, 1-(2-hydroxyethyl)-2-imidazolidinone, 2-(3,5-dimethylpyrazol-1-yl)-ethanol, 3-chlorobenzylalcohol, 3-methoxybenzylalcohol, 3-nitrobenzylalcohol, (5-phenyl-1,2,4-oxadiazole)-3-methanol, 3-trifluoromethylbenzylalcohol, 3-cyanobenzylalcohol, 5-chlorothiophen-2-methanol, 3,4-difluorobenzylalcohol, 4-fluorobenzylalcohol or 2-chloro-4,5-methylenedioxybenzylalcohol respectively instead of the above 1° alcohol.

### 5-Amidino-2-hydroxy-N-[2-(4-isopropylphenyl)ethyl]-N-[2-(2-pyrroridin-1-yl)ethyl]benzenesulfonamide (compound 61)

MS(m/e):473(M+H)

### 5-Amidino-2-hydroxy-N-[2-(4-isopropylphenyl)ethyl]-N-(2-phenoxyethyl)benzenesulfonamide (compound 62)

MS(m/e):482(M+H)

### 5-Amidino-2-hydroxy-N-[2-(4-isopropylphenyl)ethyl]-N-[2-(4-methylthiazol-5-yl)ethyl]benzenesulfonamide (compound 63)

MS(m/e):487(M+H)

### 5-Amidino-2-hydroxy-N-[2-(4-isopropylphenyl)ethyl]-N-[2-(2-pyridyl)ethyl]benzenesulfonamide (compound 64)

MS(m/e):467(M+H)

### 5-Amidino-2-hydroxy-N-[2-(2-imidazolidin-1-yl)ethyl]-N-[2-(4-isopropylpheny)ethyl]benzenesulfonamide (compound 65)

MS(m/e):474(M+H)

### 5-Amidino-N-[2-(3,5-dimethylpyrazol-1-yl)ethyl]-2-hydoxy-N-[2-(4-isopropylphenyl)ethyl]benzenesulfonamide (compound 66)

MS(m/e):484(M+H)

### 5-Amidino-N-(3-chlorobenzyl)-2-hydroxy-N-[2-(4-isopropylphenyl)ethyl]benzenesulfonamide (compound 67)

MS(m/e):486(M+H)

### 5-Amidino-2-hydroxy-N-[2-(4-isopropylphenyl)ethyl]-N-(3-methoxybenzyl)benzenesulfonamide (compound 68)

MS(m/e):482(M+H)

### 5-Amidino-2-hydroxy-N-[2-(4-isopropylphenyl)ethyl]-N-(3-nitrobenzyl)benzenesulfonamide (compound 69)

MS(m/e):497(M+H)

### 5-Amidino-2-hydroxy-N-[2-(4-isopropylphenyl)ethyl]-N-(5-phenyl-1,2,4-oxadiazol-3-ylmethyl)benzenesulfonamide (compound 70)

MS(m/e):520(M+H)

### 5-Amidino-2-hydroxy-N-[2-(4-isopropylphenyl)ethyl]-N-(3-trifluoromethylbenxyl)benzenesulfonamide (compound 71)

MS(m/e):520(M+H)

### 5-Amidino-N-(3-amidinobenzyl)-2-hydroxy-N-[2-(4-isopropylphenyl)ethyl]benzenesulfonamide (compound 72)

MS(m/e):494(M+H)

### 5-Amidino-N-(2-chlorothiophen-5-ylmethyl)-2-hydroxy-N-[2-(4-isopropyl)ethyl]benzenesulfonamide (compound 73)

MS(m/e):492(M+H)

### 5-Amidino-N-(3,4-difluorobenzyl)-2-hydroxy-N-[2-(4-isopropylphenyl)ethyl]benzenesulofonamide (compound 74)

MS(m/e):488(M+H)

### 5-Amidino-N-(4-fluorobenzyl)-2-hydroxy-N-[2-(4-isopropyl phenyl)ethyl]benzenesulfonamide (compound 75)

MS(m/e):470(M+H)

### 5-Aminidino-N-(2-chloro-4,5-methylenedioxybenzyl)-2-hydroxy-N-[2(4-isopropylphenyl)ethyl]benzenesulfonamide (Compound 76)

MS(m/e):530(M+H)

### Example 22

To each of the 5 reaction vessels in the semi-automated synthesizer Quest210 (Argonaut) was added a suspension of *N*-cyclohexylcarbodiimide-N'-methylpolystyrene (144mg, 0.244mmol, Novabiochem, 200-400 mesh, crosslinked with 2% DVB, 1.55mmol *N*-cyclohexyl-N'-methylcarbodiimide /g resin) in the 10% dichloromethane/*N,N*-dimethylformamide(1m1) at room temperature and to the mixture was added a solution of {(5-Cyano-2-hydroxy-benzenesulfonyl)-[2-(4-isopropyl-phenyl )-ethyl]-amino}-acetic acid (0.149mmol) ) in the 10% dichloromethane/*N,N*-dimethylformamide(0.149M, 1.0ml). To each of the 5 columns was added amine (0.134mmol) of (±)-3-hydroxypiperidine, 2-aminoethanol, 3-aminobenzamide, 2-aminothazole-4-acetic acid ethyl ester or (±)-3-piperidinecarboxamide, one amine per column, as a stock solution in 10% dichloromethane/*N,N*-dimethylformamide (2.06M, 0.65mL) and agitated at room temperature for 6 hours. The product solutions were filtered through the frits in the bottom of the columns directly into vials and the solvent was removed under reduced pressure. Each of resulting 5 acetamide derivatives was treated one by one as follows. A acetamide derivative was dissolved in 50% ethylacetate/ethanol and treated with NH₂ ion exchange cartridge (Varian, 2.0g, pre-conditioned with ethyl acetate, washing with 50% ethyl acetate/ethanol and methanol, eluting with 20% 1mol/L-hydrochloric acid/acetnitrile) and concentrated under reduced pressure.

To the residue was added 34% HCl-EtOH (1.65ml) and agitated at room temperature for 4 hours. The solvent was removed under the reduced pressure and to the residue was added a solution of ammonium acetate (1.19mmol) in ethanol (0.8M, 1.50ml) and agitated at room temperature for 12 hours. To the reaction mixture was added ethyl acetate and treated with NH₂ ion exchange cartridge (Varian, 500mg, pre-conditioned with ethyl acetate, washing with ethyl acetate, eluting with methanol) and concentrated under reduced pressure. The residue was dissolved in *N,N*-dimethylformamide and methanol then treated with SCX ion exchange cartridge (Varian, 500mg, pre-conditioned with methanol, washing with methanol and 0.56% aqueous ammonia solution/acetnitrile, eluting with 10% 1mol/L hydrochloric acid/acetnitrile) and the filtrate was concentrated under the reduced pressure. The residue was dissolved in methanol and treated with NH2 ion exchange cartridge (Varian, 500mg, pre-conditioned with methanol, eluting with methanol) and concentrated under reduced pressure to give following compounds.

### (±) 5-Amidino-2-hydroxy-N-[2-(3-hydroxypiperidin-1-yl)-2-oxoethyl]-N-[2-(4-isopropylphenyl)ethyl]benzenesulfonamide (compound 77)

MS(m/e):503(M+H)

### 2-[(5-Amidino-2-hydroxybenzenesulfonyl)-[2-(4-isopropylphenyl)ethyl]amino]-N-(2-hydroxyethyl)acetamide (compound 78)

MS(m/e):463(M+H)

### 3-[[[(5-Amidino-2-hydroxybenzenesulfonyl)-2-[2-(4-isopropylphenyl)ethyl]amino]acetyl]benzamide (compound 79)

MS(m/e):538(M+H)

### Ethyl [2-[[[(5-amidino-2-hydroxybenzenesulfonyl)-[2-(4-isopropylphenyl)ethyl]amino]acetyl]amino]thiazol-4-yl]acetate (compound 80)

MS(m/e):588(M+H)

### (±) -1-[[(5-Amidino-2-hydroxybenzenesulfony)-[2-(4-isopropylphenyl)ethyl]amino]acetyl]piperidine-3-carboxamide (compound 81)

MS(m/e):530(M+H)

### Example 23

The following compounds were prepared in a similar manner to that described in Example 22 using 2-(3-pyridyl)ethylamine, 2-piperazynylethanol, (±)-2-(*N*,*N*-dimethylamino)pyrrolidine, 4-aminobenzamide, 2-methoxyethylamine, 1-[3-(N,N-dimethylamino)propyl]piperazine, 4-cabamoylpiperidine, 2-aminopyridyl-5-carboxamide, 1-acetylpiperazine, (S)-proline methyl ester or 1-methylpiperazine respectively instead of the above amines.

### 2-[[(5-Amidino-2-hydroxybenzenesulfonyl)-[2-(4-isopropylphenyl)ethyl]]amino]-N-[2-(3-pyridyl)ethyl]acetamide (compound 82)

MS(m/e):524(M+H)

### 5-Amidino-2-hydroxy-N-[2-[4-hydroxyethyl]piperazin-1-yl]-2-oxoethyl]-N-[2-(4-isopropylphenyl)ethyl]benzenesufonamide (compound 83)

MS(m/e):532(M+H)

### (±) -5-amidino-N-[2-[3-(N,N-dimethylamino)-pyrrolidin-1-yl]-2-oxoethyl]-2-hydroxy-N-[2-(4-isopropylphenyl)ethyl]benzenesulfonamide (compound 84)

MS(m/e):516(M+H)

### 4-[[[(5-Amidino-2-hydroxybenzenesulfonyl)-[2-(4-isopropylphenyl)ethyl]amino]acetyl]amino]benzamide (compound 85)

MS(m/e):538(M+H)

### 2-[[(5-Amidino-2-hydroxybenzenesulfonyl)-[2-(4-isopropylphenyl)ethyl]]amino]-N-(2-methoxyethyl)acetaminde (compound 86)

MS(m/e):477(M+H)

### 5-Amidino-N-[2-[4-[3-(N,N-dimethylamino)propyl]piperazin-1-yl]-2-hydroxy-N-[2-(4-isopropylphenyl)ethyl]benzenesulfonamide (compound 87)

MS(m/e):573(M+H)

### 1-[[(5-Amidino-2-hydroxybenzenesulfonyl)-[2-(4-isopropylphenyl)ethyl]amino]acetyl]piperizine-4-carboxamide (Compound 88)

MS(m/e):530(M+H)

### 2-[[[(5-Amidino-2-hydroxybenzensulfonyl)-[2-(4-isopropylphenyl)ethyl]amino]acetyl]amino]pyridine-5-carboxamide (compound 89)

MS(m/e):539(M+H)

### N-[2-(4-Acetylpiperazin-1-yl)-2-oxoethyl]-5-amidino-2-hydroxy-N-[2-(4-isopropylphenyl)ethyl]benzenesulfonamide (compound 90)

MS(m/e):530(M+H)

### (S)-1-[[(5-Amidino-2-hydroxybenzenesulfonyl)-[2-(4-isopropylphenyl)ethyl]amino]acetyl]proline ethyl ester (compound 91)

MS(m/e):545(M+H)

### 5-Amidino-2-hydroxy-N-[2-(4-isopropylphenyl)ethyl]-N-[2-(4-methylpiperazin-1-yl)-2-oxoethyl]benzenesulfonamide (compound 92)

MS(m/e):502(M+H)

### Test Example 1

### Measurement of inhibitory activity for activated blood coagulation factor X

2.5 µL of a dimethylsulfoxide solution of a test compound, 187.5 µL of 100 mM tris-200 mM NaCl buffer (pH 8.4) and 50 µL of 1 mM S-2222 (Daiichi Pure Chemicals) aqueous solution were poured into 96 well microplate. Then 10 µL of 0.6 U/mL human activated blood coagulation factor X (Calbiochem) in gelatin-glycine buffer was added and the mixture was incubated for 10 minutes at 37 °C. The reaction was terminated with the addition of 50 µL of 60 % acetic acid and absorbance (405 nm) was measured by a microplate reader (SPECTRAmax250, Molecular Devices).

The group with 2.5 µL of the dimethylsulfoxide solution instead of the test compound solution was defined as the control, and the group with 10 µL of the gelatin-glycine buffer solution instead of human activated blood coagulation factor X was defined as the blank. The concentration of a test compound that inhibited the absorbance of control by 50% (IC₅₀) was obtained, and this value was used as the index of inhibitory activity for activated blood coagulation factor X. Results were shown as Table 1.

**[Table 1]**

| Test compound No. | Inhibitory activity for activated blood coagulation factor X (IC₅₀, µM) |
|---|---|
| Compound 14 | 0.051 |
| Compound 15 | 0.0038 |
| Compound 17 | 0.16 |

### Test Example 2

### Measurement of inhibitory activity for thrombin

2. 5 µL of a dimethylsulfoxide solution of a test compound, 187.5 µL of 100 mM tris-200 mM NaCl buffer (pH 8.4) and 50 µL of 1 mM S-2238 (Daiichi Pure Chemicals) aqueous solution were poured into 96 well microplate. Then 10 µL of 2.0 U/mL human thrombin (Sigma Chemical Company) in gelatin-glycine buffer was added and the mixture was incubated for 10 minutes at 37 °C. The reaction was terminated with the addition of 50 µL of 60 % acetic acid and absorbance (405 nm) was measured by a microplate reader (SPECTRAmax250, Molecular Devices).

The group with 2.5 µL of the dimethylsulfoxide solution instead of the test compound solution was defined as the control, and the group with 10 µL of the gelatin-glycine buffer solution instead of human thrombin was defined as the blank. The concentration of a test compound that inhibited the absorbance of control by 50% (IC₅₀) was obtained, and this value was used as the index of inhibitory activity for thrombin. Results were shown as Table 2.

**[Table 2]**

| Test compound No. | Inhibitory activity for thrombin (IC₅₀, µM) |
|---|---|
| Compound 14 | 27 |
| Compound 17 | 26 |

### Industrial Applicability

The 5-amidino-2-hydroxybenzenesulfonamide derivatives represented by the above general formulae (I) and pharmaceutically acceptable salts thereof of present inventors show a potent and selective activated blood coagulation factor X inhibitory activity. The present invention can provide novel compounds having excellent properties as activated blood coagulation factor X inhibitors. In addition, the 5-cyano-2-hydroxybenzenesulfonamide derivatives represented by the above general formulae (II) and salts thereof of the present invention are important as intermediates in the production of the compounds represented by the above general formula (I). Accordingly, the compounds represented by the above general formula (I) of the present invention can be readily prepared via these compounds.

## Claims

1. A 5-amidino-2-hydroxybenzenesulfonamide derivative represented by the general formula: 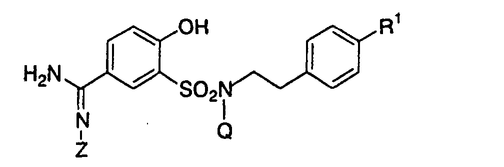 wherein R¹ represents a lower alkyl group which may have -COOR^{A} in which R^{A} is a hydrogen atom or a lower alkyl group, a lower alkoxy group which may have -COOR^{A} in which R^{A} has the same meaning as defined above, a lower alkenyl group which may have -COOR^{A} in which R^{A} has the same meaning as defined above , a 3 to 10-membered cycloalkyl group, a lower acyl group, -COOR^{A} in which R^{A} has same meaning as defined above, -CONR^{B}R^{C} in which R^{B} and R^{C} are independently a hydrogen atom or a lower alkyl group, or -NR^{B}R^{C} forms a cyclic amino group, an amino group, a mono or di ( lower alkyl)amino group, a cyclic amino group which may have an oxo group, a 3 to 10-membered heterocycloalkyl group which may have an oxo group, a 6 to 10-membered aryl group which may have one to three substituents selected from the following group (A), or a 5 to 10-membered aromatic heterocyclic group which may have a substituent selected from the following group (B);
Q represents a hydrogen atom, a lower alkenyl group, a lower alkynyl group or a lower alkyl group which may have a substituent selected from the following group (C);
Z represents a hydrogen atom, a hydroxy group, -COOR^{N} in which R^{N} is a halo (lower alkyl) group, a 6 to 10-membered aryl group or a lower alkyl group which may have a substituent selected from the following group (vii);
(A) an oxo group, a lower alkyl group, a halo (lower alkyl) group, -Y¹-R^{D} in which Y¹ is a oxygen atom or a sulfur atom; R^{D} is a hydrogen atom, a halo(lower alkyl) group or a lower alkyl group which may have -COOR^{D1} in which R^{D1} is a hydrogen atom, a 3 to 10-membered cycloalkyl group or a lower alkyl group, a halogen atom, a nitro group, an amino group, -COOR^{E} in which R^{E} is a hydrogen atom, a 3 to 10-membered cycloalkyl group or a lower alkyl group, a carbamoyl group, a sulfamoyl group, a (lower alkyl)sulfonyl group, a mono (lower alkyl)sulfamoyl group which may have -COOR^{F} in which R^{F} is a hydrogen atom, a 3 to 10-membered cycloalkyl group or a lower alkyl group, and a (lower alkyl)sulfonylamino-substituted (lower alkyl) group;
(B) a lower alkyl group, an amino group and -COOR^{G} in which R^{G} is a hydrogen atom, a 3 to 10-membered cycloalkyl group and a lower alkyl group;
(C) -OR^{H} in which R^{H} is a hydrogen atom, a lower alkenyl group, a phenyl group or a lower alkyl group which may have -OR^{H1} in which R^{H1} is a hydrogen atom or a lower alkyl group, -COOR^{I} in which R^{I} is a hydrogen atom, a 3 to 10-membered cycloalkyl group or a lower alkyl group which may have a substituent selected from the following group (iii), -CONR^{J}R^{K} in which R^{J} and R^{K} are independently a hydrogen atom, an amino group, a 6 to 10-membered aryl group which may have a carbamoyl group, a 5 to 10-membered aromatic heterocyclic group which may have a substituent selected from the following group (iv) or a lower alkyl group which may have a substituent selected from the following group (v) , or -NR^{J}R^{K} forms a cyclic amino group which may have a substituent selected from the following group (vi), a cyclic amino group which may have an oxo group, a 3 to 10-membered heterocycloalkyl group which may have an oxo group or a halogen atom, a 6 to 10-membered aryl group which may have one to three substituents selected from the following group (i), and a 5 to 10-membered aromatic heterocyclic group which may have one to three substituents selected from the following group (ii);
(i) a halogen atom, a nitro group, an amidino group, a lower alkyl group, a halo(lower alkyl) group, -OR^{L} in which R^{L} is a hydrogen atom or a lower alkyl group, and -COOR^{M} in which R^{M} is a hydrogen atom or a lower alkyl group;
(ii) a halogen atom, an oxo group, a lower alkyl group and a phenyl group;
(iii) -COOR^{I1} in which R^{I1} is a hydrogen atom, a 3 to 10-membered cycloalkyl group or a lower alkyl group, -OCOR^{I2} in which R^{I2} is a 3 to 10-membered cycloalkyl group or a lower alkyl group, -OCOOR^{I3} in which R^{I3} is a 3 to 10-membered cycloalkyl group or a lower alkyl group, -OR^{I4} in which R^{I4} is a hydrogen atom, a 3 to 10-membered cycloalkyl group or a lower alkyl group, -CONR^{I5}R^{I6} in which R^{I5} and R^{I6} are independently a hydrogen atom or a lower alkyl group, a 3 to 10-membered cycloalkyl group, a 6 to 10-membered aryl group, or -NR^{I5}R^{I6} forms a cyclic amino group, a 3 to 10-membered heterocycloalkyl group and a 5 to 10-membered aromatic heterocyclic group;
(iv) a halogen atom, a lower alkyl group which may have -COOR^{J1} in which R^{J1} is a hydrogen atom or a lower alkyl group, a carbamoyl group and -COOR^{J2} in which R^{J2} is a hydrogen atom or a lower alkyl group;
(v) -OR^{J3} in which R^{J3} is a hydrogen atom or a lower alkyl group, and a 5 to 10-membered aromatic heterocyclic group;
(vi) a hydroxy group, a lower alkyl group, a hydroxy (lower alkyl) group, a di(lower alkyl)amino-substituted (lower alkyl) group, a carbamoyl group, a di(lower alkyl)amino group, a lower acyl group, and -COOR^{J4} in which R^{J4} is a hydrogen atom or a lower alkyl group;
(vii) -OR^{N1} in which R^{N1} is a hydrogen atom or a lower alkyl group which may have a 6 to 10-membered aryl group, -COOR^{N2} in which R^{N2} is a lower alkyl group which may have -COOR^{N21} where R^{N21} is a lower alkyl group, -CONR^{N3}R^{N4} in which R^{N3} and R^{N4} are independently a hydrogen atom or a lower alkyl group, or -NRR^{N3}R^{N4} forms a cyclic amino group, -OCOR^{N5} in which R^{N5} is a lower alkyl which may have -OCOR^{N51} where R^{N51} is a lower alkyl group, a cyclic amino group, a 3 to 10-membered heterocycloalkyl group, and a 6 to 10-membered aryl group;
with the proviso that Q does not represent a hydrogen atom when Z represents a hydrogen atom and R¹ represents a lower alkyl group, a lower alkoxy group or a 3 to 10-membered heterocycloalkyl group, or a pharmaceutically acceptable salt thereof.

2. A 5-amidino-2-hydroxybenzenesulfonamide derivative as claimed in claim 1 or a pharmaceutically acceptable salt thereof , wherein R¹ represents a 6 to 10-membered aryl group which may have one to three substituents selected from the following group (A);
(A) an oxo group, a lower alkyl group, a halo (lower alkyl) group, -Y¹-R^{D} in which Y¹ is an oxygen atom or a sulfur atom; R^{D} is a hydrogen atom, a halo(lower alkyl) group, a lower alkyl group which may have -COOR^{D1} in which R^{D1} is a hydrogen atom, a 3 to 10-membered cycloalkyl group or a lower alkyl group, a halogen atom, a nitro group, an amino group, -COOR^{E} in which R^{E} is a hydrogen atom, a 3 to 10-membered cycloalkyl group or a lower alkyl group, a carbamoyl group, a sulfamoyl group, a (lower alkyl) sulfonyl group, a mono( lower alkyl) sulfamoyl group which may have -COOR^{F} in which R^{F} is a hydrogen atom, a 3 to 10-membered cycloalkyl group or a lower alkyl group, and a (lower alkyl)sulfonylamino-substituted (lower alkyl) group.

3. A pharmaceutical composition comprising as an active ingredient a 5-amidino-2-hydroxybenzenesulfonamide derivative as claimed in claim 1 or 2, or a pharmaceutically acceptable salt thereof.

4. An activated blood coagulation factor X inhibitor comprising as an active ingredient a 5-amidino-2-hydroxybenzenesulfonamide derivative as claimed in claim 1 or 2, or a pharmaceutically acceptable salt thereof.

5. An agent for the prevention or treatment of a disease occurred associating an activated blood coagulation factor X comprising as an active ingredient a 5-amidino-2-hydroxybenzenesulfonamide derivative as claimed in claim 1 or 2, or a pharmaceutically acceptable salt thereof.

6. An agent for the prevention or treatment as claimed in claim 5 wherein the disease occurred associating an activated blood coagulation factor X is a disease selected from the group consisting of cerebral infarction, cerebral thrombosis, cerebral embolism, transient cerebral ischemic attack, subarachnoid hemorrhage-induced cerebral vasospasm, alzheimer's disease, myocardial infarction, unstable angina, heart failure, thrombosis followed by atrial fibrillation, pulmonary infarction, pulmonary embolism, acute respiratory distress syndrome, Berger disease, peripheral arterial obstruction, deep vein thrombosis, disseminated intravascular coagulation syndrome, atherosclerosis, behcet's disease, diabetic neuropathy, diabetic nephropathy, diabetic retinopathy, diabetic thrombotic complications, acute progressive glomerulonephritis, chronic glomerulonephritis, IgA nephropathy, nephritic syndrome, focal segmental glomerulosclreosis, membranous nephropathy, membranoproliferative glomerulonephritis, crescentic glomerulonephritis, lupus nephritis, purpura nephritis, interplanting rejection, systemic inflammatory response syndrome, dialysis- or operation-induced thrombocytopenia, thrombus formation after artificial blood vessel operation or after artificial valve replacement, restenosis and reocculusion after coronary intervention, thrombus formation at the time of extracorporeal circulation, blood coagulation at the time of insertion of blood vessel catheter and influenza virus infection.

7. A method for the prevention or treatment of a disease occurred associating an activated blood coagulation factor X, which comprises administering a therapeutically effective amount of a 5-amidino-2-hydroxybenzenesulfonamide derivative as claimed in claim 1 or 2, or a pharmaceutically acceptable salt thereof.

8. A use of a 5-amidino-2-hydroxybenzenesulfonamide derivative as claimed in claim 1 or 2, or a pharmaceutically acceptable salt thereof for the manufacture of a pharmaceutical composition for the prevention or treatment of a disease occurred associating an activated blood coagulation factor X.

9. A pharmaceutical combination which comprises (a) a 5-amidino-2-hydroxybenzenesulfonamide derivative as claimed in claim 1 or 2, or a pharmaceutically acceptable salt thereof, and (b) at least one member selected from the group consisting of adrenocortical hormone, platelet aggregation inhibitors, adenylate cyclase activators, PGF2α antagonists, cyclooxygenase inhibitors, adenosine antagonists, GPIIb/IIIa antagonists, anticoagulants, thrombolitic drugs, antithrombin drugs, free-radical sacavengers, immunosuppressant drugs, erythropoietin, fish oil, angiotensin-converting enzyme inhibitors, angiotensin II receptor antagonists, glycation inhibitors, protein kinase C inhibitors, aldose reductase inhibitors, endothelin receptor antagonists, endothelin-converting enzyme inhibitors, neutral endopeptidase inhibitors, thromboxane A₂ synthetase inhibitors, thromboxane A₂ receptor antagonists and PGI₂ agonists.

10. An activated blood coagulation factor X inhibitor which comprises (a) a 5-amidino-2-hydroxybenzenesulfonamide derivative as claimed in claim 1 or 2, or a pharmaceutically acceptable salt thereof, and (b) at least one member selected from the group consisting of adrenocortical hormone, platelet aggregation inhibitors, adenylate cyclase activators, PGF2α antagonists, cyclooxygenase inhibitors, adenosine antagonists, GPIIb/IIIa antagonists, anticoagulants, thrombolitic drugs, antithrombin drugs, free-radical scavengers, immunosuppressant drugs, erythropoietin, fish oil, angiotensin-converting enzyme inhibitors, angiotensin II receptor antagonists, glycation inhibitors, protein kinase C inhibitors, aldose reductase inhibitors, endothelin receptor antagonists, endothelin-converting enzyme inhibitors, neutral endopeptidase inhibitors, thromboxane A₂ synthetase inhibitors, thromboxane A₂ receptor antagonists and PGI₂ agonists.

11. An agent for the prevention or treatment of a disease occurred associating an activated blood coagulation factor X which comprises (a) a 5-amidino-2-hydroxybenzenesulfonamide derivative as claimed in claim 1 or 2, or a pharmaceutically acceptable salt thereof, and (b) at least one member selected from the group consisting of adrenocortical hormone, platelet aggregation inhibitors, adenylate cyclase activators, PGF2α antagonists, cyclooxygenase inhibitors, adenosine antagonists, GPIIb/IIIa antagonists, anticoagulants, thrombolitic drugs, antithrombin drugs, immunosuppressant drugs, erythropoietin, fish oil, angiotensin-converting enzyme inhibitors, angiotensin II receptor antagonists, glycation inhibitors, protein kinase C inhibitors, aldose reductase inhibitors, endothelin receptor antagonists, endothelin-converting enzyme inhibitors, neutral endopeptidase inhibitors, thromboxane A₂ synthetase inhibitors, thromboxane A₂ receptor antagonists and PGI₂ agonists.

12. A method for the prevention or treatment of a disease occurred associating an activated blood coagulation factor X, which comprises administering an effective amount of (a) a 5-amidino-2-hydroxybenzenesulfonamide derivative as claimed in claim 1 or 2, or a pharmaceutically acceptable salt thereof, and (b) one member at least selected from the group consisting of adrenocortical hormone, platelet aggregation inhibitors, adenylate cyclase activators, PGF2α antagonists, cyclooxygenase inhibitors, adenosine antagonists, GPIIb/IIIa antagonists, anticoagulants, thrombolitic drugs, antithrombin drugs, free-radical scavengers, immunosuppressant drugs, erythropoietin, fish oil, angiotensin-converting enzyme inhibitors, angiotensin II receptor antagonists, glycation inhibitors, protein kinase C inhibitors, aldose reductase inhibitors, endothelin receptor antagonists, endothelin-converting enzyme inhibitors, neutral endopeptidase inhibitors, thromboxane A₂ synthetase inhibitors, thromboxane A₂ receptor antagonists and PGI₂ agonists.

13. A use of (a) a 5-amidino-2-hydroxybenzenesulfonamide derivative as claimed in claim 1 or 2, or a pharmaceutically acceptable salt thereof, and (b) one member at least selected from the group consisting of adrenocortical hormone, platelet aggregation inhibitors, adenylate cyclase activators, PGF2α antagonists, cyclooxygenase inhibitors, adenosine antagonists, GPIIb/IIIa antagonists, anticoagulants, thrombolitic drugs, antithrombin drugs, free-radical scavengers,immunosuppressant drugs, erythropoietin, fish oil, angiotensin-converting enzyme inhibitors, angiotensin II receptor antagonists, glycation inhibitors, protein kinase C inhibitors, aldose reductase inhibitors, endothelin receptor antagonists, endothelin-converting enzyme inhibitors, neutral endopeptidase inhibitors, thromboxane A₂ synthetase inhibitors, thromboxane A₂ receptor antagonists and PGI₂ agonists, for the manufacture of a pharmaceutical composition for the prevention or treatment of a disease occurred associating an activated blood coagulation factor X.

14. A 5-cyano-2-hydroxybenzenesulfonamide derivative represented by the general formula: 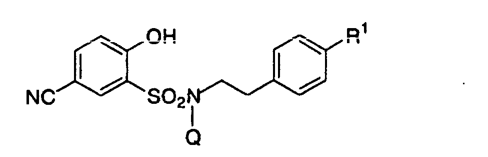 wherein R¹ represents a lower alkyl group which may have -COOR^{A} in which R^{A} is a hydrogen atom or a lower alkyl group, a lower alkoxy group which may have -COOR^{A} in which R^{A} has the same meaning as defined above, a lower alkenyl group which may have -COOR^{A} in which R^{A} has the same meaning as def ined above, a 3 to 10-membered cycloalkyl group, a lower acyl group, -COOR^{A} in which R^{A} has same meaning as defined above, -CONR^{B}R^{C} in which R^{B} and R^{C} are independently a hydrogen atom or a lower alkyl group, or -NR^{B}R^{C} forms a cyclic amino group, an amino group, a mono or di(lower alkyl)amino group, a cyclic amino group which may have an oxo group, a 3 to 10-membered heterocycloalkyl group which may have an oxo group, a 6 to 10-membered aryl group which may have one to three substituents selected from the following group (A), or a 5 to 10-membered aromatic heterocyclic group which may have a substituent selected from the following group (B);
Q represents a hydrogen atom, a lower alkenyl group, a lower alkynyl group or a lower alkyl group which may have a substituent selected from the following group (C);
(A) an oxo group, a lower alkyl group, a halo (lower alkyl) group, -Y¹-R^{D} in which Y¹ is an oxygen atom or a sulfur atom; R^{D} is a hydrogen atom, a halo(lower alkyl ) group, a lower alkyl group which may have -COOR^{D1} in which R^{D1} is a hydrogen atom, a 3 to 10-membered cycloalkyl group or a lower alkyl group, a halogen atom, a nitro group, an amino group, -COOR^{E} in which R^{E} is a hydrogen atom, a 3 to 10-membered cycloalkyl group or a lower alkyl group, a carbamoyl group, a sulfamoyl group, a (lower alkyl)sulfonyl group, a mono (lower alkyl) sulfamoyl group which may have -COOR^{F} in which R^{F} is a hydrogen atom, a 3 to 10-membered cycloalkyl group or a lower alkyl group, and a (lower alkyl)sulfonylamino-substituted (lower alkyl) group;
(B) a lower alkyl group, an amino group and -COOR^{G} in which R^{G} is a hydrogen atom, a 3 to 10-membered cycloalkyl group and a lower alkyl group;
(C) -OR^{H} in which R^{H} is a hydrogen atom, a lower alkenyl group, a phenyl group or a lower alkyl group which may have -OR^{H1} in which R^{H1} is a hydrogen atom or a lower alkyl group, -COOR^{I} in which R^{I} is a hydrogen atom, a 3 to 10-membered cycloalkyl group or a lower alkyl group which may have a substituent selected from the following group (iii), -CONR^{J}R^{K} in which R^{J} and R^{K} are independently a hydrogen atom, an amino group, a 6 to 10-membered aryl group which may have a carbamoyl group, a 5 to 10-membered aromatic heterocyclic group which may have a substituent selected from the following group (iv), a lower alkyl group which may have a substituent selected from the following group (v), or -NR^{J}R^{K} forms a cyclic amino group which may have a substituent selected from the following group (vi), a cyclic amino group which may have an oxo group, a 3 to 10-membered heterocycloalkyl group which may have an oxo group or a halogen atom, a 6 to 10-membered aryl group which may have one to three substituents selected from the following group (i), and a 5 to 10-membered aromatic heterocyclic group which may have one to three substituents selected from the following group (ii);
(i) a halogen atom, a nitro group, an amidino group, a lower alkyl group, a halo(lower alkyl) group, -OR^{L} in which R^{L} is a hydrogen atom or a lower alkyl group, and -COOR^{M} in which R^{M} is a hydrogen atom or a lower alkyl group;
(ii) a halogen atom, an oxo group, a lower alkyl group and a phenyl group;
(iii) -COOR^{I1} in which R^{I1} is a hydrogen atom, a 3 to 10-membered cycloalkyl group or a lower alkyl group, -OCOR^{I2} in which R^{I2} is a 3 to 10-membered cycloalkyl group or a lower alkyl group, -OCOOR^{I3} in which R^{I3} is a 3 to 10-membered cycloalkyl group or a lower alkyl group, -OR^{I4} in which R^{I4} is a hydrogen atom, a 3 to 10-membered cycloalkyl group or a lower alkyl group, -CONR^{I5}R^{I6} in which R^{I5} and R^{I6} are independently a hydrogen atom or a lower alkyl group, or -NR^{I5}R^{I6} forms a cyclic amino group, a 3 to 10-membered cycloalkyl group, a 6 to 10-membered aryl group, a 3 to 10-membered heterocycloalkyl group and a 5 to 10-membered aromatic heterocyclic group;
(iv) a halogen atom, a lower alkyl group which may have -COOR^{J1} in which R^{J1} is a hydrogen atom or a lower alkyl group, a carbamoyl group and -COOR^{J2} in which R^{J2} is a hydrogen atom or a lower alkyl group;
(v) -OR^{J3} in which R^{J3} is a hydrogen atom or a lower alkyl group, and a 5 to 10-membered aromatic heterocyclic group;
(vi) a hydroxy group, a lower alkyl group, a hydroxy (lower alkyl) group, a di (lower alkyl) amino-substituted lower alkyl group, a carbamoyl group, a di(lower alkyl)amino group, a lower acyl group, and -COOR^{J4} in which R^{J4} is a hydrogen atom or a lower alkyl group;
with the proviso that Q does not represent a hydrogen atom when Z represents a hydrogen atom and R¹ represents a lower alkyl group, a lower alkoxy group or a 3 to 10-membered heterocycloalkyl group, or a salt thereof.
